# EUROPEAN PATENT APPLICATION

(11) **EP 4 834 383 A1**
(43) Date of publication of application: **04.11.2026**
(21) Application number: 24911637.7
(22) Date of filing: 30.12.2024
(51) Int. Cl.: A61M 1/06

(54) **WEARING POSITION DETECTION METHOD AND APPARATUS, STORAGE MEDIUM, BRASSIERE AND SYSTEM**

(30) Priority: 30.12.2023 CN 202311870611; 30.12.2023 CN 202311868746; 30.12.2023 CN 202311872090; 30.12.2023 CN 202311873661; 30.12.2023 CN 202311865517; 30.12.2023 CN 202311869893
(71) Applicant: Shenzhen Lute Jiacheng Supply Chain Management Co., Ltd., Shenzhen, Guangdong 518000 (CN)
(72) Inventor: XIE, Guangbao, Shenzhen, Guangdong 518000 (CN); TANG, Jiyan, Shenzhen, Guangdong 518000 (CN); WEI, Feng, Shenzhen, Guangdong 518000 (CN); HU, Jinling, Shenzhen, Guangdong 518000 (CN); YU, Li, Shenzhen, Guangdong 518000 (CN)
(74) Representative: Santarelli
(86) International application number: PCT/CN2024/143938
(87) International publication number: WO 2025/140724

(57) **Abstract**

The present disclosure provides a wearing position detection method and apparatus for a breast pump, a storage medium, a bra, and a system. The wearing position detection method includes: obtaining induction data of a target breast pump or an inductor upon receiving a wearing completion operation for the target breast pump; and determining a wearing position detection result of the target breast pump based on the induction data. This solution eliminates the need to manually set the wearing position of a breast pump after the breast pump is worn by a user, thereby improving the reliability of milk expression data recorded by the breast pump.

## Description

### TECHNICAL FIELD

The present disclosure relates to the technical field of intelligent devices, and in particular, to a wearing position detection method and apparatus for a breast pump, a storage medium, a bra, and a system.

### BACKGROUND

A breast pump is a commonly used tool for assisting lactating women in expressing milk and clearing mammary ducts. Currently, there are three types of breast pumps available on the market: manual, semi-automatic, and automatic. The automatic breast pumps feature a letdown mode and an expression mode, and are widely used due to their convenient and labor-saving operation. However, various performance metrics, such as suction force, vary among breast pumps on the market. Electric breast pumps have become an essential device for breastfeeding mothers.

Nevertheless, when wearing a breast pump, a user currently needs to manually set the wearing position of the breast pump. If the wearing position manually selected by the user is inconsistent with the actual wearing position, the reliability of milk expression data subsequently recorded by the breast pump may be compromised.

Upon completing the wearing process, the user cannot know whether the breast pump is correctly worn, which may thus lead to problems such as the breast pump failing to express the breast milk in use.

When wearing the breast pump, the user is required to manually set the wearing position on the breast pump. An existing breast pump indicates the wearing position merely via a simple tab or button. After the user finishes setting, there is no clear visual indication to confirm whether the wearing position of the breast pump set by the user corresponds to the actual wearing position of the user. This requires users to perform confirmation for a plurality of times, resulting in a time-consuming process and poor efficiency in recording the wearing position.

### BRIEF SUMMARY

In view of this, the present disclosure provides a wearing position detection method and apparatus for a breast pump, a storage medium, a bra, and a system, enabling automatic setting of the wearing position of the breast pump and thereby improving the reliability of milk expression data recorded by the breast pump.

In the first aspect, an embodiment of the present disclosure provides a wearing position detection method for a breast pump, including:
obtaining induction data of a target breast pump or the inductor upon receiving a wearing completion operation for the target breast pump; and
determining a wearing position detection result of the target breast pump based on the induction data.

In the second aspect, an embodiment of the present disclosure provides a wearing position detection apparatus for a breast pump, including:
an obtaining module configured to obtain induction data of a target breast pump or an inductor upon receiving a wearing completion operation for the target breast pump; and
a determination module configured to determine a wearing position detection result of the target breast pump based on the induction data.

In the third aspect, an embodiment of the present disclosure provides a computer-readable storage medium, having a computer program stored thereon, where the computer program, when executed by a processor, causes the processor to perform the steps of the wearing position detection method for a breast pump as described above.

In the fourth aspect, an embodiment of the present disclosure further provides an electronic device including a memory and a processor, where the memory has a computer program stored thereon that, when executed by the processor, causes the processor to perform the steps of the wearing position detection method for a breast pump as described above.

Embodiments of the present disclosure provide a wearing position detection method and apparatus for a breast pump, a storage medium, a bra, and a system. The method includes: obtaining induction data of a target breast pump or an inductor upon receiving a wearing completion operation for the target breast pump; and determining a wearing position detection result of the target breast pump based on the induction data. In the wearing position detection solution for a breast pump provided by the present disclosure, when the wearing completion operation for the target breast pump is received, the induction data of the bra corresponding to the target breast pump may be obtained. The wearing position detection result of the target breast pump is output via the position detection strategy corresponding to the induction data. Thus, a user does not need to manually set the wearing position of the breast pump after wearing the breast pump. As a result, the reliability of milk expression data recorded by the breast pump is improved.

The interaction ways for the user with the electronic device are enriched, thereby meeting the requirements of different users in different scenarios.

In the first aspect, an embodiment of the present disclosure provides a wearing position detection method for a breast pump, including:
configuring a sensor disposed on the breast pump to acquire human body data;
obtaining the human body data acquired by the sensor upon detecting that a target breast pump is in a wearing state; and
determining a position wearing result of the target breast pump based on the human body data.

In the second aspect, an embodiment of the present disclosure provides a breast pump, including:
a breast pump body configured to be worn on a breast to express milk from the breast;
an inductive assembly disposed on the breast pump body and configured to acquire human body data and determine a position wearing result of a target breast pump based on the human body data.

In the third aspect, an embodiment of the present disclosure provides a computer-readable storage medium, having a computer program stored thereon, where the computer program, when executed by a processor, causes the processor to perform the steps of the wearing position detection method for a breast pump as described above.

Embodiments of the present disclosure provide a wearing position detection method for a breast pump, a breast pump, and a computer-readable storage medium. The method includes: configuring a sensor disposed on the breast pump to acquire human body data; obtaining the human body data acquired by the sensor upon detecting that a target breast pump is in a wearing state; and finally, determining a position wearing result of the target breast pump according to the human body data. In the wearing position detection solution for a breast pump provided by the present disclosure, when wearing the target breast pump, the user can automatically set the wearing position of the target breast pump according to the human body data acquired by the sensor. Thus, the reliability of the milk expression data recorded by the breast pump is improved.

In the first aspect, an embodiment of the present disclosure provides a wearing position detection method for a breast pump, including:
when a two-side wearing operation is detected, receiving sensor data reported by a first breast pump, where the sensor data is data of an inductor of a second breast pump that is acquired by a sensor disposed on the first breast pump; and
determining position wearing results of the first breast pump and the second breast pump according to the sensor data.

In the second aspect, an embodiment of the present disclosure provides a wearing position detection apparatus for a breast pump, including:
a first breast pump and a second breast pump;
a first inductive assembly disposed on the first breast pump; and
a second inductive assembly disposed on the second breast pump, where the first inductive assembly and the second inductive assembly are configured to interact inductively with each other to detect left/right wearing positions of the first breast pump and the second breast pump.

In the third aspect, an embodiment of the present disclosure provides a computer-readable storage medium, having a computer program stored thereon, where the computer program, when executed by a processor, causes the processor to perform the steps of the wearing position detection method for a breast pump as described above.

In the fourth aspect, an embodiment of the present disclosure further provides an electronic device including a memory and a processor, where the memory has a computer program stored thereon that, when executed by the processor, causes the processor to perform the steps of the wearing position detection method for a breast pump as described above.

Embodiments of the present disclosure provide a wearing position detection method and apparatus for a breast pump, a storage medium, and an electronic device. When a two-side wearing operation is detected, sensor data reported by a first breast pump is received, where the sensor data is data of an inductor of a second breast pump that is acquired by a sensor disposed on the first breast pump. Position wearing results of the first breast pump and the second breast pump are determined according to the sensor data. In the wearing position detection solution for a breast pump provided by the present disclosure, when the user wears the breast pumps on the breasts on both sides, the data of the inductor of the second breast pump is acquired by using the sensor disposed in the first breast pump. Based on the sensor data, the wearing positions of the first breast pump and the second breast pump can be set automatically, thereby improving the reliability of the milk expression data recorded by the breast pump.

On this basis, the present disclosure provides a wearing prompting method and system for a breast pump, a storage medium, and an electronic device. A corresponding prompting instruction may be determined according to wearing information acquired by a sensor of the breast pump. Then, the breast pump and/or a current device is controlled to execute a prompting event according to the prompting instruction so that a user can obtain the actual wearing status of the breast pump.

In the first aspect, an embodiment of the present disclosure provides a wearing prompting method for a breast pump, including:
detecting wearing information corresponding to the breast pump in response to a wearing operation triggered for the breast pump, where the wearing information is acquired by a sensor built in the breast pump; and
controlling the breast pump and/or a current device to execute a prompting event according to the wearing information.

In the second aspect, an embodiment of the present disclosure further provides a wearing prompting system for a breast pump, including:
a breast pump body;
a wearing detection mechanism configured to detect wearing information of the breast pump body; and
a signal prompting element configured to output prompting information based on the wearing information of the breast pump.

Optionally, in some embodiments of the present disclosure, the wearing prompting system further includes:
a communication connection element configured for communication connection between the breast pump body and a terminal device to output prompting information via the terminal device.

Optionally, in some embodiments of the present disclosure, the prompting information includes a prompt tone, a prompting page, and a wearing guidance.

Optionally, in some embodiments of the present disclosure, the wearing detection mechanism includes a sensor configured to detect whether the breast pump body closely fits a breast and/or detect whether a mother's nipple is placed along a nipple channel and/or detect whether the breast pump body is worn obliquely.

In the third aspect, an embodiment of the present disclosure provides a computer-readable storage medium, having a computer program stored thereon, where the computer program, when executed by a processor, causes the processor to perform the steps of the wearing prompting method for a breast pump as described above.

In the fourth aspect, an embodiment of the present disclosure further provides an electronic device including a memory and a processor, where the memory has a computer program stored thereon that, when executed by the processor, causes the processor to perform the steps of the wearing prompting method for a breast pump as described above.

Embodiments of the present disclosure provide a wearing prompting method and system for a breast pump, a storage medium, and an electronic device. The wearing information corresponding to the breast pump is obtained in response to the wearing operation triggered for the breast pump, where the wearing information is acquired by the sensor built in the breast pump. The prompting instruction corresponding to the wearing information is then determined. Finally, the breast pump and/or the current device is controlled to execute the prompting event according to the prompting instruction. In the wearing prompting solution for a breast pump provided by the present disclosure, the corresponding prompting instruction is determined according to the wearing information acquired by the sensor of the breast pump. The breast pump and/or the current device is controlled to execute the prompting event according to the prompting instruction so that the user can obtain the actual wearing status of the breast pump. This avoids the problem that the user is unable to express the breast milk when using the breast pump due to incorrect wearing.

On this basis, the present disclosure provides a wearing guidance method for a breast pump and a breast pump system. A user can be guided to wear the breast pump via a wearing guidance page. This enables the user to precisely and effectively wear the breast pump.

The interaction ways for the user with the electronic device are enriched, thereby meeting the requirements of different users in different scenarios.

In the first aspect, an embodiment of the present disclosure provides a wearing guidance method for a breast pump, including:
showing a wearing guidance page for a target breast pump in response to a wearing operation triggered for the target breast pump, where the wearing guidance page includes wearing guidance information for the target breast pump, and the wearing guidance information is configured for guiding a user to wear the target breast pump in an operating mode.

In the second aspect, an embodiment of the present disclosure further provides a breast pump system, including:
a breast pump body configured to be worn on a mother's breast and express milk from the breast, where the breast pump body includes:
a breast milk container including a milk storage container and a connection portion, where the milk storage container is connected to the connection portion;
a milk pumping member including an attachment portion and a nipple channel, where a shape of the attachment portion is adapted to the breast, and the nipple channel is connected to the milk storage container via the connection portion; and
a wearing detection mechanism configured to detect whether the milk pumping member is worn correctly.

Optionally, in some embodiments of the present disclosure, the wearing detection mechanism includes a first sensor disposed on the attachment portion and a second sensor disposed on the nipple channel.

Optionally, in some embodiments of the present disclosure, the first sensor is configured to detect whether the attachment portion closely fits the breast.

Optionally, in some embodiments of the present disclosure, the second sensor is configured to detect whether the mother's nipple is placed along the nipple channel.

Optionally, in some embodiments of the present disclosure, the wearing detection mechanism further includes a third sensor configured to detect whether the breast pump body is worn obliquely.

Optionally, in some embodiments of the present disclosure, the breast pump system further includes a communication connection module configured to connect the breast pump body and a terminal device or a server and transmit milk expression-related information of the breast pump body.

Optionally, in some embodiments of the present disclosure, the breast pump system further includes an identification element configured at least to identify one target breast pump body such that the terminal device or the server identifies and matches the target breast pump body.

Optionally, in some embodiments of the present disclosure, the breast pump system further includes a first identification element and a second identification element that are configured at least to identify two target breast pump bodies, respectively, such that the terminal device or the server identifies and matches each of the target breast pump bodies.

In the third aspect, an embodiment of the present disclosure provides a computer-readable storage medium, having a computer program stored thereon, where the computer program, when executed by a processor, causes the processor to perform the steps of the wearing guidance method for a breast pump as described above.

In the fourth aspect, an embodiment of the present disclosure further provides an electronic device including a memory and a processor, where the memory has a computer program stored thereon that, when executed by the processor, causes the processor to perform the steps of the wearing guidance method for a breast pump as described above.

Embodiments of the present disclosure provide a wearing guidance method for a breast pump and a breast pump system. A wearing guidance page for a target breast pump is shown in response to a wearing operation for the target breast pump, where the wearing guidance page includes wearing guidance information for the target breast pump, and the wearing guidance information is configured for instructing the wearing of the target breast pump in an operating mode. It is then determined whether the current wearing state of the target breast pump satisfies a preset operating wearing state. When the current wearing state of the target breast pump satisfies the operating wearing state, the target breast pump is configured with configuration parameters corresponding to a target operating mode, and prompting information for successful wearing is shown in the wearing guidance page. In the wearing guiding solution for a breast pump provided by the present disclosure, when the user triggers the wearing operation for the breast pump, the wearing guidance page for the target breast pump may be shown, where the wearing guidance page contains the wearing guidance information. Therefore, the user can be instructed by the wearing guidance information to wear the breast pump in the operating mode. When the user uses the breast pump for the first time or wears the breast pump again, the user can be guided by the wearing guidance page to correctly wear it.

On this basis, the present disclosure provides a wearing position recording method for a breast pump. The time taken by a user to set the wearing position of the breast pump can be reduced, and the recording efficiency of the wearing position can be improved.

The interaction ways for the user with the electronic device are enriched, thereby meeting the requirements of different users in different scenarios.

In the first aspect, an embodiment of the present disclosure provides a wearing position recording method for a breast pump, including:
displaying a wearing marking interface upon detecting that a target breast pump is in a wearing state; and
marking a wearing position of the target breast pump in the wearing marking interface and/or enabling a marking prompt for the target breast pump in response to a first marking operation.

In the second aspect, an embodiment of the present disclosure provides a wearing position recording method for a breast pump, including:
upon detecting that a target breast pump is in a wearing state, activating a marking indicator lamp of the target breast pump and/or showing a wearing mark in a wearing marking interface in response to a second marking operation for the target breast pump.

In the third aspect, an embodiment of the present disclosure provides a wearing position recording apparatus for a breast pump, including:
a display module configured to display a wearing marking interface upon detecting that a target breast pump is in a wearing state; and
a marking module configured to mark a wearing position of the target breast pump in the wearing marking interface and/or enable a marking prompt for the target breast pump in response to a first marking operation.

In the fourth aspect, an embodiment of the present disclosure provides a wearing position recording apparatus for a breast pump, including:
an activation module configured to, upon detecting that a target breast pump is in a wearing state, activate a marking indicator lamp of the target breast pump and/or show a wearing mark in a wearing marking interface in response to a second marking operation for the target breast pump.

In a fifth aspect, an embodiment of the present disclosure provides a computer-readable storage medium, having a computer program stored thereon, where the computer program, when executed by a processor, causes the processor to perform the steps of the wearing position recording method for a breast pump as described above.

In a sixth aspect, an embodiment of the present disclosure further provides an electronic device including a memory and a processor, where the memory has a computer program stored thereon that, when executed by the processor, causes the processor to perform the steps of the wearing position recording method for a breast pump as described above.

Embodiments of the present disclosure provide a wearing position recording method and apparatus for a breast pump, a storage medium, and an electronic device. A wearing marking interface is displayed upon detecting that the target breast pump is in a wearing state. In response to a first marking operation, a wearing position of the target breast pump is marked in the wearing marking interface and/or a marking prompt for the target breast pump is enabled. In the wearing position recording solution for a breast pump provided by the present disclosure, when the user wears the target breast pump, the wearing marking interface may be displayed, and according to the first marking operation for the wearing marking interface by the user, the wearing position of the target breast pump is marked and/or the marking prompt for the target breast pump is enabled. That is, with a striking interface prompt and/or breast pump prompt, the user can know the wearing position of the target breast pump without confirmation for a plurality of times. Thus, the time taken by the user to set the wearing position of the breast pump can be reduced, thereby improving the recording efficiency of the wearing position.

Embodiments of the present disclosure provide a wearing position recording method and apparatus for a breast pump, a storage medium, and an electronic device. Upon detecting that a target breast pump is in a wearing state, in response to a second marking operation for the target breast pump, a marking indicator lamp of the target breast pump is activated and/or a wearing mark is shown in a wearing marking interface. In the wearing position recording solution for a breast pump provided by the present disclosure, the marking indicator lamp of the target breast pump may be activated and/or the wearing mark may be shown in the wearing marking interface, so that the user can know the wearing position of the target breast pump without confirmation for a plurality of times. Thus, the time taken by the user to set the wearing position of the breast pump can be reduced, thereby improving the recording efficiency of the wearing position.

### BRIEF DESCRIPTION OF THE DRAWINGS

To describe the technical solutions in the embodiments of the present disclosure or in the prior art more clearly, the following briefly describes the accompanying drawings required for describing the embodiments or the prior art. Apparently, the accompanying drawings in the following description show some embodiments of the present disclosure, and a person of ordinary skill in the art may still derive other drawings from these accompanying drawings without creative efforts.

In the drawings,
FIG. 1 is a schematic diagram showing a scenario where a wearing position detection method for a breast pump provided by an embodiment of the present disclosure is applied;
FIG. 2 is a flowchart of a wearing position detection method for a breast pump provided by an embodiment of the present disclosure;
FIG. 3 is a schematic structural diagram of a wearing position detection system for a breast pump provided by an embodiment of the present disclosure;
FIG. 4 is another schematic structural diagram of a wearing position detection system for a breast pump provided by an embodiment of the present disclosure;
FIG. 5 is yet another schematic structural diagram of a wearing position detection system for a breast pump provided by an embodiment of the present disclosure;
FIG. 6 is a schematic structural diagram of an electronic device provided by the present disclosure;
FIG. 7 is a schematic diagram showing a scenario where a wearing position detection method for a breast pump provided by an embodiment of the present disclosure is applied;
FIG. 8 is a flowchart of a wearing position detection method for a breast pump provided by an embodiment of the present disclosure;
FIG. 9 is a schematic structural diagram of a wearing position detection system for a breast pump provided by an embodiment of the present disclosure;
FIG. 10 is a schematic structural diagram of an electronic device provided by the present disclosure;
FIG. 11 is a schematic diagram showing a scenario where a wearing position detection method for a breast pump provided by an embodiment of the present disclosure is applied;
FIG. 12 is a flowchart of a wearing position detection method for a breast pump provided by an embodiment of the present disclosure;
FIG. 13 is a schematic structural diagram of a first embodiment of an arrangement position of an inductive element provided by an embodiment of the present disclosure;
FIG. 14 is a schematic structural diagram of a second embodiment of an arrangement position of an inductive element provided by an embodiment of the present disclosure;
FIG. 15 is a schematic structural diagram of a third embodiment of an arrangement position of an inductive element provided by an embodiment of the present disclosure;
FIG. 16 is a schematic structural diagram of a fourth embodiment of an arrangement position of an inductive element provided by an embodiment of the present disclosure;
FIG. 17 is a schematic structural diagram of a first embodiment of a wearing position detection apparatus for a breast pump provided by an embodiment of the present disclosure;
FIG. 18 is a schematic structural diagram of a second embodiment of a wearing position detection apparatus for a breast pump provided by an embodiment of the present disclosure;
FIG. 19 is a schematic structural diagram of a third embodiment of a wearing position detection apparatus for a breast pump provided by an embodiment of the present disclosure;
FIG. 20 is a schematic structural diagram of a fourth embodiment of a wearing position detection apparatus for a breast pump provided by an embodiment of the present disclosure;
FIG. 21 is a schematic structural diagram of a fifth embodiment of a wearing position detection apparatus for a breast pump provided by an embodiment of the present disclosure;
FIG. 22 is a schematic structural diagram of an electronic device provided by the present disclosure;
FIG. 23 is a schematic diagram showing a scenario where a wearing prompting method for a breast pump provided by an embodiment of the present disclosure is applied;
FIG. 24 is flowchart of a wearing prompting method for a breast pump provided by an embodiment of the present disclosure;
FIG. 25 is a diagram showing a prompting page for wearing on the left and the right in a wearing prompting method for a breast pump provided by an embodiment of the present disclosure;
FIG. 26 is a schematic diagram showing correct wearing in a wearing prompting method for a breast pump provided by an embodiment of the present disclosure;
FIG. 27 is another schematic diagram showing correct wearing in a wearing prompting method for a breast pump provided by an embodiment of the present disclosure;
FIG. 28 is a schematic diagram showing unsuccessful wearing in a wearing prompting method for a breast pump provided by an embodiment of the present disclosure;
FIG. 29 is another schematic diagram showing unsuccessful wearing in a wearing prompting method for a breast pump provided by an embodiment of the present disclosure;
FIG. 30 is a schematic structural diagram of a wearing prompting system for a breast pump provided by an embodiment of the present disclosure;
FIG. 31 is a schematic structural diagram of an electronic device provided by the present disclosure;
FIG. 32 is a schematic diagram showing a scenario where a wearing guidance method for a breast pump provided by an embodiment of the present disclosure is applied;
FIG. 33 is flowchart of a wearing guidance method for a breast pump provided by an embodiment of the present disclosure;
FIG. 34 is a schematic diagram showing a wearing guidance in a wearing guidance method for a breast pump provided by an embodiment of the present disclosure;
FIG. 35 is a schematic diagram showing successful wearing in a wearing guidance method for a breast pump provided by an embodiment of the present disclosure;
FIG. 36 is a schematic diagram showing unsuccessful wearing in a wearing guidance method for a breast pump provided by an embodiment of the present disclosure;
FIG. 37 is another schematic diagram showing a wearing guidance in a wearing guidance method for a breast pump provided by an embodiment of the present disclosure;
FIG. 38 is yet another schematic diagram showing a wearing guidance in a wearing guidance method for a breast pump provided by an embodiment of the present disclosure;
FIG. 39 is a schematic structural diagram of a breast pump system provided by an embodiment of the present disclosure;
FIG. 40 is another schematic structural diagram of a breast pump system provided by an embodiment of the present disclosure;
FIG. 41 is a schematic structural diagram of an electronic device provided by the present disclosure;
FIG. 42 is a schematic diagram showing a scenario where a wearing position recording method for a breast pump provided by an embodiment of the present disclosure is applied;
FIG. 43 is a flowchart of a wearing position recording method for a breast pump provided by an embodiment of the present disclosure;
FIG. 44 is a schematic diagram showing a wearing marking interface in a wearing position recording method for a breast pump provided by an embodiment of the present disclosure is applied;
FIG. 45 is another schematic diagram showing a wearing marking interface in a wearing position recording method for a breast pump provided by an embodiment of the present disclosure is applied;
FIG. 46 is another flowchart of a wearing position recording method for a breast pump provided by an embodiment of the present disclosure;
FIG. 47 is yet another flowchart of a wearing position recording method for a breast pump provided by an embodiment of the present disclosure;
FIG. 48 is a schematic structural diagram of a wearing position recording apparatus for a breast pump provided by an embodiment of the present disclosure;
FIG. 49 is another schematic structural diagram of a wearing position recording apparatus for a breast pump provided by an embodiment of the present disclosure;
FIG. 50 is yet another schematic structural diagram of a wearing position recording apparatus for a breast pump provided by an embodiment of the present disclosure; and
FIG. 51 is a schematic structural diagram of an electronic device provided by the present disclosure.

### DETAILED DESCRIPTION OF THE DRAWINGS AND THE PRESENTLY PREFERRED EMBODIMENTS

The technical solutions in the embodiments of the present disclosure are clearly and completely described below with reference to the drawings in the embodiments of the present disclosure. Apparently, the described embodiments are only some rather than all of the embodiments of the present disclosure. All other embodiments derived from the embodiments of the present disclosure by a person of ordinary skill in the art without creative efforts shall fall within the protection scope of the present disclosure.

The present disclosure provides a wearing position detection method and apparatus for a breast pump, a storage medium, a bra, and a system.

The wearing position detection apparatus for a breast pump provided by the present disclosure may be integrated in an electronic device. The electronic device may be directly or indirectly connected to another electronic device in a wired or wireless communication manner. The electronic device may be, but is not limited to, a smartphone, a tablet computer, a laptop computer, a desktop computer, a smart speaker, a smart watch, or the like. Furthermore, the electronic device may also be connected to a server. The server may be an independent physical server, a server cluster or distributed system formed by a plurality of physical servers, or a cloud server that provides fundamental cloud computing services such as cloud services, cloud databases, cloud computing, cloud functions, cloud storage, network services, cloud communication, middleware services, domain name services, security services, content delivery network (CDN), and big data and artificial intelligence platforms. The present disclosure imposes no limitation in this regard.

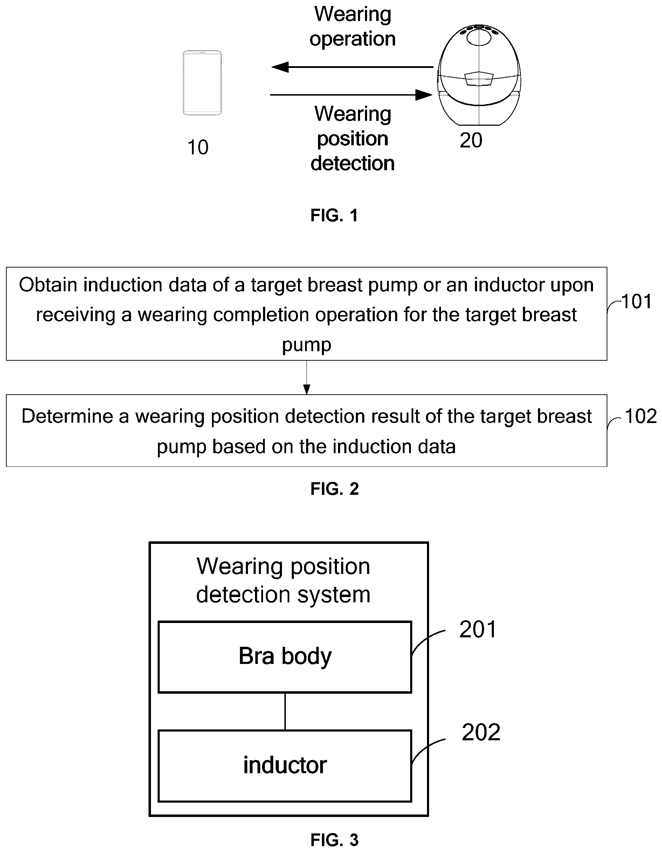

For example, referring to FIG. 1, the present disclosure provides a wearing position detection apparatus for a breast pump. The wearing position detection apparatus for a breast pump is integrated in a terminal 10. The terminal 10 may be a mobile phone, a tablet computer, or an independently configured operation control setup. When receiving a wearing completion operation for a target breast pump, the terminal 10 obtains induction data between the target breast pump and a bra. The induction data is data detected by the target breast pump from the inductor disposed on the bra or data of the target breast pump detected by the inductor disposed on the bra. The wearing position of the target breast pump 20 may be on the user's left breast or the user's right breast. After receiving the induction data, the terminal 10 determines a wearing position detection result of the target breast pump based on the induction data.

By distinguishing the induction data between the target breast pump and the inductor on the left cup of the bra versus the induction data between the target breast pump and the inductor on the right cup of the bra, it can be determined whether the target breast pump is worn on the left breast or the right breast. For example, the induction data may be distinguished according to the presence/absence of a signal, signal strength, differential setting, a signal type, etc.

The induction data may be distinguished based on whether the target breast pump and the inductor disposed on the bra interact inductively with each other. For example, an inductor is disposed on the left cup of the bra, while no inductor is disposed on the right cup of the bra, and the target breast pump is placed inside the bra. If the target breast pump interacts inductively with the inductor, it can be determined that the target breast pump is currently worn on the left side. If the target breast pump does not interact inductively with the inductor, it can be determined that the target breast pump is currently worn on the right side. Alternatively, the inductor may be disposed in the middle of the bra, i.e., between the cups on both sides, while a sensor is disposed on the left side/right side of the target breast pump. For example, the sensor is disposed on the left side of the target breast pump, and the target breast pump is placed inside the bra. If the target breast pump interacts inductively with the inductor, it can be determined that the target breast pump is currently worn on the right side. If the target breast pump does not interact inductively with the inductor, it can be determined that the target breast pump is currently worn on the left side.

The induction data may be distinguished according to the size, strength, and the like of the induction data between the target breast pump and the inductor disposed on the bra. For example, a first inductor is disposed on the left cup of the bra, while a second inductor is disposed on the right cup of the bra, and the first inductor is different from the second inductor. The target breast pump is placed inside the bra. If the target breast pump interacts inductively with the first inductor, it can be determined that the target breast pump is currently worn on the left side. If the target breast pump interacts inductively with the second inductor, it can be determined that the target breast pump is currently worn on the right side.

According to the wearing position detection method for a breast pump provided by the present disclosure, when the wearing completion operation for the target breast pump is received, the induction data between the target breast pump and the bra may be obtained. The wearing position detection result of the target breast pump is output via the position detection strategy corresponding to the induction data. Thus, the user does not need to manually set the wearing position of the breast pump after wearing the breast pump. The wearing position of the breast pump, i.e., whether the breast pump is worn on the left breast or the right breast, can be automatically identified. As a result, the reliability of milk expression data recorded by the breast pump is improved.

A wearing position detection method for a breast pump provided by the present disclosure includes: obtaining induction data of a target breast pump or an inductor upon receiving a wearing completion operation for the target breast pump; determining a data type of the induction data; and determining a wearing position detection result of the target breast pump based on a position detection strategy corresponding to the data type.

FIG. 2 is a flowchart of a wearing position detection method for a breast pump provided by the present disclosure. The specific process of the wearing position detection method for a breast pump may include the following steps.

In step 101, induction data of a target breast pump or an inductor is obtained upon receiving a wearing completion operation for the target breast pump.

The target breast pump refers to the breast pump currently being worn. Generally, breast pumps are provided in pairs for pumping breast milk from a mother's left and right breasts, respectively. That is, the breast pump purchased by the user may include a first breast pump and a second breast pump. During use, the user may wear the first breast pump first or the second breast pump first, and may initially wear the first breast pump on the left breast or on the right breast. Furthermore, the user may wear a breast pump on only one breast or wear the breast pumps on both breasts simultaneously, which can be selected based on actual circumstances.

For example, specifically, after the user triggers a wearing operation for the target breast pump, induction data between a sensor disposed on the target breast pump and an inductor disposed on the bra is transmitted to an electronic device wirelessly or via Bluetooth.

Additionally, the inductor may also be an iron plate or an iron block. A hall sensor is built in the target breast pump. Specifically, taking a breast pump set as an example, the breast pump set includes a first breast pump and a second breast pump. A first hall sensor is disposed on the left/right side of the first breast pump, while a detachable strongly magnetic module is disposed on the right/left side of the same. A second hall sensor is disposed on the left side of the second breast pump, and a detachable strongly magnetic module is disposed on the right side of the same as well. The inductor (e.g., an iron plate) is disposed in the middle of the bra (i.e., the center gore of the bra). Before use, the detachable strongly magnetic module on the target breast pump is removed and attached to the iron plate in the middle of the bra. The target breast pump is then fixed inside the bra for use. When the target breast pump detects, via the hall sensor on the left side inside the device, the strongly magnetic module fixed in the middle position of the bra, it can be determined that the target breast pump is currently worn on the right side. Similarly, if the target breast pump does not detect, via the hall sensor on the left side inside the device, the strongly magnetic module fixed in the middle position of the bra, it can be determined that the target breast pump is currently worn on the left side.

Additionally, the inductor may also be configured as a magnetic member, and a hall sensor is disposed on the left/right side of the target breast pump. For example, a first hall sensor is disposed on the left side of the breast pump, and the inductor is disposed in the middle position of the bra. When the target breast pump is placed inside the bra for use, if the target breast pump detects the inductor via the hall sensor, it can be determined that the target breast pump is currently worn on the right side. Similarly, if the target breast pump does not detect the inductor via the hall sensor, it can be determined that the target breast pump is currently worn on the left side. In this way, it can be detected whether the target breast pump is worn on the left side or the right side. If two breast pumps are worn simultaneously, since the position of one breast pump has already been determined according to the above method, it is naturally known that the other breast pump is worn on the left breast. For example, if the first worn breast pump is detected to be on the left side, it is automatically determined that the other breast pump is worn on the right side. In some embodiments, the positions of the inductor and the hall sensor may be interchanged. That is, the hall sensor is disposed in the middle position of the bra (between the two cups), while the magnetic member is disposed on the left/right side of the breast pump.

It should be noted that, in this embodiment, if the user wears breast pumps on both sides, it is only necessary to detach the strongly magnetic module once and attach it to the iron plate in the middle of the bra. Thus, there is no need for the user to manually set the wearing position of the breast pump.

Optionally, in some embodiments, the sensor disposed on the target breast pump may be a capacitive sensor or an infrared sensor. The capacitive sensor is a conversion device that uses various types of capacitors as sensing elements to transform measured physical or mechanical quantities into changes in capacitance. Essentially, it is a capacitor with variable parameters. Capacitive sensors are widely used for measuring displacement, angle, vibration, velocity, pressure, composition analysis, medium properties, and the like. The most commonly used types are parallel-plate capacitors or cylindrical capacitors.

The infrared sensor is a sensor that utilizes infrared radiation for data processing and offers advantages such as high sensitivity. The infrared sensor can control the operation of drives. Infrared sensors are commonly employed in non-contact temperature measurement, gas composition analysis, and non-destructive testing, finding extensive applications in fields such as medicine, military, space technology, and environmental engineering. For example, by using an infrared sensor to remotely measure the thermal image of the human body's surface temperature, areas with abnormal temperatures can be found. In the present disclosure, based on the distance and human body surface temperature acquired by the infrared sensor, it is determined that the user is currently wearing the breast pump.

In step 102, a wearing position detection result of the target breast pump is determined based on the induction data.

It should be noted that, in the embodiments of the present disclosure, an inductor is disposed on the bra. The inductor may be a tag based on near field communication (NFC) technology. When the user uses the breast pump, the NFC tag can be detachably provided on or embedded within the bra. For example, the bra may be provided with a small pocket for placing or removing the NFC tag, or the NFC tag may be adhered to or peeled off from the bra, or attached by any other detachable means. Alternatively, the NFC tag may be integrated into the bra, thereby forming a bra with the NFC tag. Data from the NFC tag is then acquired by an NFC sensor inside the target breast pump. NFC technology evolves from the integration of non-contact radio frequency identification and interconnection technologies. By integrating an inductive card reader, an inductive card, and peer-to-peer communication functionality onto a single chip, it enables applications such as mobile payments, electronic ticketing, access control, mobile identity recognition, and anti-counterfeiting via mobile terminals. In the present disclosure, it is applied for sensing the position of the breast pump.

Optionally, in some embodiments, an NFC tag may be provided on one side of the bra, while no NFC tag is provided on the other side. For example, an NFC tag is provided on the left side of the bra, while no NFC tag is provided on the right side. When the target breast pump is placed inside the bra for use, if the NFC tag is detected by the target breast pump via an NFC sensor, it can be determined that the breast pump is currently worn on the left side. If no NFC tag is detected by the target breast pump via the NFC sensor, it can be determined that the breast pump is currently worn on the right side. In this way, it can be detected whether the breast pump is worn on the left side or the right side. In some embodiments, the positions of the NFC sensor and the NFC tag may be interchanged. That is, the NFC sensor is provided on the bra, while the NFC tag is provided on the breast pump. Alternatively, two different NFC tags may be provided on the left and right sides of the bra in advance, and the data information of these two different NFC tags may be pre-stored, including the respective positions (left side/right side) of the two different NFC tags. When the target breast pump is powered on for use and placed inside the bra, if the NFC tag on the left side is detected by the target breast pump via the NFC sensor, it can be determined that the target breast pump is currently worn on the left side. If the NFC tag on the right side is detected by the target breast pump via the NFC sensor, it can be determined that the target breast pump is currently worn on the right side. In this way, it can be detected whether the target breast pump is worn on the left side or the right side. When an NFC tag reading device inside the target breast pump identifies a closer NFC tag sticker, by reading the internal data of the NFC tag sticker and distinguishing between left and right based on the data, it is automatically identified whether the device is positioned on the left side or the right side during use.

The inductor may also be an iron plate or an iron block. However, unlike the foregoing embodiments, the inductor is disposed on one side of the bra, for example, on the left side or the right side of the bra. During practical use, the capacitive sensor and the infrared sensor acquire data simultaneously. The capacitive sensor acquires data from the iron plate, while the infrared sensor acquires the distance between the target breast pump and the human breast. That is, the infrared data from the infrared sensor may be used to determine that the user is currently wearing the target breast pump. If the inductor is disposed on the left side of the bra, when the capacitive sensor acquires data from the inductor, both the infrared data and the induction data are reported to the electronic device. If the capacitive sensor does not acquire data from the inductor, only the infrared data is reported to the electronic device. Subsequently, the wearing position of the breast pump may be determined based on the reported data.

Optionally, in some embodiments, the inductor may also be a signal sensor. This signal sensor emits a first induction signal and a second induction signal on both sides, respectively, for example, emits the first induction signal on the left side and the second induction signal on the right side, with the first induction signal being different from the second induction signal. When the user wears the breast pump, the target breast pump may acquire either the first induction signal or the second induction signal to subsequently determine the wearing position. When the target breast pump is placed inside the bra for use, if the first induction signal or the second induction signal is detected by the target breast pump via the signal sensor, it can thus be detected whether the target breast pump is worn on the left side or the right side. If two breast pumps are worn simultaneously, since the position of one breast pump has already been determined according to the above method, it is naturally known that the other breast pump is worn on the left breast. For example, if the first worn breast pump is detected to be on the left side, it is automatically determined that the other breast pump is worn on the right side. The first induction signal and the second induction signal may be distinguished by the strength of the acquired first induction signal and second induction signal, enabling the determination of the wearing position.

In step 103, milk expression data of the breast pumps on breasts of different sides is recorded correspondingly.

The wearing positions of the breast pumps on different sides are fed back/announced for user confirmation. This can correct system determination errors.

It will be understood that, in the present disclosure, different sensors and inductors correspond to different position detection strategies. For example, for hall data, the corresponding position detection strategy is to detect whether the target breast pump can acquire the signal from the strongly magnetic module located at the center gore of the bra. For composite induction data from the capacitive sensor and the infrared sensor, the position is determined by determining the presence or absence of the capacitive sensor.

For the same pair of breast pumps, the sensors mounted on them may be the same or different. That is, different inductors may also be mounted on the bra. For example, a hall sensor and a strongly magnetic module are built in a breast pump 1, while an NFC acquisition device is built in a breast pump 2. An NFC tag is provided on the left side of the bra, and an iron plate is disposed at the center gore. When the user wears a pump on one side only, if the user selects the breast pump 1, during use, the strongly magnetic module is attached to the iron plate. The hall sensor of the breast pump 1 reports the acquired data to the electronic device. After receiving the induction data, the electronic device analyzes its data type as hall data. In this case, the position detection strategy corresponding to the hall data is determined as follows: it is determined whether data from the strongly magnetic module has been acquired; if the data has been acquired, it is determined that the user is currently wearing the breast pump on the right side; and if not, it is determined that the user is wearing it on the left side. Additionally, if the user selects the breast pump 2, only the left side is equipped with an NFC tag in this case. After the user wears the breast pump, if the reported induction data includes NFC data, it is determined that the user is wearing the breast pump on the left side; and if the reported induction data does not include the NFC data, it is determined that the user is wearing it on the right side. That is, the position detection strategy here is to determine the presence or absence of the NFC data.

In some embodiments, a capacitive sensor is built in the breast pump 1, while an NFC acquisition device is built in the breast pump 2. An iron plate is disposed on the left side of the bra, and an NFC tag is provided on the right side. When a breast pump is worn on one side only, if the breast pump 1 acquires capacitance data, it is determined that the user is wearing the breast pump on the left side; and if no capacitance data is acquired, it is determined that the user is wearing it on the right side. If the breast pump 2 acquires NFC data, it is determined that the user is wearing the breast pump on the right side; and if no NFC data is acquired, it is determined that the user is wearing it on the left side. In this way, there is no need for the user to detach the breast pump. During use, the user only needs to properly wear the breast pump, and the wearing position detection can be completed automatically. The entire detection process is imperceptible to the user. This not only allows for automatic setting of the wearing position of the breast pump, improving the reliability of the milk expression data recorded by the breast pump, but also enhances the user experience.

Thus, the wearing position detection process for a breast pump according to the present disclosure is completed.

As described above, the present disclosure provides a wearing position detection method for a breast pump. The induction data of the bra corresponding to the target breast pump is obtained upon receiving the wearing completion operation for the target breast pump. The data type of the induction data is then determined. Finally, the wearing position detection result of the target breast pump is output based on the position detection strategy corresponding to the data type. In the wearing position detection method for a breast pump provided by the present disclosure, when the wearing completion operation for the target breast pump is received, the induction data of the bra corresponding to the target breast pump may be obtained. The wearing position detection result of the target breast pump is output via the position detection strategy corresponding to the induction data. Thus, the user does not need to manually set the wearing position of the breast pump after wearing the breast pump. As a result, the reliability of milk expression data recorded by the breast pump is improved.

An embodiment of the present disclosure provides a wearing position detection apparatus for a breast pump. The wearing position detection apparatus includes an inductor disposed on a bra and configured to interact inductively with a sensor disposed on a breast pump to detect whether a target breast pump is worn on a left breast or a right breast.

Correspondingly, referring to FIG. 3, an embodiment of the present disclosure provides a wearing position detection apparatus (hereinafter referred to as "detection apparatus") for a breast pump. The detection apparatus includes a bra body 201 and an inductor 202.

The bra body 201 is configured to be worn on a breast. A breast pump may be placed between the bra body 201 and the breast to express milk from the breast.

The inductor 202 is disposed on the bra body 201.

A controller is disposed on the breast pump body and configured to detect whether the breast pump is worn on the left breast or the right breast according to induction information of the inductor and the sensor.

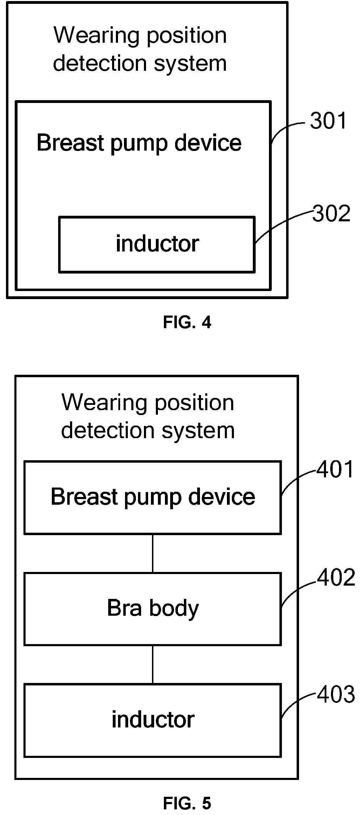

Optionally, referring to FIG. 4, an embodiment of the present disclosure provides a wearing position detection system for a breast pump. The wearing position detection system may specifically include a breast pump device 301 and an inductor 302.

The breast pump device 301 is configured to express breast milk and includes a sensor 302.

The inductor 302 is disposed on the bra and configured to interact inductively with the sensor to detect whether a target breast pump is worn on the left breast or the right breast.

Optionally, in some embodiments, the inductor 302 is configured to be disposed on the left side or the right side of the bra. The breast pump is configured to determine whether the breast pump is worn on the left breast or the right breast based on whether the sensor detects the inductor.

Optionally, in some embodiments, the inductor 302 is configured to be disposed in the middle position of the bra.

Optionally, referring to FIG. 5, an embodiment of the present disclosure provides a wearing position detection system for a breast pump. The wearing position detection system may specifically include a breast pump device 301, a bra body 302, and an inductor 303.

The breast pump device 301 is configured to express breast milk and includes a sensor.

The bra body 302 is configured to be worn on a breast. A breast pump may be placed between the bra body 302 and the breast to express milk from the breast.

The inductor 303 is disposed on the bra body 302 and configured to interact inductively with the sensor 301 to detect whether a target breast pump is worn on the left breast or the right breast.

In the wearing position detection method for a breast pump provided by the present disclosure, when the wearing completion operation for the target breast pump is received, the induction data of the bra corresponding to the target breast pump may be obtained. The wearing position detection result of the target breast pump is output via the position detection strategy corresponding to the induction data. Thus, the user does not need to manually set the wearing position of the breast pump after wearing the breast pump. As a result, the reliability of milk expression data recorded by the breast pump is improved.

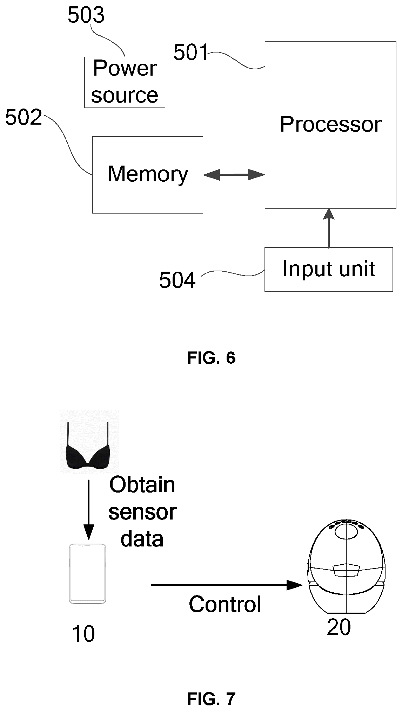

Furthermore, the present disclosure further provides an electronic device. FIG. 6 illustrates a schematic structural diagram of an electronic device involved in the present disclosure. Specifically, the electronic device may include components such as a processor 501 with one or more processing cores, a memory 502 with one or more computer-readable storage media, a power source 503, and an input unit 504. It will be understood by those skilled in the art that the structure of the electronic device as shown in FIG. 6 does not constitute a limitation on the electronic device, which may include more or less components than those shown in the figure, or some components are combined, or different component arrangements are provided.

The processor 501 is a control center of the electronic device, connects to various parts of the electronic device by using various interfaces and lines, and performs various functions and data processing of the electronic device by running or executing software programs and/or modules stored in the memory 502 and calling data stored in the memory 502, so as to perform overall monitoring on the electronic device. Optionally, the processor 501 may include one or more processing cores. Preferably, the processor 501 may be integrated with an application processor and a modulation and demodulation processor. The application processor is configured to mainly process an operating system, a user interface, an application program, and the like. The modulation and demodulation processor is configured to mainly process wireless communication. It will be understood that the modulation and demodulation processor may not be integrated into the processor 501.

The memory 502 may be configured to store a software program and a module, and the processor 501 is configured to run the software and the module stored in the memory 502 to perform various functional applications and data processing. The memory 502 may mainly include a program storage area and a data storage area. The program storage area may store an operating system, an application program required by at least one function (such as a sound playing function and an image playing function), and the like. The data storage area may store data created based on the use of the electronic device, and the like. Moreover, the memory 502 may include a high-speed random access memory, and may also include a non-volatile memory, for example, at least one magnetic disk storage device, a flash memory device, or other volatile solid storage device. Correspondingly, the memory 502 may further include a memory controller to provide access to the memory 502 by the processor 501.

The electronic device may further include the power source 503 that supplies power to various components. Preferably, the power source 503 can be logically connected to the processor 501 via a power management system. Thus, functions such as charging, discharging, and power consumption management may be achieved via the power management system. The power source 503 may further include any component such as one or more direct-current or alternating-current power sources, a recharging system, a power failure detection circuit, a power converter or inverter, and a power status indicator.

The electronic device may further include the input unit 504 that can be configured to receive entered numbers or character information, and generate keyboard, mouse, joystick, optical or trackball signal inputs associated with user settings and function control.

Although not shown, the electronic device may further include a display unit and the like, which will not be repeated herein. Specifically, in this embodiment, the processor 501 in the electronic device may load executable files corresponding to processes of one or more application programs into the memory 502 according to instructions as described below, and the application programs stored in the memory 502 may be run by the processor 501 to achieve various functions as follows:

obtaining the induction data of the target breast pump or the inductor upon receiving the wearing completion operation for the target breast pump; and determining the wearing position detection result of the target breast pump based on the induction data.

### Solution II:

For example, referring to FIG. 7, the present disclosure provides a wearing position detection apparatus for a breast pump. The wearing position detection apparatus for a breast pump is integrated in a terminal 10 such as a mobile phone, a tablet computer, or an independently configured operation control setup or a breast pump. The mobile phone 10 serves as a data receiving end and computing center. Human body data acquired by a sensor is obtained upon detecting that a target breast pump 10 is in a wearing state. Finally, a position wearing result of the target breast pump is determined based on the human body data. For example, it is determined that the breast pump 20 is worn on the left breast, or it is determined that the breast pump 20 is worn on the right breast. Optionally, in some embodiments of the present disclosure, the mobile phone 10 may issue a control instruction to control a working indicator lamp of the breast pump 20 to flash. For example, the working indicator lamp of the breast pump 20 is firstly controlled to flash a plurality of times, and the display screen of the mobile phone 10 displays: "The breast pump is worn on the left breast". Alternatively, the working indicator lamp of the breast pump 20 is controlled to flash a plurality of times, and the display screen of the mobile phone 10 displays: "The breast pump is worn on the right breast". In this way, the user can know whether the breast pump is capable of recording correct milk expression data and may confirm or correct the stored left/right wearing result through interface interaction.

According to the wearing position detection method for a breast pump provided by the present disclosure, when wearing the target breast pump, the user can automatically set the wearing position of the target breast pump according to the human body data acquired by the sensor. Thus, the reliability of the milk expression data recorded by the breast pump is improved.

A wearing position detection method for a breast pump provided by the present disclosure includes: obtaining human body data acquired by a sensor upon detecting that a target breast pump is in a wearing state; and determining a position wearing result of the target breast pump according to the human body data.

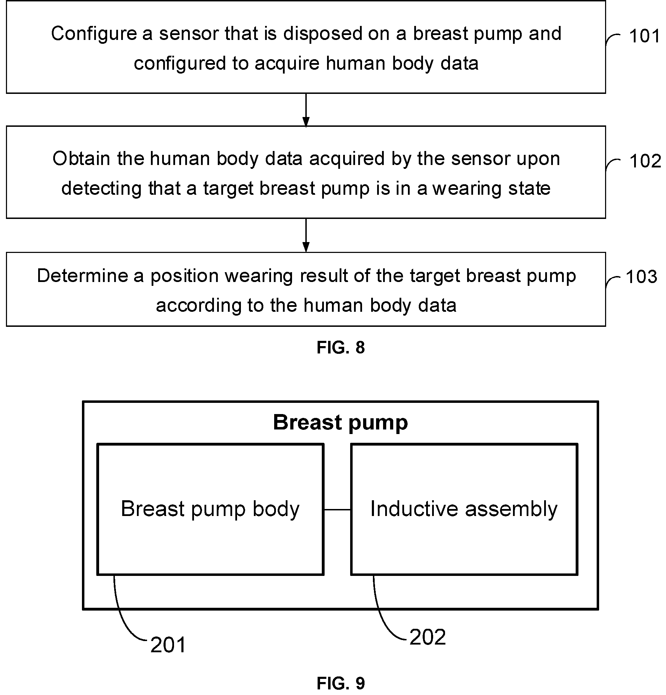

FIG. 8 is a flowchart of a wearing position detection method for a breast pump provided by the present disclosure. The specific process of the wearing position detection method for a breast pump may include the following steps.

In step 101, a sensor that is disposed on the breast pump is configured to acquire human body data.

In step 102, the human body data acquired by the sensor is obtained upon detecting that a target breast pump is in a wearing state.

The target breast pump refers to the breast pump currently being worn. The human body data refers to biometric data acquired by the sensor. The human body data may be data for characterizing human body information. Optionally, the human body data is at least one of blood oxygen concentration, heartbeat, breast shape, transmittance, body fat percentage, muscle mass, hardness, or elasticity data of a human body. The sensor may be disposed in the breast pump. For example, the sensor is disposed on a bra. Upon detecting that the target breast pump is in the wearing state, an acquisition instruction is issued to the sensor on the bra, and then the human body data acquired by the sensor is obtained. For another example, the sensor is disposed in the breast pump and is configured to acquire human body data in real time after the target breast pump is turned on. Upon detecting that the target breast pump is in the wearing state, the human body data acquired by the sensor may be obtained directly.

It will be appreciated that breast pumps are generally sold in pairs. Therefore, to detect whether breast pumps are worn on both sides, in some embodiments of the present disclosure, sensors may include a first sensor and a second sensor. The first sensor is mounted in the bra or the breast pump or the second sensor is mounted in the bra or the breast pump, which can be selected based on actual circumstances.

Optionally, the first sensor and the second sensor may be of the same type, for example, both the first sensor and the second sensor are infrared sensors. For example, specifically, infrared sensors are disposed on a breast pump a and a breast pump b, designated as a first infrared sensor and a second infrared sensor, respectively. When the user wears a breast pump on one side only, the first infrared sensor and the second infrared sensor disposed on the breast pump a and the breast pump b can respectively acquire infrared data. If the user wears the breast pump on the left side and uses the breast pump b, the human body data acquired by the first infrared sensor is null, while the human body data is acquired by the second infrared sensor. If the breast pump a is used, the human body data is acquired by the first infrared sensor, while the human body data acquired by the second infrared sensor is null.

It should be noted that, in the present disclosure, the principle of using infrared sensors to measure the human body data essentially involves measuring human body temperature. Methods for measuring human body temperature may be divided into two main categories: contact and non-contact. Contact sensors include thermocouples, thermistors, resistance temperature detectors (RTDs), and semiconductor temperature sensors, etc. The signals output by such sensors actually reflect changes in their own temperature. They need to be in full contact with the object being measured so that their temperatures become consistent. Non-contact temperature measurement operates based on the relationship between an object's thermal radiation and its temperature. Heat is typically transferred in three ways: heat conduction, convection, and thermal radiation. Thermal radiation is essentially electromagnetic waves of certain wavelengths ranging from 0.7 microns to 1000 microns. In practical use, infrared thermometers measure thermal radiation within the wavelength range of 0.7 microns to 14 microns, as most objects emit the strongest radiation within this range.

When an object absorbs energy (including thermal energy), its temperature rises, and it also radiates thermal energy. At thermal equilibrium, the absorbed thermal energy (Wa) equals the emitted thermal energy (We). The temperature of an object is reflected in its radiated thermal energy in two forms. An infrared sensor measures the radiant energy in two different wavelength bands and calculates the temperature of the object. This method is straightforward but can be influenced by the object's surface emissivity. Infrared sensors can measure an object's temperature based on the two principles described above. They typically include components such as an optical lens, an infrared detector, a signal amplifier and conditioner, an analog-to-digital converter (ADC), a result display, and an operation interface. Among these, the infrared sensor is the core of the sensor. It is divided into two main categories: one for temperature detection and one for photoelectric detection.

In infrared sensors, the field of view is an important concept. A value displayed by a thermometer reflects an average temperature within the field of view. Infrared sensors generally provide a ratio between the visual measurement distance and the radius of the field of view. The larger this ratio, the smaller the field of view of temperature measurement at the same temperature measurement distance, reflecting accurate temperature measurement position. Therefore, in some embodiments, the sensor is controlled to acquire the human body data only when it is detected that the target breast pump is in the wearing state and the breast shield of the target breast pump is in contact with the breast. Thus, the accuracy of detection can be ensured.

Optionally, both the first sensor and the second sensor are electrical signal sensors. The electrical signal sensors assess cardiac activity by measuring electrical signals on the body's surface. Compared to other sensors, electrical signal sensors offer advantages such as high accuracy, good reliability, and straightforward data processing. In scenarios where a breast pump is worn on one side only, the degree of heartbeat vibration in the left breast is significantly higher than that in the right breast. Therefore, wearing position detection can be performed based on this difference.

It should be noted that, for cases where breast pumps are worn on both sides, in the present disclosure, the position of the breast pump that is worn first is determined first. Once the position of this breast pump is determined, when the sensor of the other breast pump detects corresponding human body data, the position wearing result is directly generated. Optionally, in heartbeat detection scenarios, the first sensor and the second sensor may also be optical signal sensors. Optical signal sensors can be utilized to measure indicators such as heart rate and cardiovascular stroke volume power. They detect changes in blood flow by reflecting and absorbing light. Optical signal sensors do not require skin contact and cause no harm to the user's body.

Optionally, both the first sensor and the second sensor are blood oxygen sensors. The working principle of the blood oxygen sensors involves measuring blood oxygen concentration via an optical sensor. This is achieved by using a transmitter and a receiver built in the optical sensor to measure the absorption of emitted light in the blood. When light passes through blood, oxygen molecules in the blood absorb light of specific wavelengths. Therefore, the lower the blood oxygen concentration in the measured tissue, the more the light absorbed, and the lower the light intensity received by the receiver. It should be noted that, before using blood oxygen sensors to detect the wearing position, it is necessary to record the blood oxygen status of the left breast and that of the right breast using the blood oxygen sensor of any breast pump in advance. During subsequent wearing position detection, the detection can be performed based on the data recorded in advance.

Optionally, in some embodiments, the first sensor and the second sensor are sensors of the same type.

Optionally, in some embodiments, the first sensor and the second sensor are sensors of different types.

In step 103, a position wearing result of the target breast pump is determined according to the human body data.

In the present disclosure, the position wearing result of the target breast pump may be automatically set based on the difference between the human body data acquired in real time and the pre-stored human body data. For example, if the sensor is a blood oxygen sensor, the human body data is compared with pre-stored data. The pre-stored blood oxygen data is 95% for the left breast and 90% for the right breast. When the blood oxygen concentration in the human body data acquired in real time is 94%, it can be determined that the target breast pump is worn on the left breast. For another example, if the blood oxygen concentration in the human body data acquired in real time is 92.5%, which is the average of the pre-stored data, the target breast pump may be controlled to vibrate or announce prompting information: "Please manually set the position".

Optionally, in some embodiments, differences between different parts of the human body can also be utilized to automatically set the position wearing result of the target breast pump. For example, the sensor is an electrical signal sensor, and the heartbeat data acquired by the sensor from the left breast is noticeably different from that acquired from the right breast. For example, the vibration intensity of the heartbeat acquired from the left breast is higher than that of the heartbeat acquired from the right breast. Therefore, based on this, the wearing position of the target breast pump may be automatically set.

Optionally, in some embodiments, the sensor may be a laser sensor. Specifically, shape data of the user's left and right breasts may be pre-stored. During use, the wearing position of the target breast pump may be automatically set based on the shape difference between the left and right breasts of the human body.

Optionally, in some embodiments, the sensor may be an infrared or ultrasonic sensor. Specifically, data such as transmittance, body fat percentage, and muscle mass of the user's left and right breasts may be pre-stored. During use, the wearing position of the target breast pump may be automatically set based on differences in transmittance, body fat percentage, and muscle mass between the left and right breasts of the human body.

In other embodiments, the sensor may be a sensor for detecting elasticity or hardness. Specifically, data such as elasticity or hardness of the user's left and right breasts may be pre-stored. During use, the wearing position of the target breast pump may be automatically set based on differences in elasticity or hardness between the left and right breasts of the human body.

Optionally, in some embodiments, the first sensor and the second sensor may be of the same type or different types. Moreover, the first sensor may be composed of a plurality of sensors, and the second sensor may also be composed of a plurality of sensors. The plurality of sensors may be a plurality of identical sensors, or a plurality of different sensors, or a combination of some identical sensors and some different sensors. This may be specifically set based on actual circumstances.

Further, when the user wears breast pumps on both sides, the left/right wearing results of the breast pumps are automatically set based on the human body data acquired by the first sensor and the second sensor.

For example, the first sensor is disposed on a first breast pump, while the second sensor is disposed on a second breast pump. The first sensor and the second sensor are identical sensors, such as electrical signal sensors that are configured to detect the degree of heartbeat vibration. The actual wearing positions of the user are as follows: the first breast pump is worn on the left breast and the second breast pump is worn on the right breast. As can be seen from the acquired human body data, the degree of vibration of the first breast pump is greater than that of the second breast pump. Therefore, it is automatically set that the first breast pump is worn on the left side and the second breast pump is worn on the right side.

For another example, the first sensor is disposed on the first breast pump, while the second sensor is disposed on the second breast pump. The first sensor and the second sensor are different sensors. For example, the first sensor is an electrical signal sensor and the second sensor is a laser sensor. The actual wearing positions of the user are as follows: the first breast pump is worn on the left breast and the second breast pump is worn on the right breast. By comparing the acquired human body data with the pre-stored data, i.e., comparing pre-stored breast data with the data from the second sensor, it is determined that the data acquired by the second sensor corresponds to the right breast. Besides, the pre-stored degree of heartbeat vibration and the data acquired by the first sensor is the data of the left breast, and therefore, it may be automatically set that the first breast pump is worn on the left side and the second breast pump is worn on the right side.

Optionally, in some embodiments, a wearing position information prompt may be displayed on the terminal device or the breast pump.

Optionally, in some embodiments of the present disclosure, after the position wearing result is determined, the same or different control instructions may be issued to control the breast pump to perform operations such as light flashing, voice prompting, or vibrating, allowing the user to know whether the position of the currently worn breast pump displayed on the electronic device is consistent with that of the actual wearing position of the breast pump.

Further, the milk expression data corresponding to the breasts on different sides may be subsequently recorded according to the position wearing result.

Optionally, in some embodiments of the present disclosure, the target breast pump may be one that the user has previously used. That is, each breast pump has respective milk expression data recorded thereon. Therefore, to improve the reliability of subsequent milk expression data, after the position wearing result, the historical wearing result of each breast pump may be obtained to record the milk expression data corresponding to the breasts on different sides. That is, the step of recording the milk expression data corresponding to the breasts on different sides according to the position wearing result may specifically include:
obtaining historical wearing information of the target breast pump; and
recording the milk expression data corresponding to the breasts on different sides according to the historical wearing information and the position wearing result.

For example, if the first breast pump was historically worn on the left breast but is currently worn on the right breast, while the second breast pump was historically worn on the right breast but is currently worn on the left breast, the historical milk expression data of the first breast pump may be transferred to the milk expression data record corresponding to the second breast pump, and the historical milk expression data of the second breast pump may be transferred to the milk expression data record corresponding to the first breast pump. In this way, the recording of the milk expression data corresponding to the breasts on different sides is completed.

Optionally, in some embodiments of the present disclosure, the wearing position detection method provided by the present disclosure may further include:

updating milk expression data of the target breast pump stored in a database in response to a position altering operation for the target breast pump.

The position altering operation may be triggered by the electronic device. For example, the historical wearing information of the breast pump obtained by the electronic device is as follows: in a historical time period, the breast pump was correspondingly worn on the left breast; however, it is currently worn on the right breast. In this case, the position altering operation is triggered to transfer the historical milk expression data of the breast pump to the database corresponding to the other breast pump while transferring the database corresponding to the other breast pump to the current database, so as to update the milk expression data of the target breast pump stored in the database.

Thus, the wearing position detection process for a breast pump according to the present disclosure is completed.

As described above, the present disclosure provides a wearing position detection method for a breast pump. The sensor disposed on the breast pump is configured to acquire the human body data. The human body data acquired by the sensor is obtained upon detecting that the target breast pump is in the wearing state. Finally, the position wearing result of the target breast pump is determined according to the human body data. In the wearing position detection method for a breast pump provided by the present disclosure, when wearing the target breast pump, the user can automatically set the wearing position of the target breast pump according to the human body data acquired by the sensor. Thus, the reliability of the milk expression data recorded by the breast pump is improved.

Correspondingly, referring to FIG. 9, an embodiment of the present disclosure provides a breast pump including a breast pump body 201 and an inductive assembly 202.

The breast pump body 201 is configured to be worn on a breast to express milk from the breast.

Optionally, in some embodiments, a control panel for displaying a wearing position of the breast pump is disposed on the breast pump body 201.

The inductive assembly 202 is disposed on the breast pump body 201 and is configured to acquire human body data and determine a position wearing result of a target breast pump based on the human body data.

Optionally, in some embodiments, the inductive assembly 202 is configured to acquire physiological data of the human body and determine whether the target breast pump is worn on the left breast or the right breast based on the human body data.

Optionally, in some embodiments, the inductive assembly 202 may be configured to detect at least one of blood oxygen concentration, heartbeat, breast shape, transmittance, body fat percentage, muscle mass, hardness, or elasticity data of the human body.

Optionally, in some embodiments, the inductive assembly 202 is at least one of a capacitive sensor, an inductive sensor, a hardness sensor, an infrared sensor, and an ultrasonic sensor.

As described above, the present disclosure provides a breast pump including the breast pump body 201 and the inductive assembly 202, where the breast pump body 201 is configured to be worn on the breast to express milk from the breast; and the inductive assembly 202 is disposed on the breast pump body 201 and is configured to acquire human body data and determine the position wearing result of the target breast pump based on the human body data. In the wearing position detection solution for a breast pump provided by the present disclosure, when wearing the target breast pump, the user can automatically set the wearing position of the target breast pump according to the human body data acquired by the sensor. Thus, the reliability of the milk expression data recorded by the breast pump is improved.

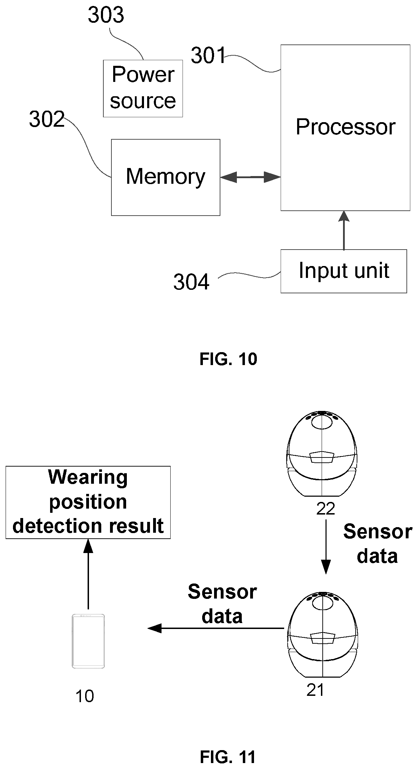

Furthermore, the present disclosure further provides an electronic device. FIG. 10 illustrates a schematic structural diagram of an electronic device involved in the present disclosure. Specifically, the electronic device may include components such as a processor 301 with one or more processing cores, a memory 302 with one or more computer-readable storage media, a power source 303, and an input unit 304. It will be understood by those skilled in the art that the structure of the electronic device as shown in FIG. 10 does not constitute a limitation on the electronic device, which may include more or less components than those shown in the figure, or some components are combined, or different component arrangements are provided.

The processor 301 is a control center of the electronic device, connects to various parts of the electronic device by using various interfaces and lines, and performs various functions and data processing of the electronic device by running or executing software programs and/or modules stored in the memory 302 and calling data stored in the memory 302, so as to perform overall monitoring on the electronic device. Optionally, the processor 301 may include one or more processing cores. Preferably, the processor 301 may be integrated with an application processor and a modulation and demodulation processor. The application processor is configured to mainly process an operating system, a user interface, an application program, and the like. The modulation and demodulation processor is configured to mainly process wireless communication. It will be understood that the modulation and demodulation processor may not be integrated into the processor 301.

The memory 302 may be configured to store a software program and a module, and the processor 301 is configured to run the software and the module stored in the memory 302 to perform various functional applications and data processing. The memory 302 may mainly include a program storage area and a data storage area. The program storage area may store an operating system, an application program required by at least one function (such as a sound playing function and an image playing function), and the like. The data storage area may store data created based on the use of the electronic device, and the like. Moreover, the memory 302 may include a high-speed random access memory, and may also include a non-volatile memory, for example, at least one magnetic disk storage device, a flash memory device, or other volatile solid storage device. Correspondingly, the memory 302 may further include a memory controller to provide access to the memory 302 by the processor 301.

The electronic device may further include the power source 303 that supplies power to various components. Preferably, the power source 303 can be logically connected to the processor 301 via a power management system. Thus, functions such as charging, discharging, and power consumption management may be achieved via the power management system. The power source 303 may further include any component such as one or more direct-current or alternating-current power sources, a recharging system, a power failure detection circuit, a power converter or inverter, and a power status indicator.

The electronic device may further include the input unit 304 that can be configured to receive entered numbers or character information, and generate keyboard, mouse, joystick, optical or trackball signal inputs associated with user settings and function control.

Although not shown, the electronic device may further include a display unit and the like, which will not be repeated herein. Specifically, in this embodiment, the processor 301 in the electronic device may load executable files corresponding to processes of one or more application programs into the memory 302 according to instructions as described below, and the application programs stored in the memory 302 may be run by the processor 301 to achieve various functions as follows:

configuring a sensor disposed on a breast pump to acquire human body data; obtaining the human body data acquired by the sensor upon detecting that a target breast pump is in a wearing state; and determining a position wearing result of the target breast pump according to the human body data.

### Solution III:

For example, referring to FIG. 11, the present disclosure provides a wearing position detection apparatus for a breast pump. The wearing position detection apparatus for a breast pump is integrated into a mobile phone 10. When the user wears breast pumps on both breasts (e.g., a first breast pump 21 and a second breast pump 22), the mobile phone 10 serves as a data receiving end and computing center. Upon detecting that the user has triggered a two-side wearing operation, sensor data reported by the first breast pump 21 is received, where the sensor data is data of an inductor of the second breast pump 22 that is acquired by a sensor disposed on the first breast pump 21. According to the sensor data, the mobile phone 10 determines the position wearing results of the first breast pump 21 and the second breast pump 22, for example, determines that the first breast pump 21 is worn on the left breast and the second breast pump 22 is worn on the right breast 22. Optionally, in some embodiments of the present disclosure, the mobile phone 10 may issue a control instruction to control working indicator lamps of the first breast pump 21 and the second breast pump 22 to flash. For example, the working indicator lamp of the first breast pump 21 is firstly controlled to flash a plurality of times, and the display screen of the mobile phone 10 displays: "The breast pump is worn on the left breast". Then, the working indicator lamp of the second breast pump 22 is controlled to flash a plurality of times, and the display screen of the mobile phone 10 displays: "The breast pump is worn on the right breast". In this way, the user can know whether the breast pump is capable of recording correct milk expression data and may confirm or correct the stored left/right wearing result through interface interaction.

In the wearing position detection method for a breast pump provided by the present disclosure, when the user wears the breast pumps on the breasts on both sides, the data of the inductor of the second breast pump is acquired by using the sensor disposed in the first breast pump. Based on the sensor data, the wearing positions of the first breast pump and the second breast pump can be identified automatically, thereby improving the reliability of the milk expression data recorded by the breast pump.

The present disclosure provides a wearing position detection method including: when a two-side wearing operation is detected, receiving sensor data reported by a first breast pump; and determining position wearing results of the first breast pump and the second breast pump according to the sensor data.

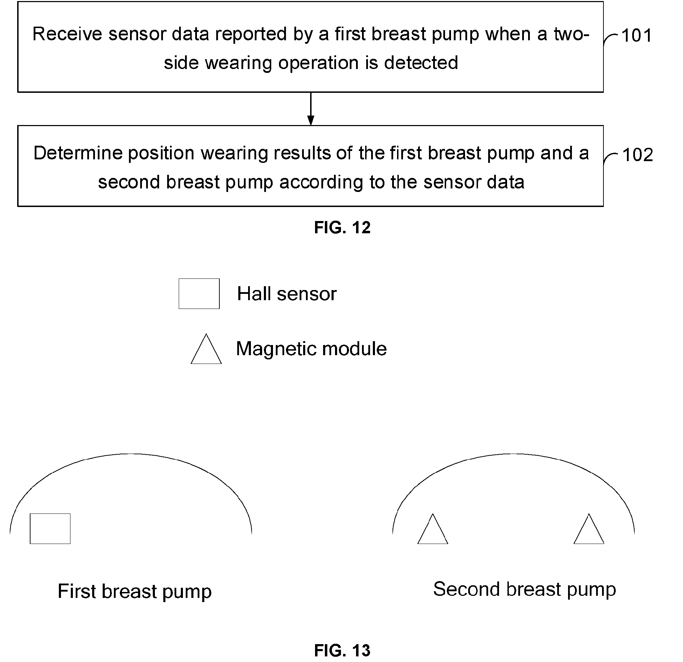

FIG. 12 is a flowchart of a wearing position detection method for a breast pump provided by the present disclosure. The specific process of the wearing position detection method for a breast pump may include the following steps.

In step 101, when a two-side wearing operation is detected, sensor data reported by a first breast pump is received.

The sensor data is induction data between a sensor disposed on the first breast pump and an inductor on a second breast pump. Generally, breast pumps are configured in pairs to express milk from a mother's left and right breasts, respectively. That is, the breast pump purchased by the user may include a first breast pump and a second breast pump. It should be noted that, in the present disclosure, the breast pump provided with a sensor is referred to as the first breast pump, while the breast pump provided with an inductor is referred to as the second breast pump. Optionally, in some embodiments, the first breast pump may be provided with only a sensor, and the second breast pump may be provided with only an inductor. Certainly, in some other embodiments, the first breast pump may be provided with both a sensor and an inductor, while the second breast pump may be provided with only an inductor.

Optionally, the sensor may be a hall sensor, and the inductor may be a magnetic module. For example, the hall sensor is disposed on the left side or the right side of the first breast pump, and the magnetic module is disposed on the left side or the right side of the second breast pump. Alternatively, any other types of sensors and inductive elements may be used, such as a capacitive sensor and an ultra-wideband sensor.

### Embodiment 1

Optionally, a hall sensor may be disposed on the left side of the first breast pump, and a magnetic module may be disposed on the right side of the second breast pump. When a two-side wearing operation is detected, if mutual inductive interaction is detected between the hall sensor and the magnetic module, it is determined that the first breast pump is worn on the right breast and the second breast pump is worn on the left breast. If the sensor data reported by the first breast pump is null, it is determined that the first breast pump is worn on the left breast and the second breast pump is worn on the right breast, as shown in FIG. 13.

Optionally, the hall sensor may be disposed on the right side of the first breast pump, and the magnetic module may be disposed on the right side of the second breast pump. When the two-side wearing operation is detected, if mutual inductive interaction is detected between the hall sensor and the magnetic module, it is determined that the first breast pump is worn on the left breast and the second breast pump is worn on the right breast. If the sensor data reported by the first breast pump is null, it is determined that the first breast pump is worn on the right breast and the second breast pump is worn on the left breast.

### Embodiment 2

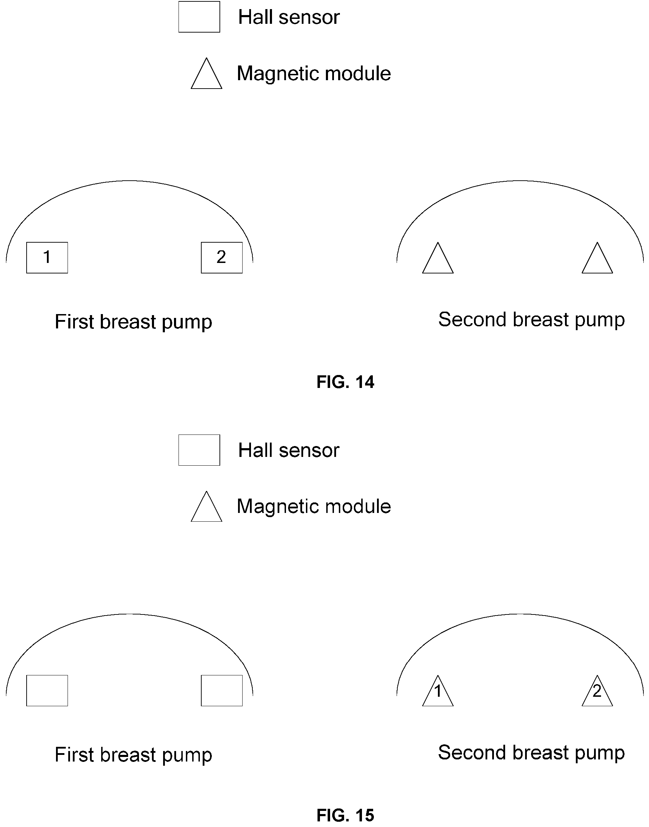

Optionally, hall sensors may be disposed on both the left and right sides of the first breast pump, with the two hall sensors configured distinctly as a first hall sensor and a second hall sensor. A magnetic module may be disposed on the second breast pump. For example, the first hall sensor is disposed on the left side of the first breast pump, while the second hall sensor is disposed on the right side of the same, and the magnetic module is disposed on the second breast pump. When a two-side wearing operation is detected, if mutual inductive interaction is detected between the second hall sensor and the magnetic module, it is determined that the first breast pump is worn on the left breast and the second breast pump is worn on the right breast. If mutual inductive interaction is detected between the first hall sensor and the magnetic module, it is determined that the first breast pump is worn on the right breast and the second breast pump is worn on the left breast, as shown in FIG. 14.

### Embodiment 3

Optionally, magnetic modules may be disposed on both the left and right sides of the first breast pump, with the two magnetic modules configured distinctly as a first magnetic module and a second magnetic module. A hall sensor is disposed on the second breast pump. For example, the first magnetic module is disposed on the left side of the first breast pump, while the second magnetic module is disposed on the right side of the same, and the hall sensor is disposed on the second breast pump. When a two-side wearing operation is detected, if mutual inductive interaction is detected between the hall sensor and the first magnetic module, it is determined that the first breast pump is worn on the left breast and the second breast pump is worn on the right breast. If mutual inductive interaction is detected between the hall sensor and the second magnetic module, it is determined that the first breast pump is worn on the right breast and the second breast pump is worn on the left breast, as shown in FIG. 15.

### Embodiment 4

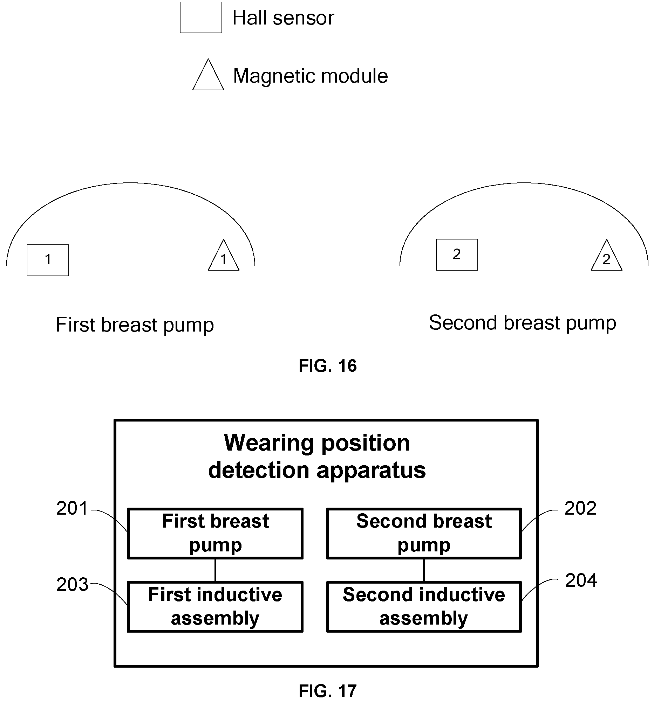

Optionally, hall sensors may be disposed on both the left sides of the first breast pump and the second breast pump, with the two hall sensors configured distinctly as a first hall sensor and a second hall sensor. Magnetic modules may be disposed on both the right sides of the first breast pump and the second breast pump, with the two magnetic modules configured distinctly as a first magnetic module and a second magnetic module. For example, the first hall sensor is disposed on the left side of the first breast pump, while the second hall sensor is disposed on the left side of the second breast pump, and the first magnetic module is disposed on the right side of the first breast pump, while the second magnetic module is disposed on the right side of the second breast pump. When a two-side wearing operation is detected, if mutual inductive interaction is detected between the first magnetic module and the second hall sensor, it is determined that the first breast pump is worn on the left breast and the second breast pump is worn on the right breast. If mutual inductive interaction is detected between the first hall sensor and the second magnetic module, it is determined that the first breast pump is worn on the right breast and the second breast pump is worn on the left breast. Alternatively, for example, the first hall sensor is disposed on the right side of the first breast pump, while the second hall sensor is disposed on the right side of the second breast pump, and the first magnetic module is disposed on the left side of the first breast pump, while the second magnetic module is disposed on the left side of the second breast pump, as shown in FIG. 16.

The combination of the hall sensors and the magnetic modules described above is merely an example, and any other sensors and inductive elements capable of mutual detection may also be used.

Additionally, the inductor may also be configured as a magnetic member, and a hall sensor is disposed on the left/right side of the first breast pump. For specific inductive interaction methods, reference may be made to the approaches described above.

Optionally, in some embodiments, the sensor disposed on the first breast pump may alternatively be a capacitive sensor. The capacitive sensor is a conversion device that uses various types of capacitors as sensing elements to transform measured physical or mechanical quantities into changes in capacitance. Essentially, it is a capacitor with variable parameters. Specifically, it may be disposed on the left side or the right side of the first breast pump, and the inductor may be an iron plate or an iron block. During use, the capacitive sensor acquires data from the iron plate.

Optionally, in some embodiments, the sensor disposed on the first breast pump may alternatively be an ultra wide band (UWB) sensor, and the sensor disposed on the second breast pump may alternatively be an UWB sensor. UWB technology is a wireless carrier communication technology that does not use sinusoidal carriers but instead uses nanosecond-level non-sinusoidal narrow pulses to transmit data, thus occupying a very wide spectrum range. Based on this, a relative position between the first breast pump and the second breast pump may be determined through phase-difference-of-arrival (PDOA) ranging.

For example, during two-side wearing use, the UWB sensor disposed on the first breast pump may serve as a reference sensor, which is configured to determine the relative positional relationship between the UWB sensor itself and the second breast pump. Subsequently, this data may be utilized for wearing position detection.

Optionally, in some embodiments, the sensor may alternatively be a signal acquisition device, and the inductor may be a signal sensor, where the inductors are respectively disposed on the left and right sides of the second breast pump and are configured to emit a first signal and a second signal, respectively, for example, emit the first signal on the left side and the second signal on the right side, with the first signal being different from the second signal. When the user wears the breast pump, the first breast pump may acquire either the first signal or the second signal to facilitate subsequent determination of the wearing position.

Furthermore, the user may wear a breast pump on only one breast or wear breast pumps on both breasts simultaneously. That is, optionally, in some embodiments, prior to step 101, the wearing position detection method for a breast pump provided in the present disclosure may further include:
detecting whether a number of currently worn breast pumps is a preset value; and
if detecting that the number of currently worn breast pumps is the preset value, triggering a two-side wearing operation.

The preset value is 2. That is, if it is detected that the number of currently worn breast pumps is 2, the two-side wearing operation is triggered. Specifically, when a wearing instruction transmitted from the first breast pump and a wearing instruction transmitted from the second breast pump are received, the two-side wearing operation is triggered. The wearing instruction transmitted from the first breast pump may be received first, or the wearing instruction transmitted from the second breast pump may be received first.

If it is detected that the number of currently worn breast pumps is not the preset value, a single-side wearing operation is triggered. In this case, in response to the single-side wearing operation, prompting information for the wearing position is output.

It should be noted that, after the prompting information is output, if a wearing operation for the other breast is received, the prompting information is hidden or closed. In this case, the process proceeds to the wearing position detection flow, thereby completing the automatic setting of the wearing position.

In step 102, position wearing results of the first breast pump and the second breast pump are determined according to the sensor data.

As described above, the position of the sensor on the first breast pump and the position of the inductor on the second breast pump may be set based on actual circumstances. Therefore, after the sensor data is received, it is also necessary to determine a mounting position of the sensor and a mounting position of the inductor and perform position detection in combination with the sensor data. That is, optionally, in some embodiments, step 102 may specifically include:
determining the mounting position of the sensor and the mounting position of the inductor, or determining a relative position of the sensor assembly to the inductive assembly.

The determining a relative position of the sensor assembly to the inductive assembly includes:
determining the relative position based on whether the sensor assembly has detected the inductive assembly; and/or
determining the relative position according to a signal strength detected by the sensor assembly regarding the inductive assembly.

The inductive assembly includes a first inductor and a second inductor, and the determining a relative position of the induced assembly to the inductive assembly includes:
when the sensor assembly has detected the first inductor, determining that the sensor assembly is close to the first inductor; and
when the sensor assembly has detected the second inductor, determining that the sensor assembly is close to the second inductor.

The sensor assembly includes a first sensor and a second sensor, and the determining a relative position of the sensor assembly to the inductive assembly includes:
when the first sensor has detected an inductor, determining that the inductor is close to the first sensor; and
when the second sensor has detected an inductor, determining that the inductor is close to the second sensor.

The position wearing results of the first breast pump and the second breast pump are determined based on the sensor data, the mounting position of the sensor, and the mounting position of the inductor.

For example, a hall sensor is disposed on the left side of the first breast pump, and a strongly magnetic module is disposed on the left side of the second breast pump. When a two-side wearing operation is detected, the actual wearing manner of the user is as follows: the first breast pump is worn on the left breast, and the second breast pump is worn on the right breast. In this case, the sensor data reported by the first breast pump is null. That is, the Hall sensor does not detect the magnetic module. In this case, in combination with the mounting position of the hall sensor and the mounting position of the strongly magnetic module, it may be determined that the first breast pump is worn on the left breast and the second breast pump is worn on the right breast.

For example, a hall sensor is disposed on the left side of the first breast pump, and a strongly magnetic module is disposed on the left side of the second breast pump. When a two-side wearing operation is detected, the actual wearing manner of the user is as follows: the first breast pump is worn on the right breast, and the second breast pump is worn on the left breast. In this case, the sensor data reported by the first breast pump is a strongly magnetic signal. That is, the Hall sensor and the magnetic module interact inductively with each other. In this case, in combination with the mounting position of the hall sensor and the mounting position of the strongly magnetic module, it is determined that the first breast pump is worn on the right breast and the second breast pump is worn on the left breast.

For example, a hall sensor is disposed on the left side of the first breast pump, and an iron plate is disposed on the right side of the second breast pump. When a two-side wearing operation is detected, the actual wearing manner of the user is as follows: the first breast pump is worn on the right breast, and the second breast pump is worn on the left breast. In this case, the sensor data reported by the first breast pump is a detected strongly magnetic signal. In this case, in combination with the mounting position of the hall sensor and the mounting position of the strongly magnetic module, it may be determined that the first breast pump is worn on the right breast and the second breast pump is worn on the left breast.

For example, a hall sensor is disposed on the left side of the first breast pump, and a magnetic module is disposed on the right side of the second breast pump. When a two-side wearing operation is detected, the actual wearing manner of the user is as follows: the first breast pump is worn on the right breast, and the second breast pump is worn on the left breast. If no mutual inductive interaction is detected between the hall sensor and the magnetic module, it is determined that the first breast pump is worn on the left breast and the second breast pump is worn on the right breast.

For example, a hall sensor is disposed on the right side of the first breast pump, and a magnetic module is disposed on the right side of the second breast pump. When a two-side wearing operation is detected, the actual wearing manner of the user is as follows: the first breast pump is worn on the right breast, and the second breast pump is worn on the left breast. If no mutual inductive interaction is detected between the hall sensor and the magnetic module, it is determined that the first breast pump is worn on the right breast and the second breast pump is worn on the left breast.

For example, hall sensors are disposed on both the left and right sides of the first breast pump, with the two hall sensors configured distinctly as a first hall sensor and a second hall sensor. A magnetic module is disposed on the second breast pump. When a two-side wearing operation is detected, the actual wearing manner of the user is as follows: the first breast pump is worn on the right breast, and the second breast pump is worn on the left breast. If mutual inductive interaction is detected between the hall sensor and the magnetic module, it is determined that the first breast pump is worn on the right breast and the second breast pump is worn on the left breast.

For example, magnetic modules are disposed on both the left and right sides of the first breast pump, with the two magnetic modules configured distinctly as a first magnetic module and a second magnetic module. A hall sensor is disposed on the second breast pump. For example, the first magnetic module is disposed on the left side of the first breast pump, while the second magnetic module is disposed on the right side of the same, and the hall sensor is disposed on the second breast pump. When a two-side wearing operation is detected, the actual wearing manner of the user is as follows: the first breast pump is worn on the left breast, and the second breast pump is worn on the right breast. If mutual inductive interaction is detected between the hall sensor and the magnetic module, it is determined that the first breast pump is worn on the right breast and the second breast pump is worn on the left breast.

For example, hall sensors are disposed on both the left sides of the first breast pump and the second breast pump, with the two hall sensors configured distinctly as a first hall sensor and a second hall sensor. Magnetic modules are disposed on both the right sides of the first breast pump and the second breast pump, with the two magnetic modules configured distinctly as a first magnetic module and a second magnetic module. When a two-side wearing operation is detected, the actual wearing manner of the user is as follows: the first breast pump is worn on the left breast, and the second breast pump is worn on the right breast. If mutual inductive interaction is detected between the hall sensor and the second magnetic module, it is determined that the first breast pump is worn on the right breast and the second breast pump is worn on the left breast.

It will be understood that the aforementioned different mounting positions of sensors and inductors correspond to different position detection strategies. That is, optionally, in some embodiments, the step of determining the position wearing results of the first breast pump and the second breast pump based on the sensor data, the mounting position of the sensor, and the mounting position of the inductor may specifically include:
determining a current position detection strategy based on the mounting position of the sensor and the mounting position of the inductor; and
determining the position wearing results of the first breast pump and the second breast pump according the sensor data and the position detection strategy.

In step 103, optionally, in some embodiments, after the position wearing result is determined, the same or different control instructions may be issued to control the breast pump to perform operations such as light flashing, voice prompting, or vibrating, allowing the user to know whether the position of the currently worn breast pump displayed on the electronic device is consistent with that of the actual wearing position of the breast pump. For example, a prompt tone for incorrect wearing is played by the current device or the breast pump. Specifically, a prompt "The breast shield is not properly fitted to the breast" or "The nipple is not aligned with the center of the breast shield" may be announced. Alternatively, the current device or the breast pump may be controlled to vibrate. Alternatively, at least two or more of the above-mentioned prompts may be performed simultaneously.

In step 104, in some embodiments, the stored left/right wearing result may be confirmed or corrected through interface interaction.

Further, the milk expression data corresponding to the breasts on different sides may be subsequently recorded according to the position wearing results.

It will be understood that the first breast pump and the second breast pump might be breast pumps that the user has previously used. That is, each breast pump has respective milk expression data recorded thereon. Therefore, to improve the reliability of subsequent milk expression data, after the position wearing result, the historical wearing result of each breast pump may be obtained to record the milk expression data corresponding to the breasts on different sides. That is, optionally, in some embodiments, the step of recording the milk expression data corresponding to the breasts on different sides according to the position wearing results may specifically include:
obtaining historical wearing results corresponding to the first breast pump and the second breast pump; and
recording the milk expression data corresponding to the breasts on different sides according to the position wearing results and the historical wearing results corresponding to the first breast pump and the second breast pump.

For example, if the first breast pump was historically worn on the left breast but is currently worn on the right breast, while the second breast pump was historically worn on the right breast but is currently worn on the left breast, the historical milk expression data of the first breast pump may be transferred to the milk expression data record corresponding to the second breast pump, and the historical milk expression data of the second breast pump may be transferred to the milk expression data record corresponding to the first breast pump. In this way, the recording of the milk expression data corresponding to the breasts on different sides is completed.

Thus, the wearing position detection process for a breast pump according to the present disclosure is completed.

As described above, the present disclosure provides a wearing position detection method for a breast pump. When the two-side wearing operation is detected, the sensor data reported by the first breast pump is received, where the sensor data is data of the inductor of the second breast pump that is acquired by the sensor built in the first breast pump. The position wearing results of the first breast pump and the second breast pump are determined according to the sensor data. In the wearing position detection solution for a breast pump provided by the present disclosure, when the user wears the breast pumps on the breasts on both sides, the data of the inductor of the second breast pump is acquired by using the sensor disposed in the first breast pump. Based on the sensor data, the wearing positions of the first breast pump and the second breast pump can be set automatically, thereby improving the reliability of the milk expression data recorded by the breast pump.

Correspondingly, referring to FIG. 17, an embodiment of the present disclosure provides a wearing position detection apparatus/system (hereinafter referred to as "detection apparatus") for a breast pump. The detection apparatus includes a first breast pump 201, a second breast pump 202, a first inductive assembly 203, and a second inductive assembly 204.

The first inductive assembly 203 is disposed on the first breast pump 201.

The second inductive assembly 204 is disposed on the second breast pump 202. The first inductive assembly 203 and the second inductive assembly 204 are configured to interact inductively with each other to detect left/right wearing positions of the first breast pump 201 and the second breast pump 202. A controller is disposed on the first breast pump or the second breast pump.

The sensor assembly is configured to acquire induction data about the inductive assembly.

The controller is configured to determine position wearing results of the first breast pump and the second breast pump according to the induction data.

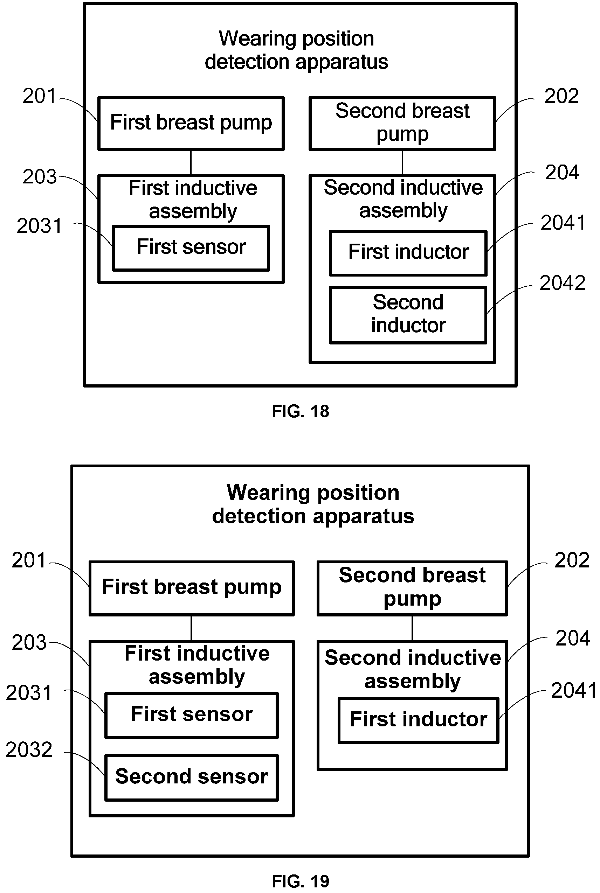

Optionally, in some embodiments, referring to FIG. 18, the first inductive assembly 203 may include a first sensor 2031, and the second inductive assembly may include a first inductor 2041 and a second inductor 2042, where the first sensor 2031 is arranged on the first breast pump 201, while the first inductor 2041 and the second inductor 2042 are arranged on the second breast pump 202, with the first inductor 2041 being different from the second inductor 2042. The first sensor 2031 may detect the first inductor 2041 and the second inductor 2042.

Optionally, in some embodiments, referring to FIG. 19, the first inductive assembly 203 may include a first sensor 2031 and a second sensor 2032, with the first sensor 2031 being different from the second sensor 2032. The second inductive assembly 204 includes a first inductor 2041, with the first inductor 2041 being arranged on the second breast pump 202. The first sensor 2031 and the second sensor 2032 may separately detect the first inductor 2041.

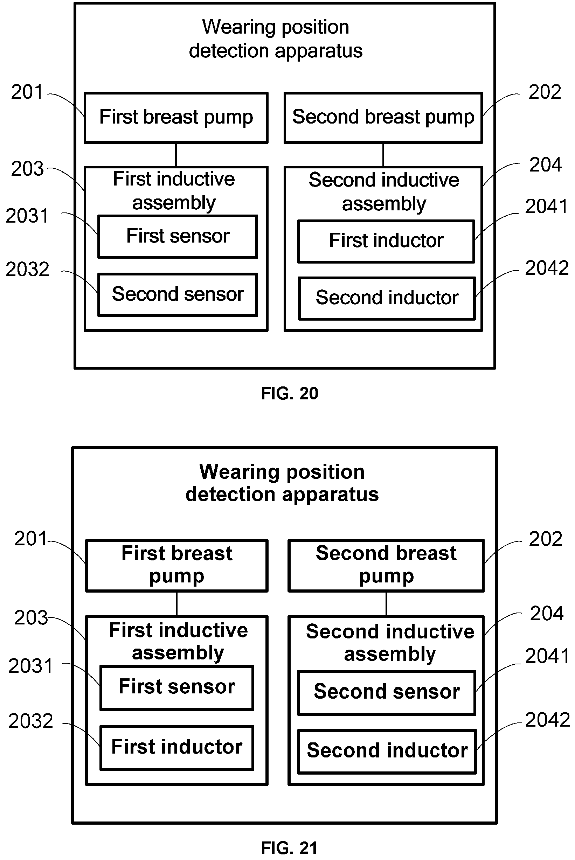

Optionally, in some embodiments, referring to FIG. 20, the first inductive assembly 203 may include a first sensor 2031 and a second sensor 2032, with the first sensor 2031 being different from the second sensor 2032. The second inductive assembly may include a first inductor 2041 and a second inductor 2042, with the first inductor 2041 being different from the second inductor 2042.

Optionally, in some embodiments, referring to FIG. 21, the first inductive assembly 203 may include a first sensor 2031 and a first inductor 2032, and the second inductive assembly 204 may include a second sensor 2041 and a second inductor 2042. The first inductor 2032 is different from the second inductor 2042, and the first sensor 2031 is different from the second sensor 2041. The first sensor 2031 may detect the first inductor 2032, and the second sensor 2041 may detect the second inductor 2042. Alternatively, the first sensor 2031 may detect the second inductor 2042, and the second sensor 2041 may detect the first inductor 2032. A controller is disposed on the first breast pump or the second breast pump.

The first sensor 2031 is configured to acquire first induction data about the first inductor 2032 and/or the second inductor 2042.

The second sensor 2041 is configured to acquire second induction data about the first inductor 2032 and/or the second inductor 2042.

The controller is configured to determine position wearing results of the first breast pump and the second breast pump according to the first induction data and/or the second induction data.

As described above, the present disclosure provides a wearing position detection apparatus for a breast pump. When the two-side wearing operation is detected, the processing module receives the sensor data reported by the first breast pump, where the sensor data is the induction data between the first inductive assembly 203 disposed on the first breast pump 201 and the second breast pump 202; and the position wearing results of the first breast pump 201 and the second breast pump 202 are determined according to the sensor data. In the wearing position detection solution for a breast pump provided by the present disclosure, when the user wears the breast pumps on the breasts on both sides, the data of the inductor of the second breast pump is acquired by using the sensor disposed in the first breast pump. Based on the sensor data, the wearing positions of the first breast pump and the second breast pump can be set automatically, thereby improving the reliability of the milk expression data recorded by the breast pump.

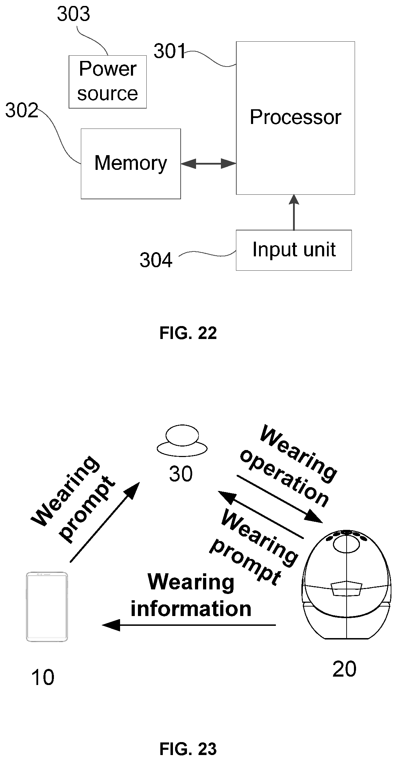

Furthermore, the present disclosure further provides an electronic device. FIG. 22 illustrates a schematic structural diagram of an electronic device involved in the present disclosure. Specifically, the electronic device may include components such as a processor 301 with one or more processing cores, a memory 302 with one or more computer-readable storage media, a power source 303, and an input unit 304. It will be understood by those skilled in the art that the structure of the electronic device as shown in FIG. 22 does not constitute a limitation on the electronic device, which may include more or less components than those shown in the figure, or some components are combined, or different component arrangements are provided.

The processor 301 is a control center of the electronic device, connects to various parts of the electronic device by using various interfaces and lines, and performs various functions and data processing of the electronic device by running or executing software programs and/or modules stored in the memory 302 and calling data stored in the memory 302, so as to perform overall monitoring on the electronic device. Optionally, the processor 301 may include one or more processing cores. Preferably, the processor 301 may be integrated with an application processor and a modulation and demodulation processor. The application processor is configured to mainly process an operating system, a user interface, an application program, and the like. The modulation and demodulation processor is configured to mainly process wireless communication. It will be understood that the modulation and demodulation processor may not be integrated into the processor 301.

The memory 302 may be configured to store a software program and a module, and the processor 301 is configured to run the software and the module stored in the memory 302 to perform various functional applications and data processing. The memory 302 may mainly include a program storage area and a data storage area. The program storage area may store an operating system, an application program required by at least one function (such as a sound playing function and an image playing function), and the like. The data storage area may store data created based on the use of the electronic device, and the like. Moreover, the memory 302 may include a high-speed random access memory, and may also include a non-volatile memory, for example, at least one magnetic disk storage device, a flash memory device, or other volatile solid storage device. Correspondingly, the memory 302 may further include a memory controller to provide access to the memory 302 by the processor 301.

The electronic device may further include the power source 303 that supplies power to various components. Preferably, the power source 303 can be logically connected to the processor 301 via a power management system. Thus, functions such as charging, discharging, and power consumption management may be achieved via the power management system. The power source 303 may further include any component such as one or more direct-current or alternating-current power sources, a recharging system, a power failure detection circuit, a power converter or inverter, and a power status indicator.

The electronic device may further include the input unit 304 that can be configured to receive entered numbers or character information, and generate keyboard, mouse, joystick, optical or trackball signal inputs associated with user settings and function control.

Although not shown, the electronic device may further include a display unit and the like, which will not be repeated herein. Specifically, in this embodiment, the processor 301 in the electronic device may load executable files corresponding to processes of one or more application programs into the memory 302 according to instructions as described below, and the application programs stored in the memory 302 may be run by the processor 301 to achieve various functions as follows:
when a two-side wearing operation is detected, receiving sensor data reported by a first breast pump; and determining position wearing results of the first breast pump and the second breast pump according to the sensor data.

Regarding to specific implementations of the above various operations, see the foregoing embodiments, which will not be repeated herein.

In the embodiments of the present disclosure, when the two-side wearing operation is detected, the sensor data reported by the first breast pump is received, where the sensor data is data of the inductor of the second breast pump that is acquired by the sensor built in the first breast pump. The position wearing results of the first breast pump and the second breast pump are determined according to the sensor data. In the wearing position detection solution for a breast pump provided by the present disclosure, when the user wears the breast pumps on the breasts on both sides, the data of the inductor of the second breast pump is acquired by using the sensor disposed in the first breast pump. Based on the sensor data, the wearing positions of the first breast pump and the second breast pump can be set automatically, thereby improving the reliability of the milk expression data recorded by the breast pump.

Those of ordinary skill in the art will understand that all or part of the steps in the methods of the above embodiments may be implemented with instructions or by controlling related hardware via instructions. The instructions may be stored in a computer-readable storage medium and loaded and executed by a processor.

To this end, the present disclosure provides a storage medium having a plurality of instructions stored therein that can be loaded by a processor to perform the steps of any wearing position detection method for a breast pump provided by the present disclosure. For example, the instructions may cause the following steps to be performed:
when a two-side wearing operation is detected, receiving sensor data reported by a first breast pump; and determining position wearing results of the first breast pump and the second breast pump according to the sensor data.

### Solution IV:

For example, referring to FIG. 23, the present disclosure provides a wearing prompting system for a breast pump. The wearing prompting system for a breast pump is integrated into a mobile phone 10. The mobile phone 10 may connect to the breast pump 20 wirelessly and/or via Bluetooth. For example, the user 30 may pre-install an application program corresponding to the breast pump 20 on the mobile phone 10. Subsequently, the mobile phone 10 can establish a communication connection with the breast pump 20 through the application program, thereby enabling the upload of the milk expression data generated by the breast pump 20 to the mobile phone 10. Specifically, after the user activates the breast pump 20 and establishes a communication connection between the breast pump 20 and the mobile phone 10, the sensor of the breast pump 20 may upload the data acquired in real time to the mobile phone 10, thereby obtaining wearing information corresponding to the breast pump. Subsequently, the mobile phone 10 determines a prompting instruction corresponding to the wearing information, such as a correct wearing prompting instruction or an incorrect wearing prompting instruction. Finally, the mobile phone 10 may control, according to the prompting instruction, the breast pump 20 and/or the mobile phone 10 to execute a corresponding prompting event.

In the wearing prompting solution for a breast pump provided by the present disclosure, the corresponding prompting instruction is determined according to the wearing information acquired by the sensor of the breast pump. The breast pump and/or the current device is controlled to execute the prompting event according to the prompting instruction so that the user can obtain the actual wearing status of the breast pump. This avoids the problem that the user is unable to express the breast milk when using the breast pump due to incorrect wearing.

The present disclosure provides a wearing prompting method for a breast pump, including: obtaining wearing information corresponding to the breast pump in response to a wearing operation triggered for the breast pump; determining a prompting instruction corresponding to the wearing information; and controlling the breast pump and/or a current device to execute a prompting event according to the prompting instruction.

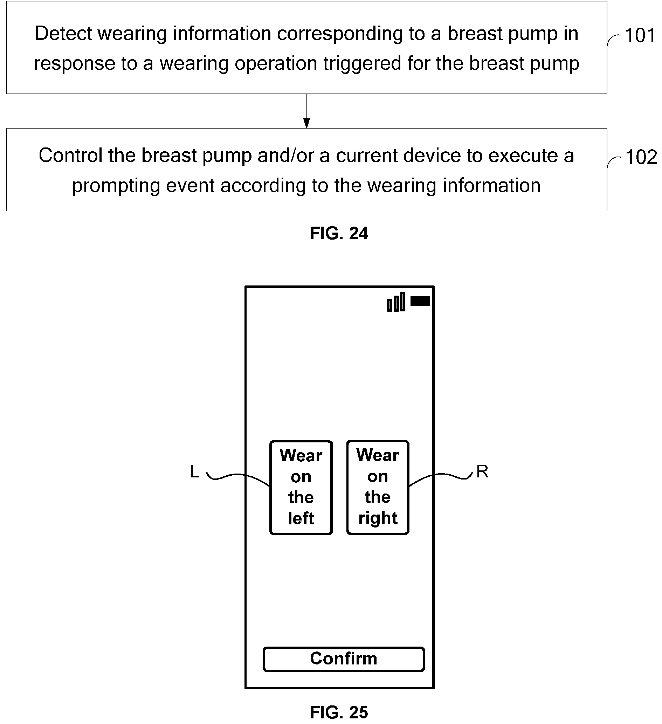

FIG. 24 is a flowchart of a wearing prompting method for a breast pump provided by the present disclosure. The specific process of the wearing prompting method for a breast pump may include the following steps.

In step 101, wearing information corresponding to the breast pump is obtained in response to a wearing operation triggered for the breast pump.

The wearing operation triggered for the target breast pump may be initiated by the user operating the control panel (e.g., buttons, keys, or a touch screen) of the target breast pump. It may alternatively be triggered by the sensor of the target breast pump. For example, when the target breast pump is in an active or standby state, and the user picks up the target breast pump, an acceleration sensor built in the target breast pump detects that the acceleration of the target breast pump changes within a short time.

Furthermore, the wearing operation may involve wearing one breast pump (i.e., one-side wearing) or two breast pumps (i.e., two-side wearing).

The wearing information corresponding to the breast pump carries an identifier (e.g., a serial number or product serial number) of the breast pump and the wearing status of the breast pump, where the wearing status of the breast pump includes changes in the relative position between the breast pump and the breast when the breast pump is in the wearing state.

As described above, the wearing information carries the identifier and wearing status of the breast pump. The following description is made with the example of the user wearing one breast pump. First, the user activates the breast pump and establishes a communication connection between the breast pump and an electronic device (e.g., a mobile phone). This communication connection may be a wireless connection or a Bluetooth connection. A wireless connection refers to establishing a communication link between devices using wireless technologies such as WiFi, 2G, 3G, 4G, or 5G, providing the foundation for data communication between the devices, also known as a wireless link. A Bluetooth connection refers to establishing a communication link between devices based on Bluetooth wireless technology, which is one of the most widely used global short-range wireless standards. The Bluetooth 4.0 specification encompasses three Bluetooth technologies: Classic Bluetooth, High-Speed Bluetooth, and Bluetooth Low Energy (BLE), and all three specifications are integrated into one. It inherits the inherent advantages of Bluetooth technology in wireless connection while incorporating the characteristics of High-Speed Bluetooth and Bluetooth Low Energy. The core of the Bluetooth 4.0 specification is Bluetooth Low Energy (BLE). The most notable characteristics of this technology are its ultra-low operational and standby power consumption. BLE features high reliability and low power consumption. BLE is designed to replace the idle state of classic Bluetooth with a deep sleep state. In the deep sleep state, the main unit remains in an ultra-low load cycle state for a long time, and is activated by the controller only when operation is required. Since the main unit consumes more energy than the controller, such a design also saves the most energy. Therefore, optionally, in some embodiments, the communication connection between the breast pump and the mobile phone is established based on BLE. Thus, the power consumption of the breast pump in the communication connection can be reduced, and the operational duration of the breast pump can be prolonged.

Further, after establishing the communication connection between the breast pump and the mobile phone, the user picks up the breast pump and wears it onto the breast. During this process, the acceleration sensor and/or tilt sensor (e.g., gyroscope) built in the breast pump or a human body detection sensor disposed on the surface of the breast pump detect(s) that the position and acceleration of the device (i.e., the breast pump) change within a short time (e.g., 2 or 3 seconds, which can be set based on actual circumstances). This triggers the wearing operation for the breast pump. At this point, the sensor built in the breast pump obtains the corresponding data (e.g., infrared data, gyroscope data, and acceleration data), thereby forming the wearing information.

In step 102, a prompting instruction corresponding to the wearing information is determined.

Optionally, in some embodiments, the wearing position of the breast pump in the operating state is that the breast shield closely fits the breast and the nipple is aligned with the central position of the nipple channel of the breast shield. Therefore, it will be understood that, in the wearing state, when the relative position between the breast pump and the breast refers to that the breast shield closely fits the breast and the nipple is aligned with the central position of the breast shield, it is determined that the user is wearing it correctly. In other cases, it is considered incorrectly worn. That is, the prompting instruction provided in the present disclosure includes a correct wearing instruction and an incorrect wearing instruction.

It should be noted that the wearing information contains data that changes over a period of time. The time point at which the change begins is determined as a starting time point, and the time point at which the change stops is determined as an ending time point. Based on the data corresponding to the ending time point, the prompting instruction corresponding to the wearing information is determined.

For example, if the user picks up the breast pump for wearing at time T1 and stops the wearing operation at time T3, the wearing information includes data corresponding to the period from the time T1 to the time T3. It is then detected whether, at the time T3, the breast shield closely fits the breast and whether the nipple is aligned with the central position of the breast shield. If it is detected that, at the time T3, the breast shield closely fits the breast and the nipple is aligned with the central position of the breast shield, the prompting instruction corresponding to the wearing information is determined to be a correct prompting instruction. If it is detected that, at the time T3, the breast shield closely fits the breast and/or the nipple is not aligned with the central position of the breast shield, the prompting instruction corresponding to the wearing information is determined to be an incorrect prompting instruction.

Furthermore, after the user triggers the wearing operation for the breast pump and the breast pump is connected to the current device, a prompting page/selection page for left/right wearing may be displayed on the current device, as shown in FIG. 25. The prompting page includes a left option L and a right option R. In response to a selection operation triggered by the user for the left option L, the breast pump is determined to be worn on the left side; and in response to a selection operation triggered for the right option R, the breast pump is determined to be worn on the right side. Certainly, the prompting page does not necessarily have to display only left and right options; it may also present options in the form of a drop-down menu or a toggle button for left/right selection, allowing the user to make a choice. For example, a question may be displayed: "On which side will the current breast pump be worn: left or right?" The left/right options are displayed as described above.

It should also be noted that, in the present disclosure, whether the nipple is aligned with the central position of the breast shield can be detected by a built-in infrared sensor, and whether the breast shield closely fits the breast can be detected by a built-in pressure sensor.

In step 103, the breast pump and/or a current device is controlled to execute a prompting event according to the prompting instruction.

In the present disclosure, the breast pump may be controlled to execute a prompting event (e.g., a vibration event) according to the prompting instruction, or the current device (e.g., a mobile phone or tablet) may be controlled to execute a prompting event (e.g., a voice announcement event) according to the prompting instruction. Certainly, it is also possible to simultaneously control the breast pump to execute a prompting event (e.g., a vibration event) and control the current device (e.g., a mobile phone or tablet) to execute a prompting event (e.g., a voice announcement event) according to the prompting instruction. This may be specifically set based on actual circumstances.

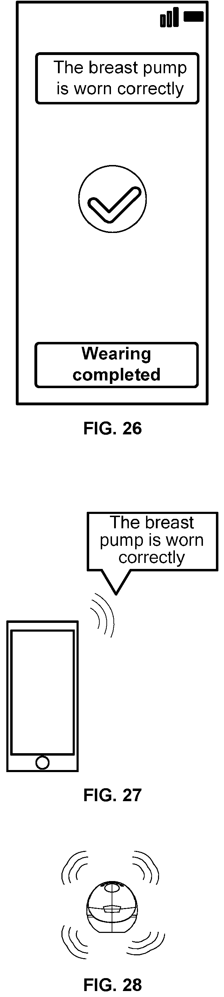

Optionally, in some embodiments, when the prompting instruction is a correct prompting instruction, a prompting page for correct wearing may be displayed on the display screen of the current device or the breast pump, as shown in FIG. 26. A prompt tone for correct wearing may be played by the current device or the breast pump, as shown in FIG. 27. Alternatively, the current device or the breast pump may be controlled to vibrate, as shown in FIG. 28. Alternatively, at least two or more of the above prompts may be performed simultaneously.

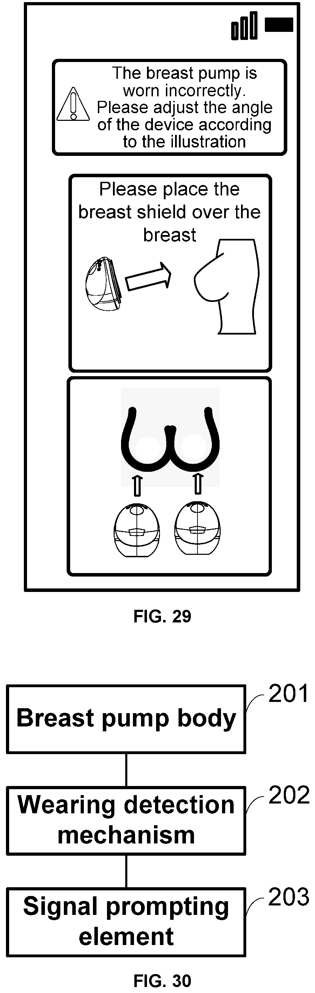

Optionally, in some embodiments, when the prompting instruction is an incorrect prompting instruction, a prompting page for incorrect wearing may be displayed on the display screen of the current device or the breast pump, as shown in FIG. 29. A wearing guidance for correct wearing may also be displayed in the prompting page. Alternatively, a prompt tone for incorrect wearing may be played by the current device or the breast pump. Specifically, a prompt "The breast shield is not properly fitted to the breast" or "The nipple is not aligned with the center of the breast shield" may be announced. Alternatively, the current device or the breast pump may be controlled to vibrate. Alternatively, at least two or more of the above-mentioned prompts may be performed simultaneously.

Optionally, in some embodiments, when both correct wearing and incorrect wearing are indicated by vibration prompts from the breast pump, the number of vibrations for correct wearing and that for incorrect wearing may be set to different counts. For example, one vibration may be set for correct wearing, while two vibrations may be set for incorrect wearing. Certainly, other counts may also be set, depending on actual circumstances.

The prompting methods for when the user wears the breast pump correctly and incorrectly may be the same or different. For example, when the user wears the breast pump correctly, the current device may play a prompt tone for correct wearing while simultaneously controlling the breast pump to vibrate once. When the user wears the breast pump incorrectly, the breast pump may be controlled to play a preset prompt tone for incorrect wearing, while the display screen of the current device displays a prompting page for incorrect wearing and shows a wearing guidance for correct wearing.

After the user wears the breast pump on the left/right side properly according to the prompt, the current device confirms, according to user operation, whether the currently worn breast pump is on the left or right side, and stores the data of the left or right breast pump accordingly.

Thus, the wearing prompting process for a breast pump according to the present disclosure is completed.

As described above, the present disclosure provides a wearing prompting method for a breast pump. The wearing information corresponding to the breast pump is detected in response to the wearing operation triggered for the breast pump, where the wearing information is acquired by the sensor built in the breast pump. The prompting instruction corresponding to the wearing information is then determined. Finally, the breast pump and/or the current device is controlled to execute the prompting event according to the prompting instruction. In the wearing prompting method for a breast pump provided by the present disclosure, the corresponding prompting instruction is determined according to the wearing information acquired by the sensor of the breast pump. The breast pump and/or the current device is controlled to execute the prompting event according to the prompting instruction so that the user can obtain the actual wearing status of the breast pump. This avoids the problem that the user is unable to express the breast milk when using the breast pump due to incorrect wearing.

Correspondingly, referring to FIG. 30, an embodiment of the present disclosure provides a wearing prompting system (hereinafter referred to as "prompting system") for a breast pump. The wearing prompting system includes a breast pump body 201, a wearing detection mechanism 202, and a signal prompting element 203.

The wearing detection mechanism 202 is configured to detect wearing information of the breast pump body 201.

The signal prompting element 203 is configured to output prompting information based on the wearing information of the breast pump.

Optionally, in some embodiments of the present disclosure, the prompting information includes a prompt tone, a prompting page, and a wearing guidance.

Optionally, in some embodiments of the present disclosure, the wearing detection mechanism includes a sensor configured to detect whether the breast pump body closely fits a breast and/or detect whether a mother's nipple is placed along a nipple channel and/or detect whether the breast pump body 201 is worn obliquely.

The wearing information corresponding to the breast pump carries an identifier (e.g., a serial number or product serial number) of the breast pump and the wearing status of the breast pump, where the wearing status of the breast pump includes changes in the relative position between the breast pump and the breast when the breast pump is in the wearing state.

Optionally, in some embodiments of the present disclosure, the sensor may be an acceleration sensor (configured to detect acceleration information of the breast pump), a tilt sensor (e.g., a gyroscope), and/or a human body detection sensor. For specific usage methods, reference is made to the foregoing embodiments, which will not be reiterated here.

As described above, the present disclosure provides a wearing prompting system for a breast pump, which includes a breast pump body 201, a wearing detection mechanism 202, and a signal prompting element 203. The wearing detection mechanism 202 is configured to detect the wearing information of the breast pump body 201. The signal prompting element 203 is configured to output the prompting information based on the wearing information of the breast pump. In the wearing prompting solution for a breast pump provided by the present disclosure, the breast pump and/or the current device is controlled to execute the prompting event according to the wearing information acquired by the wearing detection mechanism 20 so that the user can obtain the actual wearing status of the breast pump. This avoids the problem that the user is unable to express the breast milk when using the breast pump due to incorrect wearing. The wearing prompting system further includes a processor configured to control the signal prompting element to output prompting information based on the wearing information. The signal prompting element includes a speaker. The processor is configured to control the speaker to play a prompt tone for correct wearing and/or a prompt tone for incorrect wearing. The signal prompting element includes a display screen. The display screen is controlled to display prompting information for incorrect wearing. The processor is further configured to control the display screen to display a wearing guidance for correct wearing. The signal prompting element includes a vibrating element. The processor is configured to: in response to correct wearing, control the breast pump and/or a current device to vibrate for a first preset number of times; and in response to incorrect wearing, control the breast pump and/or the current device to vibrate for a second preset number of times. The wearing prompting system further includes: a processor configured to generate the prompting information according to the wearing information; and a transmitter configured to transmit the prompting information to a terminal device. The wearing prompting system further includes: a transmitter configured to transmit the wearing information to a terminal device.

A terminal device includes: a receiver configured to receive wearing information of a breast pump transmitted from the breast pump; a signal prompting element; and a processor configured to control the signal prompting element to output prompting information according to the wearing information.

A terminal device includes a signal prompting element including a speaker. The step of controlling the signal prompting element to output the prompting information based on the wearing information includes: controlling the speaker to play a prompt tone for correct wearing and/or a prompt tone for incorrect wearing. The signal prompting element includes a display screen. The step of controlling the signal prompting element to output the prompting information based on the wearing information includes: controlling the display screen to display prompting information for incorrect wearing. The processor is further configured to control the display screen to display a wearing guidance for correct wearing. The signal prompting element includes a vibrating element; and the step of controlling the signal prompting element to output the prompting information based on the wearing information includes:
in response to correct wearing, controlling the breast pump and/or a current device to vibrate for a first preset number of times; and
in response to incorrect wearing, controlling the breast pump and/or the current device to vibrate for a second preset number of times.

A terminal device is further provided, including: a receiver configured to receive wearing information transmitted from a breast pump; a signal prompting element; and a processor configured to control, based on the wearing information, the signal prompting element to output wearing prompting information according to the wearing information.

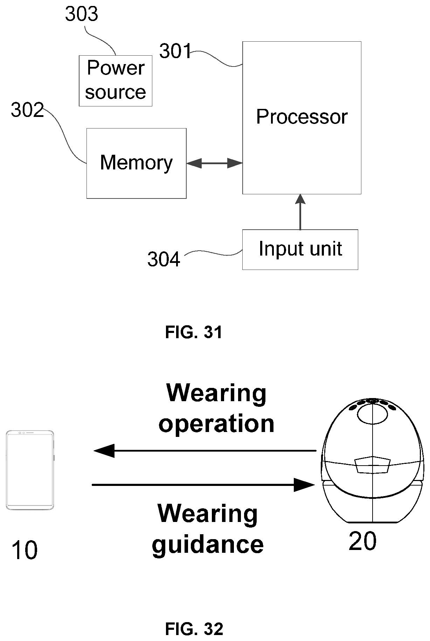

Furthermore, the present disclosure further provides an electronic device. FIG. 31 illustrates a schematic structural diagram of an electronic device involved in the present disclosure. Specifically, the electronic device may include components such as a processor 301 with one or more processing cores, a memory 302 with one or more computer-readable storage media, a power source 303, and an input unit 304. It will be understood by those skilled in the art that the structure of the electronic device as shown in FIG. 31 does not constitute a limitation on the electronic device, which may include more or less components than those shown in the figure, or some components are combined, or different component arrangements are provided.

The processor 301 is a control center of the electronic device, connects to various parts of the electronic device by using various interfaces and lines, and performs various functions and data processing of the electronic device by running or executing software programs and/or modules stored in the memory 302 and calling data stored in the memory 302, so as to perform overall monitoring on the electronic device. Optionally, the processor 301 may include one or more processing cores. Preferably, the processor 301 may be integrated with an application processor and a modulation and demodulation processor. The application processor is configured to mainly process an operating system, a user interface, an application program, and the like. The modulation and demodulation processor is configured to mainly process wireless communication. It will be understood that the modulation and demodulation processor may not be integrated into the processor 301.

The memory 302 may be configured to store a software program and a module, and the processor 301 is configured to run the software and the module stored in the memory 302 to perform various functional applications and data processing. The memory 302 may mainly include a program storage area and a data storage area. The program storage area may store an operating system, an application program required by at least one function (such as a sound playing function and an image playing function), and the like. The data storage area may store data created based on the use of the electronic device, and the like. Moreover, the memory 302 may include a highspeed random access memory, and may also include a non-volatile memory, for example, at least one magnetic disk storage device, a flash memory device, or other volatile solid storage device. Correspondingly, the memory 302 may further include a memory controller to provide access to the memory 302 by the processor 301.

The electronic device may further include the power source 303 that supplies power to various components. Preferably, the power source 303 can be logically connected to the processor 301 via a power management system. Thus, functions such as charging, discharging, and power consumption management may be achieved via the power management system. The power source 303 may further include any component such as one or more direct-current or alternating-current power sources, a recharging system, a power failure detection circuit, a power converter or inverter, and a power status indicator.

The electronic device may further include the input unit 304 that can be configured to receive entered numbers or character information, and generate keyboard, mouse, joystick, optical or trackball signal inputs associated with user settings and function control.

Although not shown, the electronic device may further include a display unit and the like, which will not be repeated herein. Specifically, in this embodiment, the processor 301 in the electronic device may load executable files corresponding to processes of one or more application programs into the memory 302 according to instructions as described below, and the application programs stored in the memory 302 may be run by the processor 301 to achieve various functions as follows:

obtaining wearing information corresponding to the breast pump in response to a wearing operation triggered for the breast pump; determining a prompting instruction corresponding to the wearing information; and controlling the breast pump and/or a current device to execute a prompting event according to the prompting instruction.

Regarding to specific implementations of the above various operations, see the foregoing embodiments, which will not be repeated herein.

In the embodiments of the present disclosure, the wearing information corresponding to the breast pump is detected in response to the wearing operation triggered for the breast pump, where the wearing information is acquired by the sensor built in the breast pump. The prompting instruction corresponding to the wearing information is then determined. Finally, the breast pump and/or the current device is controlled to execute the prompting event according to the prompting instruction. In the wearing prompting method for a breast pump provided by the present disclosure, the corresponding prompting instruction is determined according to the wearing information acquired by the sensor of the breast pump. The breast pump and/or the current device is controlled to execute the prompting event according to the prompting instruction so that the user can obtain the actual wearing status of the breast pump. This avoids the problem that the user is unable to express the breast milk when using the breast pump due to incorrect wearing.

Those of ordinary skill in the art will understand that all or part of the steps in the methods of the above embodiments may be implemented with instructions or by controlling related hardware via instructions. The instructions may be stored in a computer-readable storage medium and loaded and executed by a processor.

To this end, the present disclosure provides a storage medium having a plurality of instructions stored therein that can be loaded by a processor to perform the steps of any wearing prompting method for a breast pump provided by the present disclosure. For example, the instructions may cause the following steps to be performed:
obtaining wearing information corresponding to the breast pump in response to a wearing operation triggered for the breast pump; determining a prompting instruction corresponding to the wearing information; and controlling the breast pump and/or a current device to execute a prompting event according to the prompting instruction.

### Solution V:

For example, referring to FIG. 32, the present disclosure provides a breast pump system, which is integrated into a terminal 10 such as a mobile phone, a tablet computer, or an independently configured operation control setup or a breast pump 20. When receiving a wearing operation triggered by the user for the target breast pump 20, the mobile phone 10 shows a wearing guidance page for the target breast pump. The wearing guidance page includes wearing guidance information for the target breast pump 20, and the wearing guidance information is configured for instructing the wearing of the target breast pump 20 in an operating mode. The wearing position of the target breast pump 20 may be on the user's left breast or the user's right breast, depending on an actual situation. When the mobile phone 10 determines that the current wearing state of the target breast pump 20 satisfies a preset operating wearing state, the target breast pump is configured with configuration parameters corresponding to a target operating mode, and prompting information for successful wearing is shown in the wearing guidance page.

In the wearing guidance method for a breast pump provided by the present disclosure, when the user triggers the wearing operation for the breast pump, the wearing guidance page for the target breast pump may be shown, where the wearing guidance page contains the wearing guidance information. Therefore, the user can be instructed by the wearing guidance information to wear the breast pump in the operating mode. When the user uses the breast pump for the first time or wears the breast pump again, the user can be guided by the wearing guidance page to correctly wear it.

The present disclosure provides a wearing guidance method for a breast pump, including: showing a wearing guidance page for a target breast pump in response to a wearing operation triggered for the target breast pump; determining whether a current wearing state of the target breast pump satisfies a preset operating wearing state; and when the current wearing state of the target breast pump satisfies the operating wearing state, configuring the target breast pump with configuration parameters corresponding to a target operating mode and/or showing prompting information for successful wearing in the wearing guidance page.

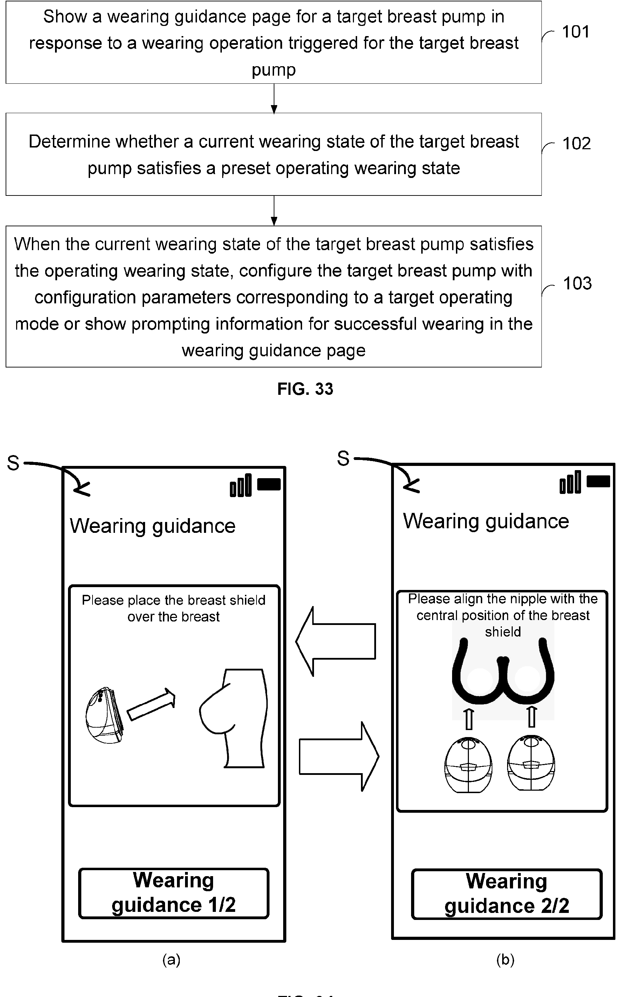

FIG. 33 is a flowchart of a wearing guidance method for a breast pump provided by the present disclosure. The specific process of the wearing guidance method for a breast pump may include the following steps.

In step 101, a wearing guidance page for a target breast pump is shown in response to a wearing operation triggered for the target breast pump.

The target breast pump refers to the breast pump currently being worn. The wearing guidance page includes wearing guidance information for the target breast pump, and the wearing guidance information is configured for instructing the wearing of the target breast pump in an operating mode. Generally, breast pumps are sold in pairs. That is, the breast pump purchased by the user may include a first breast pump and a second breast pump. During wearing by the user, the user may wear the first breast pump first or the second breast pump first. Furthermore, the user may wear a breast pump on only one breast or wear the breast pumps on both breasts simultaneously, which can be selected based on actual circumstances.

For example, specifically, after the user triggers the wearing operation for the target breast pump, the target breast pump may issue a wearing guidance instruction to an electronic device wirelessly or via Bluetooth. Upon receiving the wearing guidance instruction, the electronic device determines the target breast pump corresponding to the wearing guidance instruction and shows the wearing guidance page corresponding to the target breast pump. The wearing guidance page includes the wearing guidance information for the target breast pump, and the wearing guidance information is configured for instructing the user to place the target breast pump in the wearing position in the operating mode. For example, the breast shield is placed over the breast, ensuring a close fit between the breast and the breast shield, and aligning the nipple with the central position of the breast shield.

Optionally, in some embodiments, the wearing guidance information may include an angle configuration guidance and a position configuration guidance. The angle configuration guidance is configured for indicating the wearing direction of the target breast pump and is a graphical and textual guidance. Meanwhile, the current configuration angle is detected automatically and in real time by the target breast pump, and the configuration angle refers to the pitch angle of the front side of the target breast pump, as shown in FIG. 34(a). The position configuration guidance may also be a graphical and textual guidance. Meanwhile, whether the device is aligned and fitted with the human breast is detected automatically and in real time by the target breast pump. The angle configuration guidance may be switched to the position configuration guidance by a switch control s, as shown in FIG. 34(b). That is, in response to an operation for the switch control s, the angle configuration guidance is switched to the position configuration guidance.

Optionally, in some embodiments, the wearing operation triggered for the target breast pump may be initiated by the user activating certain components (e.g., buttons or keys) of the target breast pump. Alternatively, it may be triggered by the sensor of the target breast pump. For example, when the breast pump is in an active or standby state, and the user picks up the target breast pump, an acceleration sensor built in the target breast pump detects that the acceleration of the target breast pump changes within a short time. In such cases, the wearing operation triggered for the target breast pump may be set based on actual circumstances.

On the wearing guidance page, the user may choose whether to display wearing guidance, or may select the type of guidance to be shown, such as a graphical and textual guidance, a video guidance, or a pointer guidance.

In step 102, it is determined whether a current wearing state of the target breast pump satisfies a preset operating wearing state.

The preset operating wearing state may refer to that the breast pump faces upward, the breast shield closely fits the breast, and the nipple is aligned with the central position of the nipple channel of the breast shield. The breast pump provided by the present disclosure may be equipped with an infrared sensor and/or a gyroscope sensor, where the gyroscope sensor is configured to determine the orientation of the target breast pump. Optionally, in some embodiments of the present disclosure, the side with a user-operable panel is determined as the forward-facing direction of the target breast pump. The gyroscope sensor can determine whether the user's wearing direction is correct. Additionally, the infrared sensor can detect whether the nipple is aligned with the central position of the nipple channel of the breast shield.

The current wearing state refers to the physical wearing state of the target breast pump, such as whether the breast shield of the target breast pump fits against the breast, whether the nipple is aligned with the central position of the breast shield, and whether the breast pump is facing upward. When the current wearing state satisfies the operating wearing state, the method proceeds to step 103. When the current wearing state of the target breast pump does not satisfy the operating wearing state, prompting information for incorrect wearing is shown in the wearing guidance page.

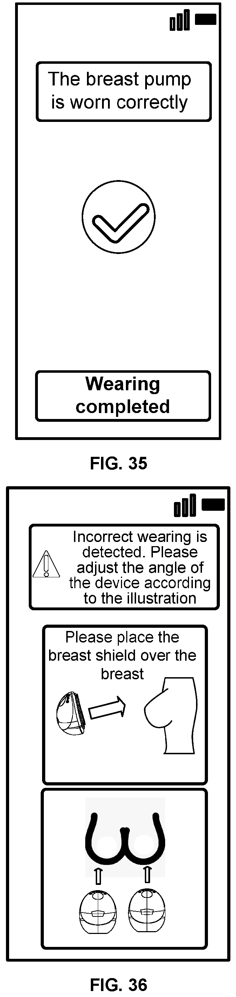

For example, specifically, referring to FIG. 35, when the current wearing state satisfies the operating wearing state, a prompt for correct wearing is displayed in the graphical and textual guidance and step 103 is performed. When the current wearing state does not satisfy the operating wearing state, the prompting information for incorrect wearing is displayed in the graphical and textual guidance, such as a prompt that the breast shield does not closely fit the breast and/or the nipple is not aligned with the center of the breast shield, as shown in FIG. 36.

Optionally, in some embodiments, after the wearing operation is triggered for the target breast pump, when it is detected that the user has not correctly worn the target breast pump within a preset time period, a voice announcement is made based on the prompting information shown in FIG. 36 to further guide the user in wearing the target breast pump.

Optionally, in some embodiments, after displaying the prompting information for incorrect wearing in the graphical and textual guidance, if the number of incorrect wearing attempts by the user exceeds a preset count, the graphical and textual guidance is updated. For example, the graphical and textual guidance composed of text and an image is updated to a graphical and textual guidance composed of text and a short video. For example, specifically, when the user repeatedly makes errors in wearing angle, the graphical and textual guidance is switched to the pointer guidance. For example, when the user repeatedly makes errors in wearing angle, the graphical and textual guidance is switched to the pointer guidance. The pointer guidance is configured for indicating a deviation between the user's current wearing angle and the reference angle, prompting the user to adjust the target breast pump upward or downward to ensure the correct wearing angle.

Through the pointer guidance, the user is more intuitively guided to correct the wearing angle of the target breast pump. When the user repeatedly experiences poor fitting, such as the breast shield not closely fitting the breast or the nipple being not aligned properly, the graphical and textual guidance is switched to a short video guidance. The short video guidance is configured for demonstrating common wearing errors and explaining how to correct the wearing angle in such situations, thereby providing guidance for the user to correctly wear the target breast pump.

In step 103, when the current wearing state of the target breast pump satisfies an operating wearing state, the target breast pump is configured with configuration parameters corresponding to a target operating mode and/or prompting information for successful wearing is shown in the wearing guidance page.

When the current wearing state of the target breast pump satisfies the operating wearing state, the wearing guidance page may be switched to a parameter configuration page. The user may set the operating parameters of the target breast pump in the target operating mode on the parameter configuration page. For example, the operating mode may include a letdown mode, an expression mode, and an electric mode.

It will be understandable that a plurality of operating modes MAY coexist for the same device, i.e., the target device may include a plurality of operating modes simultaneously. The user can select the target operating mode for the target breast pump to operate according to actual needs. However, it is not required that the operating modes of different breast pumps must be the same. For example, for a pair of breast pump a and breast pump b, the breast pump a may operate in the letdown mode, while the breast pump b may operate in the expression mode. Certainly, they may also operate in other modes, without limitation.

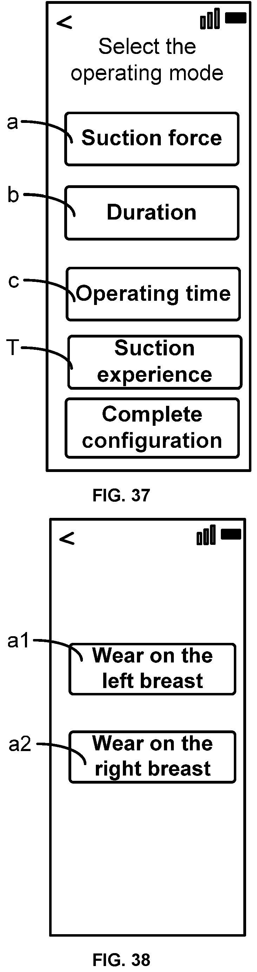

When the current wearing state of the target breast pump satisfies the operating wearing state, the operating mode selected by the user on the parameter configuration page is determined as the target operating mode, and parameter configuration options corresponding to the target operating mode are shown. Optionally, in some embodiments, the user can set parameters such as suction force, expression duration, and operating time on the parameter configuration page. For example, specifically, referring to FIG. 37, taking the target operating mode as the letdown mode for illustration, the present disclosure provides a parameter configuration page, which includes a suction force option A, a duration option B, and an operating time option C. In some embodiments, the suction force under the suction force option a may be the lowest suction force by default, the expression duration under the duration option b may be 5 minutes by default, and the operating time under the operating time option c may be immediate execution by default. That is, if the user does not configure the configuration parameters of the target breast pump after the current wearing state of the target breast pump satisfies the operating wearing state, the target breast pump is controlled to operate according to the aforementioned default options. When the user makes selections regarding the above options, the content of each option may be adjusted based on the user's selections. For example, the suction force option a is adjusted to the second level, the duration option b is adjusted to 10 minutes, and/or the operating time option c is adjusted to start after 10 minutes, etc. These settings can be customized by the user according to their needs. Once the user confirms the selections, the breast pump operates according to the selected settings. That is, in response to a confirmation operation for the operating options, the target breast pump is controlled to operate based on the confirmed operating options.

Furthermore, in some embodiments, continuously referring to FIG. 37, a suction experience control T may also be added to the parameter configuration page. In response to a tapping operation triggered for the suction experience control T, the target breast pump is controlled to operate in the target operating mode according to a preset suction force strategy. Taking the letdown mode as an example, when the tapping operation triggered by the user for the suction experience control is detected, the target breast pump is controlled to gradually increase the suction force in the letdown mode, with a time interval of 10 seconds for each increase in suction force. Assuming that the letdown mode corresponds to the suction force of the third level, the suction experience duration corresponding to the letdown mode would be 30 seconds, after which the target breast pump stops operating.

After the wearing is completed, to facilitate subsequent monitoring of the milk expression data of different breasts, option controls for the left and right breasts may be shown in the wearing guidance page. As shown in FIG. 38, controls a1 and a2 are shown in the wearing guidance page, where the control a1 corresponds to the left breast and the control a2 corresponds to the right breast. The user can tap on the corresponding controls based on actual needs, facilitating subsequent monitoring of the milk expression data of different breasts via an electronic device such as a mobile phone.

It should be noted that each time the user completes the wearing process, the option controls may be displayed. The user may select the corresponding breast to correspond to the breast pump after completing the wearing process. Therefore, it will be understood that, after the user wears the target breast pump on the left/right breast and taps on the control a1, when the user wears the other breast pump, the user automatically selects the control a2 in the wearing guidance page upon completion of wearing, thereby enabling monitoring of the milk expression data of the right breast.

Optionally, in some embodiments, after the initial wearing is completed, the user gains some experience in how to wear the breast pump. Thus, for the user wearing the breast pump for the second time, to save the time of wearing the breast pump, the aforementioned suction experience flow may be skipped. That is, the suction experience control is hidden in the wearing guidance page, and the user can be prompted in the wearing guidance page to select the left or right breast to facilitate subsequent monitoring of the milk expression data.

Thus, the wearing guiding process for a breast pump according to the present disclosure is completed.

As described above, the present disclosure provides a wearing guidance method for a breast pump. The wearing guidance page for the target breast pump is shown in response to the wearing operation triggered for the target breast pump, where the wearing guidance page includes the wearing guidance information for the target breast pump, and the wearing guidance information is configured for instructing the wearing of the target breast pump in the operating mode. It is then determined whether the current wearing state of the target breast pump satisfies the preset operating wearing state. When the current wearing state of the target breast pump satisfies the operating wearing state, the target breast pump is configured with the configuration parameters corresponding to the target operating mode, and the prompting information for successful wearing is shown in the wearing guidance page. In the wearing guidance method for a breast pump provided by the present disclosure, when the user triggers the wearing operation for the breast pump, the wearing guidance page for the target breast pump may be shown, where the wearing guidance page contains the wearing guidance information. Therefore, the user can be instructed by the wearing guidance information to wear the breast pump in the operating mode. When the user uses the breast pump for the first time or wears the breast pump again, the user can be guided by the wearing guidance page to correctly wear it, and the breast pump can be instructed to operate normally.

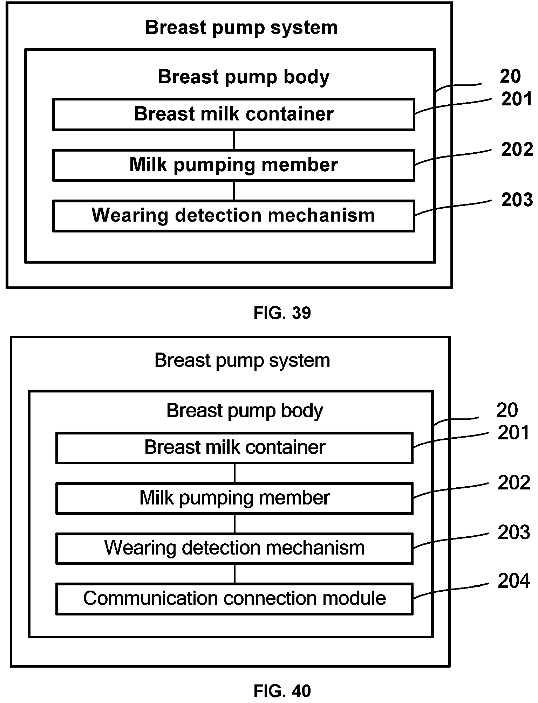

Correspondingly, referring to FIG. 39, an embodiment of the present disclosure provides a breast pump system that may specifically include a breast pump body 20. The breast pump body 20 includes a breast milk container 201, a milk pumping member 202, and a wearing detection mechanism 203.

The breast pump body 20 is configured to be worn on a mother's breast and express milk from the breast. The breast pump body 20 may specifically include:
the breast milk container 201 including a milk storage container and a connection portion, where the milk storage container is connected to the connection portion;
the milk pumping member 202 including an attachment portion and a nipple channel, where a shape of the attachment portion is adapted to the breast, and the nipple channel is connected to the milk storage container via the connection portion; and
the wearing detection mechanism 203 configured to detect whether the milk pumping member is worn correctly.

Optionally, in some embodiments of the present disclosure, the wearing detection mechanism 203 includes a first sensor disposed on the attachment portion and a second sensor disposed on the nipple channel.

Optionally, in some embodiments of the present disclosure, the first sensor is configured to detect whether the attachment portion closely fits the breast.

Optionally, in some embodiments of the present disclosure, the second sensor is configured to detect whether the mother's nipple is placed along the nipple channel.

Optionally, in some embodiments of the present disclosure, the wearing detection mechanism 203 further includes a third sensor configured to detect whether the breast pump body 20 is worn obliquely.

Optionally, in some embodiments of the present disclosure, referring to FIG. 40, the breast pump system further includes a communication connection module 204 configured to connect the breast pump body and a terminal device or a server and transmit milk expression-related information of the breast pump body 20.

Optionally, in some embodiments of the present disclosure, the breast pump system further includes an identification element 205 configured at least to identify one target breast pump body 20 such that the terminal device or the server identifies and matches the target breast pump body.

Optionally, in some embodiments of the present disclosure, the breast pump system further includes a first identification element and a second identification element that are configured at least to identify two target breast pump bodies 20, respectively, such that the terminal device or the server identifies and matches each of the target breast pump bodies 20.

As described above, the present disclosure provides a breast pump system including the breast pump body 20. The breast pump body 20 includes the breast milk container 201, the milk pumping member 202, and the wearing detection mechanism 203. The breast pump body 20 is configured to be worn on a mother's breast and express milk from the breast. The breast milk container 201 includes the milk storage container and the connection portion, where the milk storage container is connected to the connection portion. The milk pumping member 202 includes the attachment portion and the nipple channel, where the shape of the attachment portion is adapted to the breast, and the nipple channel is connected to the milk storage container via the connection portion. The wearing detection mechanism 203 is configured to detect whether the milk pumping member is worn correctly. In the breast pump system provided by the present disclosure, the wearing detection mechanism 203 detects whether the milk pumping member is correctly worn. When the user uses the breast pump for the first time or wears the breast pump again, the user can be guided by the wearing guidance page to correctly wear it and the breast pump can be instructed to operate normally. The breast pump system further includes a controller that is disposed on the breast pump body and is configured to determine whether the milk pumping member is correctly worn according to induction information of the first sensor and induction information of the second sensor.

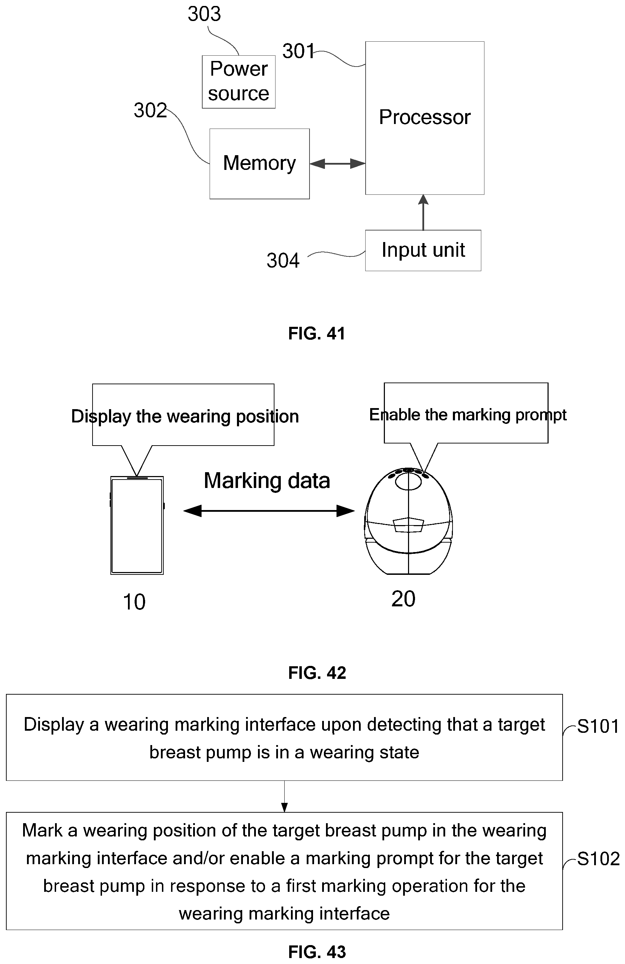

Furthermore, the present disclosure further provides an electronic device. FIG. 41 illustrates a schematic structural diagram of an electronic device involved in the present disclosure. Specifically, the electronic device may include components such as a processor 301 with one or more processing cores, a memory 302 with one or more computer-readable storage media, a power source 303, and an input unit 304. It will be understood by those skilled in the art that the structure of the electronic device as shown in FIG. 41 does not constitute a limitation on the electronic device, which may include more or less components than those shown in the figure, or some components are combined, or different component arrangements are provided.

The processor 301 is a control center of the electronic device, connects to various parts of the electronic device by using various interfaces and lines, and performs various functions and data processing of the electronic device by running or executing software programs and/or modules stored in the memory 302 and calling data stored in the memory 302, so as to perform overall monitoring on the electronic device. Optionally, the processor 301 may include one or more processing cores. Preferably, the processor 301 may be integrated with an application processor and a modulation and demodulation processor. The application processor is configured to mainly process an operating system, a user interface, an application program, and the like. The modulation and demodulation processor is configured to mainly process wireless communication. It will be understood that the modulation and demodulation processor may not be integrated into the processor 301.

The memory 302 may be configured to store a software program and a module, and the processor 301 is configured to run the software and the module stored in the memory 302 to perform various functional applications and data processing. The memory 302 may mainly include a program storage area and a data storage area. The program storage area may store an operating system, an application program required by at least one function (such as a sound playing function and an image playing function), and the like. The data storage area may store data created based on the use of the electronic device, and the like. Moreover, the memory 302 may include a highspeed random access memory, and may also include a non-volatile memory, for example, at least one magnetic disk storage device, a flash memory device, or other volatile solid storage device. Correspondingly, the memory 302 may further include a memory controller to provide access to the memory 302 by the processor 301.

The electronic device may further include the power source 303 that supplies power to various components. Preferably, the power source 303 can be logically connected to the processor 301 via a power management system. Thus, functions such as charging, discharging, and power consumption management may be achieved via the power management system. The power source 303 may further include any component such as one or more direct-current or alternating-current power sources, a recharging system, a power failure detection circuit, a power converter or inverter, and a power status indicator.

The electronic device may further include the input unit 304 that can be configured to receive entered numbers or character information, and generate keyboard, mouse, joystick, optical or trackball signal inputs associated with user settings and function control.

Although not shown, the electronic device may further include a display unit and the like, which will not be repeated herein. Specifically, in this embodiment, the processor 301 in the electronic device may load executable files corresponding to processes of one or more application programs into the memory 302 according to instructions as described below, and the application programs stored in the memory 302 may be run by the processor 301 to achieve various functions as follows:

showing a wearing guidance page for a target breast pump in response to a wearing operation for the target breast pump; determining whether a current wearing state of the target breast pump satisfies a preset operating wearing state; and when the current wearing state of the target breast pump satisfies the operating wearing state, configuring the target breast pump with configuration parameters corresponding to a target operating mode and/or showing prompting information for successful wearing in the wearing guidance page.

Regarding to specific implementations of the above various operations, see the foregoing embodiments, which will not be repeated herein.

In the embodiments of the present disclosure, the wearing guidance page for the target breast pump is shown in response to the wearing operation triggered for the target breast pump, where the wearing guidance page includes the wearing guidance information for the target breast pump, and the wearing guidance information is configured for instructing the wearing of the target breast pump in the operating mode. It is then determined whether the current wearing state of the target breast pump satisfies the preset operating wearing state. When the current wearing state of the target breast pump satisfies the operating wearing state, the target breast pump is configured with the configuration parameters corresponding to the target operating mode, and the prompting information for successful wearing is shown in the wearing guidance page. In the wearing guidance method for a breast pump provided by the present disclosure, when the user triggers the wearing operation for the breast pump, the wearing guidance page for the target breast pump may be shown, where the wearing guidance page contains the wearing guidance information. Therefore, the user can be instructed by the wearing guidance information to wear the breast pump in the operating mode. When the user uses the breast pump for the first time or wears the breast pump again, the user can be guided by the wearing guidance page to correctly wear it.

Those of ordinary skill in the art will understand that all or part of the steps in the methods of the above embodiments may be implemented with instructions or by controlling related hardware via instructions. The instructions may be stored in a computer-readable storage medium and loaded and executed by a processor.

To this end, the present disclosure provides a storage medium having a plurality of instructions stored therein that can be loaded by a processor to perform the steps of any wearing guidance method for a breast pump provided by the present disclosure. For example, the instructions may cause the following steps to be performed:

showing a wearing guidance page for a target breast pump in response to a wearing operation for the target breast pump; determining whether a current wearing state of the target breast pump satisfies a preset operating wearing state; and when the current wearing state of the target breast pump satisfies the operating wearing state, configuring the target breast pump with configuration parameters corresponding to a target operating mode and/or showing prompting information for successful wearing in the wearing guidance page.

### Solution VI:

When the user wears the breast pump 20, the electronic device 10 displays a wearing marking interface on its display screen. The user may perform a first marking operation in the wearing marking interface. That is, the electronic device 10, in response to the first marking operation for the wearing marking interface, marks the wearing position of the breast pump 20 in the wearing marking interface, and/or enables a marking prompt for the breast pump 20.

Optionally, in some embodiments, when the breast pump 20 is in the wearing state, the breast pump 20 activates the marking indicator lamp of the breast pump 20 and/or shows the wearing mark in the wearing marking interface of the electronic device 10 in response to the second marking operation for the breast pump 20.

In the wearing position recording method for a breast pump provided by the present disclosure, with a striking interface prompt and/or breast pump prompt, the user can know the wearing position of the target breast pump without confirmation for a plurality of times. Thus, the time taken by the user to set the wearing position of the breast pump can be reduced, thereby improving the recording efficiency of the wearing position.

The present disclosure provides a wearing position recording method for a breast pump, including: displaying a wearing marking interface upon detecting that a target breast pump is in a wearing state; and in response to a first marking operation for the wearing marking interface, marking a wearing position of the target breast pump in the wearing marking interface and/or enabling a marking prompt for the target breast pump.

FIG. 43 is a flowchart of a wearing position recording method for a breast pump provided by the present disclosure. The specific process of the wearing position recording method for a breast pump may include the following steps.

In step S101, a wearing marking interface is displayed upon detecting that a target breast pump is in a wearing state.

When the electronic device detects that the target breast pump is in the wearing state, it displays the wearing marking interface. The wearing marking interface may show content such as a first option and a second option, or an auxiliary marking object, to assist the user in marking. The first option and the second option may be respectively set on the left and right sides of the wearing marking interface, or they may be respectively set in an upper region and a lower region. For example, the first option is set on the left side of the wearing marking interface, and the second option is set on the right side of the wearing marking interface. The auxiliary marking object may be a virtual representation, an electronic compass, a spherical rolling ball, or the like, to assist the user in marking. For example, if the auxiliary marking object is a virtual character, the virtual character changes according to variations in data from a gyroscope sensor of the electronic device. For example, if the gyroscope sensor of the electronic device indicates that the device is tilted to the left, the virtual character may perform a gesture with a finger pointing to the left, prompting the user to wear the breast pump on the left breast.

In step S102, in response to a first marking operation for the wearing marking interface, a wearing position of the target breast pump is marked in the wearing marking interface and/or a marking prompt for the target breast pump is enabled.

Optionally, in some embodiments of the present disclosure, the first marking operation may be an operation for the electronic device, an operation for the first marking operation, or an operation for a preset region, depending on actual requirements.

Optionally, in some embodiments of the present disclosure, the first marking operation may be at least one of the following operations: long press, tap, double tap, consecutive taps, swipe in a preset direction, drag, or shake.

Optionally, in some embodiments of the present disclosure, a long press refers to a long-press operation performed on a screen page of the electronic device via a finger or a touch tool.

Optionally, in some embodiments of the present disclosure, a double tap refers to a double-tap operation performed on a screen page of the electronic device via a finger or a touch tool.

Optionally, in some embodiments of the present disclosure, consecutive taps refer to continuous taps, such as three or more taps, performed on a screen page of the electronic device via a finger or a touch tool.

Optionally, in some embodiments of the present disclosure, a swiping operation in a preset direction refers to a swipe performed on a screen page of the electronic device via a finger or a touch tool along a preset swiping direction. Optionally, the preset direction may be a direction from top to bottom, a direction from bottom to top, a direction of swiping up and down first and then from side to side, a direction of swiping from side to side first and then up and down, a direction along an upper semicircle in a clockwise direction, a direction along a lower semicircle in a counterclockwise direction, a direction along an arc in the clockwise direction, and the like.

Optionally, in some embodiments of the present disclosure, a drag refers to selecting a target object on a screen page of the mobile phone via a finger or a touch tool and then moving it a certain distance.

Optionally, in some embodiments of the present disclosure, a shake refers to controlling a smart terminal to vibrate a certain number of times within a specific time period via a hand or another tool.

Optionally, in some embodiments of the present disclosure, the wearing marking interface may include a first option and a second option; and the step of marking the wearing position of the target breast pump in the wearing marking interface in response to the first marking operation may specifically include:
marking the wearing position of the target breast pump as a first position in response to a tapping operation for the first option; and
marking the wearing position of the target breast pump as a second position in response to a tapping operation for the second option.

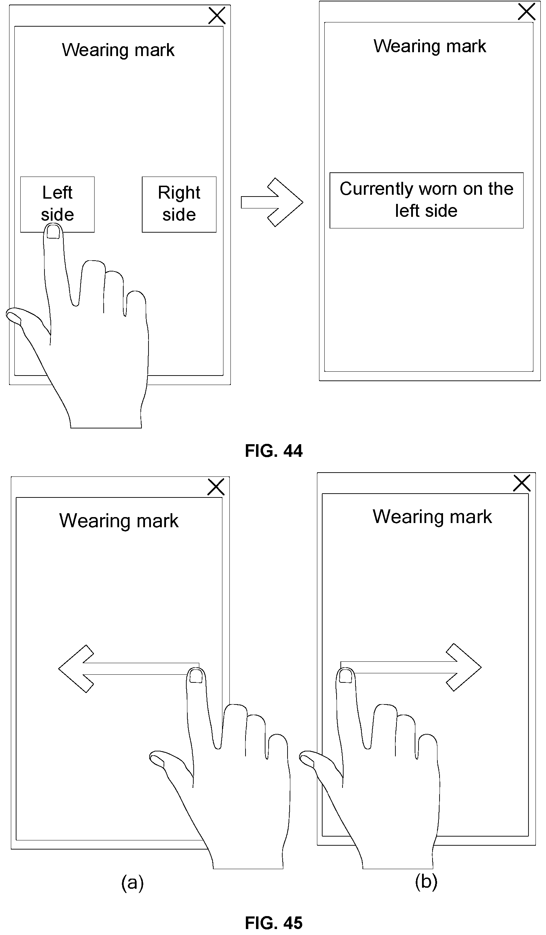

Referring to FIG. 44, the wearing marking interface may include a first option s1 and a second option s2. The first option s1 corresponds to the first position, and the second option s2 corresponds to the second position. Optionally, the first position is the left side, and the second position is the right side. In response to a tapping operation for the first option s1, the wearing position of the target breast pump is on the left side. In response to a tapping operation for the second option s2, the wearing position of the target breast pump is on the right side.

Optionally, in some embodiments of the present disclosure, the first marking operation may be a swiping operation. That is, the step of marking the wearing position of the target breast pump in the wearing marking interface in response to the first marking operation may specifically include:
identifying an operation direction of the first marking operation in response to the first marking operation;
when the operation direction is identified as a first preset direction, marking the wearing position of the target breast pump as a first position; and
when the operation direction is identified as a second preset direction, marking the wearing position of the target breast pump as a second position.

For example, specifically, referring to FIG. 45, the first preset direction may be horizontally leftward, and the second preset direction may be horizontally rightward. When it is identified that the operation direction of the user's first marking operation for the wearing marking interface is horizontally leftward, the wearing position of the target breast pump is marked as the left side, as shown in FIG. 45(a). When it is identified that the operation direction of the user's second marking operation for the wearing marking interface is horizontally rightward, the wearing position of the target breast pump is marked as the right side, as shown in FIG. 45(b).

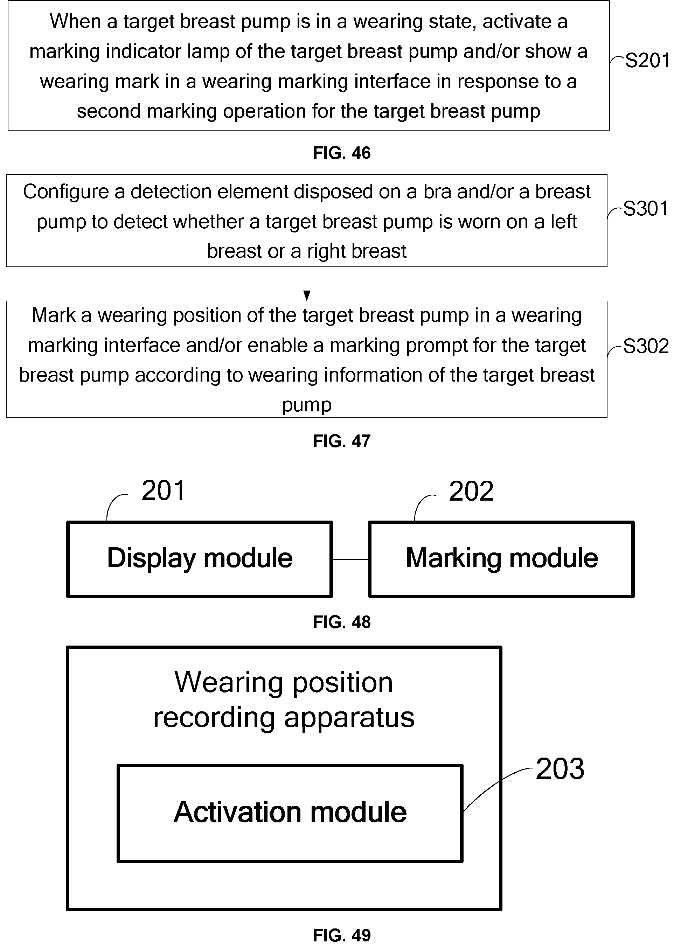

For example, specifically, a slider s may also be displayed in the wearing marking interface, as shown in FIG. 46. In response to a swiping operation for the slider s, when the swiping direction corresponding to the swiping operation is determined to be horizontally leftward, the wearing position of the target breast pump is on the left side. When the swiping direction corresponding to the swiping operation is determined to be horizontally rightward, the wearing position of the target breast pump is on the right side. That is, optionally, in some embodiments, an auxiliary marking object is shown in the wearing marking interface; and the step of marking the wearing position of the target breast pump in the wearing marking interface in response to the first marking operation may specifically include:
in response to a triggering operation for the auxiliary marking object, controlling the auxiliary marking object to vary according to operation information of the triggering operation; and
marking the wearing position of the target breast pump in the wearing marking interface based on the varied auxiliary marking object.

Optionally, in some embodiments of the present disclosure, the auxiliary marking object may not only be a slider, but may also be a virtual representation, such as a virtual animal and a virtual character. Certainly, the auxiliary marking object may also take the form of a compass or a pointer.

It will be understood that the first marking operation may target not only the auxiliary marking object, but also the electronic device. When the first marking operation is an operation for the electronic device, e.g., a rotating operation for the electronic device, after the user rotates the electronic device, an attitude sensor of the electronic device may detect a change in the attitude of the electronic device and determine the rotation direction of the electronic device at this time. For example, a leftward rotation of the electronic device corresponds to wearing the breast pump on the left side, while a rightward rotation of the electronic device corresponds to wearing the breast pump on the right side. That is, in response to the first marking operation, based on the rotation direction of the electronic device after the first marking operation, the wearing position of the target breast pump is marked in the wearing marking interface.

Optionally, in some embodiments of the present disclosure, the first marking operation may be a tapping operation for the electronic device. That is, the step of marking the wearing position of the target breast pump in the wearing marking interface in response to the first marking operation may specifically include:
in response to the first marking operation, obtaining a number of taps corresponding to the first marking operation; and
marking the wearing position of the target breast pump in the wearing marking interface based on the number of taps.

Thus, the wearing position recording process for a breast pump according to the present disclosure is completed.

Optionally, in some embodiments of the present disclosure, the first marking operation may alternatively be the marking operation for the electronic device. That is, the step of enabling the marking prompt for the target breast pump in response to the first marking operation may specifically include:
in response to the first marking operation, determining a marking position corresponding to the first marking operation; and
enabling a marking prompt corresponding to the marking position for the target breast pump.

Optionally, in some embodiments, a display region is set on the target breast pump to display the marking prompt.

Optionally, the marking prompt is prompting information for wearing the target breast pump on a left breast or a right breast.

As described above, the present disclosure provides a wearing position recording method for a breast pump, including: displaying the wearing marking interface upon detecting that the target breast pump is in the wearing state; and in response to the first marking operation, marking the wearing position of the target breast pump in the wearing marking interface and/or enabling the marking prompt for the target breast pump. In the wearing position recording solution for a breast pump provided by the present disclosure, the wearing marking interface may be displayed, and according to the first marking operation for the wearing marking interface by the user, the wearing position of the target breast pump is marked and/or the marking prompt for the target breast pump is enabled. That is, with a striking interface prompt and/or breast pump prompt, the user can know the wearing position of the target breast pump without confirmation for a plurality of times. Thus, the time taken by the user to set the wearing position of the breast pump can be reduced, thereby improving the recording efficiency of the wearing position.

The present disclosure further provides a wearing position recording method for a breast pump, including: when the target breast pump is in the wearing state, activating a marking indicator lamp of the target breast pump and/or showing a wearing mark in a wearing marking interface in response to a second marking operation for the target breast pump.

FIG. 46 is another flowchart of a wearing position recording method for a breast pump provided by the present disclosure. The specific process of the wearing position recording method for a breast pump may include the following steps.

In step S201, when the target breast pump is in the wearing state, a marking indicator lamp of the target breast pump is activated and/or a wearing mark is shown in a wearing marking interface in response to a second marking operation for the target breast pump.

The second marking operation may be triggered by the user or by the target breast pump itself. For example, the user can manually activate the marking switch of the target breast pump to turn on the marking indicator lamp. Alternatively, the target breast pump may have a control interface for selecting whether to place the target breast pump on the left breast or the right breast. Optionally, the marking indicator lamp of the target breast pump is activated, while the wearing mark is shown in the wearing marking interface. Furthermore, in some embodiments, the user may manually activate the marking switch of the target breast pump. Upon receiving the instruction to activate the switch, the target breast pump transmits the instruction to the electronic device, thereby showing the wearing mark in the wearing marking interface.

Optionally, by distinguishing the induction data between the target breast pump and the inductor on the left cup of the bra versus the induction data between the target breast pump and the inductor on the right cup of the bra, it can be determined whether the target breast pump is worn on the left breast or the right breast. For example, the induction data may be distinguished according to the presence/absence of a signal, signal strength, differential setting, a signal type, etc.

The induction data may be distinguished based on whether the target breast pump and the inductor disposed on the bra interact inductively with each other. For example, an inductor is disposed on the left cup of the bra, while no inductor is disposed on the right cup of the bra, and the target breast pump is placed inside the bra. If the target breast pump interacts inductively with the inductor, it can be determined that the target breast pump is currently worn on the left side. If the target breast pump does not interact inductively with the inductor, it can be determined that the target breast pump is currently worn on the right side. Alternatively, an inductor may be disposed in the middle of the bra, i.e., between the cups on both sides, while a sensor is disposed on the left side/right side of the target breast pump. For example, the sensor is disposed on the left side of the target breast pump, and the target breast pump is placed inside the bra. If the target breast pump interacts inductively with the inductor, it can be determined that the target breast pump is currently worn on the right side. If the target breast pump does not interact inductively with the inductor, it can be determined that the target breast pump is currently worn on the left side.

The induction data may be distinguished according to the size, strength, and the like of the induction data between the target breast pump and the inductor disposed on the bra. For example, a first inductor is disposed on the left cup of the bra, while a second inductor is disposed on the right cup of the bra, and the first inductor is different from the second inductor. The target breast pump is placed inside the bra. If the target breast pump interacts inductively with the first inductor, it can be determined that the target breast pump is currently worn on the left side. If the target breast pump interacts inductively with the second inductor, it can be determined that the target breast pump is currently worn on the right side.

It will be understood that the target breast pump refers to the breast pump currently being worn. Generally, breast pumps are provided in pairs for pumping breast milk from a mother's left and right breasts, respectively. That is, the breast pump purchased by the user may include a first breast pump and a second breast pump. During use, the user may wear the first breast pump first or the second breast pump first, and may initially wear the first breast pump on the left breast or on the right breast.

Optionally, a hall sensor may be disposed on the left side of the first breast pump, and a magnetic module may be disposed on the right side of the second breast pump. When a two-side wearing operation is detected, if mutual inductive interaction is detected between the hall sensor and the magnetic module, it is determined that the first breast pump is worn on the right breast and the second breast pump is worn on the left breast. If the sensor data reported by the first breast pump is null, it is determined that the first breast pump is worn on the left breast and the second breast pump is worn on the right breast.

Optionally, the hall sensor may be disposed on the right side of the first breast pump, and the magnetic module may be disposed on the right side of the second breast pump. When the two-side wearing operation is detected, if mutual inductive interaction is detected between the hall sensor and the magnetic module, it is determined that the first breast pump is worn on the left breast and the second breast pump is worn on the right breast. If the sensor data reported by the first breast pump is null, it is determined that the first breast pump is worn on the right breast and the second breast pump is worn on the left breast.

Optionally, hall sensors may be disposed on both the left and right sides of the first breast pump, with the two hall sensors configured distinctly as a first hall sensor and a second hall sensor. A magnetic module may be disposed on the second breast pump. For example, the first hall sensor is disposed on the left side of the first breast pump, while the second hall sensor is disposed on the right side of the same, and the magnetic module is disposed on the second breast pump. When a two-side wearing operation is detected, if mutual inductive interaction is detected between the second hall sensor and the magnetic module, it is determined that the first breast pump is worn on the left breast and the second breast pump is worn on the right breast. If mutual inductive interaction is detected between the first hall sensor and the magnetic module, it is determined that the first breast pump is worn on the right breast and the second breast pump is worn on the left breast.

Optionally, magnetic modules may be disposed on both the left and right sides of the first breast pump, with the two magnetic modules configured distinctly as a first magnetic module and a second magnetic module. A hall sensor is disposed on the second breast pump. For example, the first magnetic module is disposed on the left side of the first breast pump, while the second magnetic module is disposed on the right side of the same, and the hall sensor is disposed on the second breast pump. When a two-side wearing operation is detected, if mutual inductive interaction is detected between the hall sensor and the first magnetic module, it is determined that the first breast pump is worn on the left breast and the second breast pump is worn on the right breast. If mutual inductive interaction is detected between the hall sensor and the second magnetic module, it is determined that the first breast pump is worn on the right breast and the second breast pump is worn on the left breast.

Optionally, hall sensors may be disposed on both the left sides of the first breast pump and the second breast pump, with the two hall sensors configured distinctly as a first hall sensor and a second hall sensor. Magnetic modules may be disposed on both the right sides of the first breast pump and the second breast pump, with the two magnetic modules configured distinctly as a first magnetic module and a second magnetic module. For example, the first hall sensor is disposed on the left side of the first breast pump, while the second hall sensor is disposed on the left side of the second breast pump, and the first magnetic module is disposed on the right side of the first breast pump, while the second magnetic module is disposed on the right side of the second breast pump. When a two-side wearing operation is detected, if mutual inductive interaction is detected between the first magnetic module and the second hall sensor, it is determined that the first breast pump is worn on the left breast and the second breast pump is worn on the right breast. If mutual inductive interaction is detected between the first hall sensor and the second magnetic module, it is determined that the first breast pump is worn on the right breast and the second breast pump is worn on the left breast. Alternatively, for example, the first hall sensor is disposed on the right side of the first breast pump, while the second hall sensor is disposed on the right side of the second breast pump, and the first magnetic module is disposed on the left side of the first breast pump, while the second magnetic module is disposed on the left side of the second breast pump.

The combination of the hall sensors and the magnetic modules described above is merely an example, and any other sensors and inductive elements capable of mutual detection may also be used.

Optionally, in some embodiments, when a position altering operation for the target breast pump is detected, the wearing mark in the wearing editing interface is updated based on an altered indicator lamp.

Thus, the wearing position recording process for a breast pump according to the present disclosure is completed.

As described above, the present disclosure provides a wearing position recording method for a breast pump. When the target breast pump is in the wearing state, in response to the second marking operation for the target breast pump, the marking indicator lamp of the target breast pump is activated and/or the wearing mark is shown in the wearing marking interface. In the wearing position recording solution for a breast pump provided by the present disclosure, the marking indicator lamp of the target breast pump may be activated and/or the wearing mark may be shown in the wearing marking interface, so that the user can know the wearing position of the target breast pump without confirmation for a plurality of times. Thus, the time taken by the user to set the wearing position of the breast pump can be reduced, thereby improving the recording efficiency of the wearing position.

FIG. 47 is yet another flowchart of a wearing position recording method for a breast pump provided by the present disclosure. The specific process of the wearing position recording method for a breast pump may include the following steps.

In step S301, a detection element disposed on a bra and/or a breast pump is configured to detect whether a target breast pump is worn on a left breast or a right breast.

In step S302, a wearing position of the target breast pump is marked in a wearing marking interface and/or a marking prompt for the target breast pump is enabled according to wearing information of the target breast pump.

The specific detection and marking approaches are similar to those in the foregoing embodiments, and reference may be made to the descriptions of the foregoing embodiments, which will not be reiterated here.

Correspondingly, referring to FIG. 48, an embodiment of the present disclosure provides a wearing position recording apparatus (hereinafter referred to as "recording apparatus") for a breast pump. The detection apparatus includes a display module 201 and a marking module 202.

The display module 201 is configured to display a wearing marking interface upon detecting that a target breast pump is in a wearing state.

The marking module 202 is configured to mark a wearing position of the target breast pump in the wearing marking interface and/or enable a marking prompt for the target breast pump in response to a first marking operation.

Optionally, in some embodiments of the present disclosure, the wearing marking interface includes a first option and a second option; and the marking module 202 may be specifically configured to: mark the wearing position of the target breast pump as a first position in response to a tapping operation for the first option; and mark the wearing position of the target breast pump as a second position in response to a tapping operation for the second option.

Optionally, in some embodiments of the present disclosure, the marking module 202 may be specifically configured to: identify an operation direction of the first marking operation in response to the first marking operation; when the operation direction is identified as a first preset direction, mark the wearing position of the target breast pump as a first position; and when the operation direction is identified as a second preset direction, mark the wearing position of the target breast pump as a second position.

Optionally, in some embodiments of the present disclosure, an auxiliary marking object is shown in the wearing marking interface; and the marking module 202 may be specifically configured to: in response to a triggering operation for the auxiliary marking object, control the auxiliary marking object to vary according to operation information of the triggering operation; and mark the wearing position of the target breast pump in the wearing marking interface based on the varied auxiliary marking object.

Optionally, in some embodiments of the present disclosure, the marking module 202 may be specifically configured to: in response to the first marking operation, obtain a number of taps corresponding to the first marking operation; and mark the wearing position of the target breast pump in the wearing marking interface based on the number of taps.

Optionally, in some embodiments of the present disclosure, the marking module 202 may be specifically configured to: in response to the first marking operation, determine a marking position corresponding to the first marking operation; and enable the marking prompt corresponding to the marking position for the target breast pump.

Optionally, in some embodiments, a display region is set on the target breast pump to display the marking prompt.

Optionally, the marking prompt is prompting information for wearing the target breast pump on a left breast or a right breast.

As described above, the present disclosure provides a wearing position recording apparatus for a breast pump. The display module 201 is configured to display the wearing marking interface upon detecting that the target breast pump is in the wearing state. The marking module 202 is configured to mark the wearing position of the target breast pump in the wearing marking interface and/or enable the marking prompt for the target breast pump in response to the first marking operation. In the wearing position recording solution for a breast pump provided by the present disclosure, the wearing marking interface may be displayed, and according to the first marking operation for the wearing marking interface by the user, the wearing position of the target breast pump is marked and/or the marking prompt for the target breast pump is enabled. That is, with a striking interface prompt and/or breast pump prompt, the user can know the wearing position of the target breast pump without confirmation for a plurality of times. Thus, the time taken by the user to set the wearing position of the breast pump can be reduced, thereby improving the recording efficiency of the wearing position.

Referring to FIG. 49, an embodiment of the present disclosure provides a wearing position recording apparatus (hereinafter referred to as "recording apparatus") for a breast pump. The detection apparatus includes an activation module 203.

The activation module 203 is configured to, upon detecting that a target breast pump is in a wearing state, activate a marking indicator lamp of the target breast pump and/or show a wearing mark in a wearing marking interface in response to a second marking operation for the target breast pump.

As described above, the present disclosure provides a wearing position recording apparatus for a breast pump. The activation module 203 is configured to, when the target breast pump is in the wearing state, in response to the second marking operation for the target breast pump, activate the marking indicator lamp of the target breast pump and/or show the wearing mark in the wearing marking interface. In the wearing position recording solution for a breast pump provided by the present disclosure, the marking indicator lamp of the target breast pump may be activated and/or the wearing mark may be shown in the wearing marking interface, so that the user can know the wearing position of the target breast pump without confirmation for a plurality of times. Thus, the time taken by the user to set the wearing position of the breast pump can be reduced, thereby improving the recording efficiency of the wearing position.

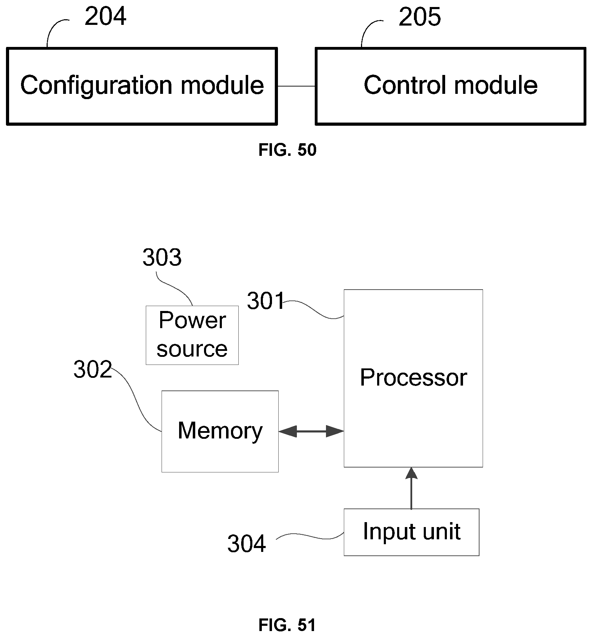

Referring to FIG. 50, an embodiment of the present disclosure provides a wearing position recording apparatus (hereinafter referred to as "recording apparatus") for a breast pump. The detection apparatus includes a configuration module 204 and a control module 205.

The configuration module 204 is configured to configure a detection element disposed on a bra and/or a breast pump to detect whether a target breast pump is worn on a left breast or a right breast.

The control module 205 is configured to mark a wearing position of the target breast pump in a wearing marking interface and/or enable a marking prompt for the target breast pump according to wearing information of the target breast pump.

Furthermore, the present disclosure further provides an electronic device. FIG. 50 illustrates a schematic structural diagram of an electronic device involved in the present disclosure. Specifically, the electronic device may include components such as a processor 301 with one or more processing cores, a memory 302 with one or more computer-readable storage media, a power source 303, and an input unit 304. It will be understood by those skilled in the art that the structure of the electronic device as shown in FIG. 50 does not constitute a limitation on the electronic device, which may include more or less components than those shown in the figure, or some components are combined, or different component arrangements are provided.

The processor 301 is a control center of the electronic device, connects to various parts of the electronic device by using various interfaces and lines, and performs various functions and data processing of the electronic device by running or executing software programs and/or modules stored in the memory 302 and calling data stored in the memory 302, so as to perform overall monitoring on the electronic device. Optionally, the processor 301 may include one or more processing cores. Preferably, the processor 301 may be integrated with an application processor and a modulation and demodulation processor. The application processor is configured to mainly process an operating system, a user interface, an application program, and the like. The modulation and demodulation processor is configured to mainly process wireless communication. It will be understood that the modulation and demodulation processor may not be integrated into the processor 301.

The memory 302 may be configured to store a software program and a module, and the processor 301 is configured to run the software and the module stored in the memory 302 to perform various functional applications and data processing. The memory 302 may mainly include a program storage area and a data storage area. The program storage area may store an operating system, an application program required by at least one function (such as a sound playing function and an image playing function), and the like. The data storage area may store data created based on the use of the electronic device, and the like. Moreover, the memory 302 may include a highspeed random access memory, and may also include a non-volatile memory, for example, at least one magnetic disk storage device, a flash memory device, or other volatile solid storage device. Correspondingly, the memory 302 may further include a memory controller to provide access to the memory 302 by the processor 301.

The electronic device may further include the power source 303 that supplies power to various components. Preferably, the power source 303 can be logically connected to the processor 301 via a power management system. Thus, functions such as charging, discharging, and power consumption management may be achieved via the power management system. The power source 303 may further include any component such as one or more direct-current or alternating-current power sources, a recharging system, a power failure detection circuit, a power converter or inverter, and a power status indicator.

The electronic device may further include the input unit 304 that can be configured to receive entered numbers or character information, and generate keyboard, mouse, joystick, optical or trackball signal inputs associated with user settings and function control.

Although not shown, the electronic device may further include a display unit and the like, which will not be repeated herein. Specifically, in this embodiment, the processor 301 in the electronic device may load executable files corresponding to processes of one or more application programs into the memory 302 according to instructions as described below, and the application programs stored in the memory 302 may be run by the processor 301 to achieve various functions as follows:
displaying a wearing marking interface upon detecting that a target breast pump is in a wearing state; in response to a first marking operation, marking a wearing position of the target breast pump in the wearing marking interface and/or enabling a marking prompt for the target breast pump;
when the target breast pump is in the wearing state, activating a marking indicator lamp of the target breast pump and/or showing a wearing mark in a wearing marking interface in response to a second marking operation for the target breast pump.
configuring a detection element disposed on a bra and/or a breast pump to detect whether a target breast pump is worn on a left breast or a right breast; and marking a wearing position of the target breast pump in a wearing marking interface and/or enabling a marking prompt for the target breast pump according to wearing information of the target breast pump.

Regarding to specific implementations of the above various operations, see the foregoing embodiments, which will not be repeated herein.

In the embodiments of the present disclosure, the wearing marking interface may be displayed, and according to the first marking operation for the wearing marking interface by the user, the wearing position of the target breast pump is marked and/or the marking prompt for the target breast pump is enabled. That is, with a striking interface prompt and/or breast pump prompt, the user can know the wearing position of the target breast pump without confirmation for a plurality of times. Thus, the time taken by the user to set the wearing position of the breast pump can be reduced, thereby improving the recording efficiency of the wearing position.

Those of ordinary skill in the art will understand that all or part of the steps in the methods of the above embodiments may be implemented with instructions or by controlling related hardware via instructions. The instructions may be stored in a computer-readable storage medium and loaded and executed by a processor.

To this end, the present disclosure provides a storage medium having a plurality of instructions stored therein that can be loaded by a processor to perform the steps of any wearing position recording method for a breast pump provided by the present disclosure. For example, the instructions may cause the following steps to be performed:
displaying a wearing marking interface upon detecting that a target breast pump is in a wearing state; in response to a first marking operation, marking a wearing position of the target breast pump in the wearing marking interface and/or enabling a marking prompt for the target breast pump;
when the target breast pump is in the wearing state, activating a marking indicator lamp of the target breast pump and/or showing a wearing mark in a wearing marking interface in response to a second marking operation for the target breast pump.

Regarding to specific implementations of the above various operations, see the foregoing embodiments, which will not be repeated herein.

In the embodiments of the present disclosure, the induction data of the target breast pump or the inductor is obtained upon receiving the wearing completion operation for the target breast pump; and the wearing position detection result of the target breast pump is determined based on the induction data. In the wearing position detection method for a breast pump provided by the present disclosure, when the wearing completion operation for the target breast pump is received, the induction data of the bra corresponding to the target breast pump may be obtained. The wearing position detection result of the target breast pump is output via the position detection strategy corresponding to the induction data. Thus, the user does not need to manually set the wearing position of the breast pump after wearing the breast pump. As a result, the reliability of milk expression data recorded by the breast pump is improved.

Those of ordinary skill in the art will understand that all or part of the steps in the methods of the above embodiments may be implemented with instructions or by controlling related hardware via instructions. The instructions may be stored in a computer-readable storage medium and loaded and executed by a processor.

To this end, the present disclosure provides a storage medium having a plurality of instructions stored therein that can be loaded by a processor to perform the steps of any wearing position detection method for a breast pump provided by the present disclosure. For example, the instructions may cause the following steps to be performed:
obtaining the induction data of the target breast pump or the inductor upon receiving the wearing completion operation for the target breast pump; and determining the wearing position detection result of the target breast pump based on the induction data.

Regarding to specific implementations of the above various operations, see the foregoing embodiments, which will not be repeated herein.

The storage medium may include a read-only memory (ROM), a random access memory (RAM), a magnetic disk, an optical disc, or the like.

Since the instructions stored in the storage medium are executable to perform the steps of any wearing position detection method for a breast pump provided by the present disclosure, the beneficial effects achievable by any wearing position detection method for a breast pump provided by the present disclosure can be achieved. For details, reference is made to the preceding embodiments, which will not be reiterated here.

According to one aspect of the present disclosure, a computer program product or computer program is provided. The computer program product or computer program includes computer instructions. The computer instructions are stored in a computer-readable storage medium. A processor of an electronic device reads the computer instructions from the computer-readable storage medium. The processor executes the computer instructions to cause the electronic device to perform the methods provided in the foregoing various optional implementations.

The wearing position detection method and apparatus for a breast pump, the storage medium, the bra, and the system provided in the present disclosure are described above in detail. Specific examples are used herein for illustration of the principles and embodiments of the present disclosure. The description of the foregoing embodiments is only used to help understand the method and its core ideas of the present disclosure. Moreover, those of ordinary skill in the art can make various modifications in terms of specific embodiments and scope of application in accordance with the ideas of the present disclosure. In conclusion, the content of this specification shall not be construed as a limitation to the present disclosure.

## Claims

1. A wearing position detection method for a breast pump, comprising:
configuring an inductor disposed on a bra, wherein the inductor is configured to interact inductively with a sensor disposed on the breast pump;
obtaining induction data of a target breast pump or the inductor upon receiving a wearing completion operation for the target breast pump; and
determining a wearing position detection result of the target breast pump based on the induction data.

2. The wearing position detection method according to claim 1, wherein the determining a wearing position detection result of the target breast pump based on the induction data comprises:
detecting the induction data; and
determining a wearing position of the target breast pump based on whether a signal from the inductor is detected.

3. The wearing position detection method according to claim 1, wherein the determining a wearing position detection result of the target breast pump based on the induction data comprises:
detecting the induction data; and
determining a wearing position of the target breast pump based on a detected first induction signal or second induction signal.

4. The wearing position detection method according to claim 1, wherein the determining a wearing position detection result of the target breast pump based on the induction data comprises:
extracting capacitance information and infrared information from the induction data;
determining a wearing position of the target breast pump as a preset position when the capacitance information is null; and
determining that the wearing position of the target breast pump corresponds to the inductor when the capacitance information is not null.

5. The wearing position detection method according to any one of claims 1 to 4, after determining the wearing position detection result of the target breast pump based on the induction data, further comprising:
outputting a wearing position prompt of the wearing position detection result.

6. The wearing position detection method according to any one of claims 1 to 5, after determining the position wearing result of the target breast pump based on the induction data, further comprising:
recording milk expression data corresponding to breasts on different sides according to the position wearing result.

7. The wearing position detection method according to any one of claims 1 to 6, wherein a wearing position information prompt is displayed on a terminal device or the breast pump.

8. A computer-readable storage medium, having a computer program stored thereon, wherein the computer program, when executed by a processor, causes the processor to perform steps of the wearing position detection method for a target breast pump according to any one of claims 1 to 7.

9. A wearing position detection system for a breast pump, comprising:
an inductor disposed on a bra and configured to interact inductively with a sensor disposed on the breast pump worn on the bra; and
a controller configured to determine whether the breast pump is worn on a left breast or a right breast according to induction information of the inductor.

10. A bra, comprising:
a bra body configured to be worn on a breast, wherein a breast pump is placed between the bra body and the breast to express milk from the breast; and
an inductor disposed on the bra body and configured to interact inductively with a sensor disposed on the breast pump to detect whether the breast pump is worn on a left breast or a right breast.

11. A wearing position detection system for a breast pump, comprising:
a breast pump device configured to express breast milk and comprising a sensor;
an inductor disposed on a bra and configured to interact inductively with the sensor; and
a controller disposed on a breast pump body of the breast pump device and configured to determine whether the breast pump device is worn on a left breast or a right breast according to induction information of the inductor and the sensor.

12. The wearing position detection system for a breast pump according to claim 11, wherein inductors are provided in pairs and comprise a first inductor and a second inductor that are disposed on a left side and a right side of the bra, respectively; the first inductor is different from the second inductor; and
the determining whether the breast pump device is worn on the left breast or the right breast according to induction information of the inductor and the sensor comprises:
determining whether the breast pump device is worn on the left breast or the right breast based on whether the sensor detects the first inductor or the second inductor.

13. The wearing position detection system for a breast pump according to claim 11, wherein the inductor is disposed on a left side or a right side of the bra; and
the determining whether the breast pump device is worn on the left breast or the right breast according to induction information of the inductor comprises:
determining whether the breast pump device is worn on the left breast or the right breast based on whether the sensor detects the inductor.

14. The wearing position detection system for a breast pump according to claim 11, wherein the inductor is disposed in a middle position of the bra.

15. A wearing position detection system for a breast pump, comprising:
a breast pump device configured to express breast milk and comprising a sensor;
a bra body configured to be worn on a breast, wherein the breast pump device is placed between the bra body and the breast to express milk from the breast; and
an inductor disposed on the bra body and configured to interact inductively with the sensor to detect whether a target breast pump is worn on a left breast or a right breast.

16. A wearing position detection method for a breast pump, comprising:
obtaining first human body data acquired by a sensor disposed on a target breast pump upon detecting that the target breast pump is in a wearing state; and
determining a position wearing result of the target breast pump based on the first human body data.

17. The wearing position detection method according to claim 16, wherein the determining a position wearing result of the target breast pump based on the first human body data comprises:
obtaining pre-stored human body data; and
determining whether the target breast pump is worn on a left breast or a right breast based on an association relationship between the first human body data and
the pre-stored human body data.

18. The wearing position detection method according to claim 16 or 17, wherein the human body data is at least one of blood oxygen concentration, heartbeat, breast shape, transmittance, body fat percentage, muscle mass, hardness, or elasticity data of a human body.

19. The wearing position detection method according to any one of claims 16 to 18, after determining the wearing position detection result of the target breast pump based on the human body data, further comprising:
recording milk expression data corresponding to breasts on different sides according to the wearing position detection result.

20. The wearing position detection method according to claim 19, wherein the recording milk expression data corresponding to breasts on different sides according to the wearing position detection result comprises:
obtaining historical wearing information of the target breast pump; and
recording the milk expression data corresponding to the breasts on different sides according to the historical wearing information and the wearing position detection result.

21. The wearing position detection method according to any one of claims 16 to 20, wherein a wearing position information prompt is displayed on a terminal device or the breast pump.

22. A breast pump, comprising:
a breast pump body configured to be worn on a breast to express milk from the breast;
an inductive assembly disposed on the breast pump body and configured to acquire human body data; and
a controller disposed on the breast pump body and configured to determine a wearing position detection result of the breast pump based on the human body data.

23. The breast pump according to claim 22, wherein the determining a wearing position detection result of the breast pump comprises: determining whether the breast pump is worn on a left breast or a right breast.

24. The breast pump according to claim 22, wherein the human body data comprises at least one of blood oxygen concentration, heartbeat, breast shape, transmittance, body fat percentage, muscle mass, hardness, or elasticity data of a human body.

25. The breast pump according to claim 22, wherein the inductive assembly comprises at least one of a capacitive sensor, an inductive sensor, a hardness sensor, an infrared sensor, and an ultrasonic sensor.

26. The breast pump according to claim 22, wherein a control panel is disposed on the breast pump body; and the controller is further configured to control the control panel to display a wearing position of the breast pump.

27. A computer-readable storage medium, having a computer program stored thereon, wherein the computer program, when executed by a processor, causes the processor to perform the wearing position detection method for a target breast pump according to any one of claims 16 to 21.

28. A wearing position detection method for a breast pump, comprising:
receiving sensor data reported by a first breast pump, wherein the sensor data comprises induction data that is about an inductive assembly disposed on a second breast pump and acquired by a sensor assembly disposed on the first breast pump; and
determining wearing position detection results of the first breast pump and the second breast pump according to the sensor data.

29. The wearing position detection method according to claim 28, wherein the determining wearing position detection results of the first breast pump and the second breast pump according to the sensor data comprises:
determining a relative position of the sensor assembly to the inductive assembly; and
determining the wearing position detection results of the first breast pump and the second breast pump based on the sensor data and the relative position of the sensor assembly to the inductive assembly.

30. The wearing position detection method according to claim 29, wherein the determining a relative position of the sensor assembly to the inductive assembly comprises:
determining the relative position based on whether the sensor assembly has detected the inductive assembly; and/or
determining the relative position according to a signal strength detected by the sensor assembly regarding the inductive assembly.

31. The wearing position detection method according to claim 29, wherein the inductive assembly comprises a first inductor and a second inductor; and
the determining a relative position of the sensor assembly to the inductive assembly comprises:
when the sensor assembly has detected the first inductor, determining that the sensor assembly is close to the first inductor; and
when the sensor assembly has detected the second inductor, determining that the sensor assembly is close to the second inductor.

32. The wearing position detection method according to claim 29, wherein the sensor assembly comprises a first sensor and a second sensor; and
the determining a relative position of the sensor assembly to the inductive assembly comprises:
when the first sensor has detected an inductor, determining that the inductor is close to the first sensor; and
when the second sensor has detected the inductor, determining that the inductor is close to the second sensor.

33. The wearing position detection method according to any one of claims 28 to 32, after determining the wearing position detection results of the first breast pump and the second breast pump according to the sensor data, further comprising:
recording milk expression data corresponding to breasts on different sides according to the wearing position detection results.

34. The wearing position detection method according to claim 33, wherein the recording milk expression data corresponding to breasts on different sides according to the wearing position detection results comprises:
obtaining historical wearing results corresponding to the first breast pump and the second breast pump; and
recording the milk expression data corresponding to the breasts on different sides according to the wearing position detection results and the historical wearing results corresponding to the first breast pump and the second breast pump.

35. A wearing position detection system for a breast pump, comprising:
a first breast pump and a second breast pump;
a sensor assembly disposed on the first breast pump;
an inductive assembly disposed on the second breast pump; and
a controller disposed on the first breast pump or the second breast pump;
wherein the sensor assembly is configured to acquire induction data about the inductive assembly; and
the controller is configured to determine wearing position detection results of the first breast pump and the second breast pump according to the induction data.

36. The wearing position detection system according to claim 35, wherein the determining wearing position detection results of the first breast pump and the second breast pump according to the induction data comprises:
determining a relative position of the sensor assembly to the inductive assembly; and
determining the wearing position detection results of the first breast pump and the second breast pump based on the induction data and the relative position of the sensor assembly to the inductive assembly.

37. The wearing position detection system according to claim 36, wherein the determining a relative position of the sensor assembly to the inductive assembly comprises:
determining the relative position based on whether the sensor assembly has detected the inductive assembly; and/or
determining the relative position according to a signal strength detected by the sensor assembly regarding the inductive assembly.

38. The wearing position detection system according to claim 36, wherein the inductive assembly comprises a first inductor and a second inductor; the first inductor is different from the second inductor; and
the determining a relative position of the sensor assembly to the inductive assembly comprises:
when the sensor assembly has detected the first inductor, determining that the sensor assembly is close to the first inductor; and
when the sensor assembly has detected the second inductor, determining that the sensor assembly is close to the second inductor.

39. The wearing position detection system according to claim 36, wherein the sensor assembly comprises a first sensor and a second sensor; the first sensor is different from the second sensor; and
the determining a relative position of the sensor assembly to the inductive assembly comprises:
when the first sensor has detected an inductor, determining that the inductor is close to the first sensor; and
when the second sensor has detected the inductor, determining that the inductor is close to the second sensor.

40. A wearing position detection system for a breast pump, comprising:
a first breast pump and a second breast pump;
a first sensor and a first inductor that are disposed on the first breast pump;
a second sensor and a second inductor that are disposed on the second breast pump; and
a controller disposed on the first breast pump or the second breast pump;
wherein the first sensor is configured to acquire first induction data about the first inductor and/or the second inductor;
the second sensor is configured to acquire second induction data about the first inductor and/or the second inductor; and
the controller is configured to determine wearing position detection results of the first breast pump and the second breast pump according to the first induction data and/or the second induction data.

41. A computer-readable storage medium, having a computer program stored thereon, wherein the computer program, when executed by a processor, causes the processor to perform steps of the wearing position detection method for a breast pump according to any one of claims 28 to 34.

42. A wearing prompting method for a breast pump, comprising:
obtaining wearing information corresponding to the breast pump, wherein the wearing information is acquired by a sensor disposed on the breast pump; and
controlling the breast pump and/or a terminal device to execute a prompting event according to the wearing information.

43. The wearing prompting method according to claim 42, wherein the prompting event comprises:
playing a prompt tone for correct wearing; and/or
playing a prompt tone for incorrect wearing.

44. The wearing prompting method according to claim 42, wherein the prompting event comprises:
displaying prompting information for incorrect wearing on a display screen of the breast pump and/or the terminal device.

45. The wearing prompting method according to claim 44, further comprising:
displaying a wearing guidance for correct wearing on the display screen of the breast pump and/or the terminal device.

46. The wearing prompting method according to claim 44, wherein the prompting event comprises:
in response to correct wearing, controlling the breast pump and/or a current device to vibrate for a first preset number of times; and
in response to incorrect wearing, controlling the breast pump and/or the current device to vibrate for a second preset number of times.

47. The wearing prompting method according to claim 46, wherein the first preset number of times is different from the second preset number of times.

48. The wearing prompting method according to any one of claims 42 to 47, wherein the obtaining wearing information corresponding to the breast pump comprises:
obtaining the wearing information corresponding to the breast pump according to acceleration information of the breast pump.

49. A breast pump, comprising:
a breast pump body; and
a wearing detection mechanism configured to detect wearing information of the breast pump body.

50. The breast pump according to claim 49, wherein the wearing detection mechanism comprises:
a signal prompting element; and
a processor configured to control, based on the wearing information, the signal prompting element to output prompting information based on the wearing information.

51. The breast pump according to claim 50, wherein
the signal prompting element comprises a speaker; and
the controlling the signal prompting element to output prompting information based on the wearing information comprises: controlling the speaker to play a prompt tone for correct wearing and/or a prompt tone for incorrect wearing.

52. The breast pump according to claim 50 or 51, wherein
the signal prompting element comprises a display screen; and
the controlling the signal prompting element to output prompting information based on the wearing information comprises: controlling the display screen to display prompting information for incorrect wearing.

53. The breast pump according to claim 52, wherein the processor is further configured to control the display screen to display a wearing guidance for correct wearing.

54. The breast pump according to any one of claims 50 to 53, wherein
the signal prompting element comprises a vibrating element; and
the controlling the signal prompting element to output prompting information based on the wearing information comprises:
in response to correct wearing, controlling the breast pump and/or a current device to vibrate for a first preset number of times; and
in response to incorrect wearing, controlling the breast pump and/or the current device to vibrate for a second preset number of times.

55. The breast pump according to claim 49, further comprising:
a processor configured to generate the prompting information according to the wearing information; and
a transmitter configured to transmit the prompting information to a terminal device.

56. The breast pump according to claim 49, further comprising:
a transmitter configured to transmit the wearing information to a terminal device.

57. A terminal device, comprising:
a receiver configured to receive wearing information of a breast pump transmitted from the breast pump;
a signal prompting element; and
a processor configured to control the signal prompting element to output prompting information according to the wearing information.

58. The terminal device according to claim 57, wherein
the signal prompting element comprises a speaker; and
the controlling the signal prompting element to output prompting information based on the wearing information comprises: controlling the speaker to play a prompt tone for correct wearing and/or a prompt tone for incorrect wearing.

59. The terminal device according to claim 57 or 58, wherein
the signal prompting element comprises a display screen; and
the controlling the signal prompting element to output prompting information based on the wearing information comprises: controlling the display screen to display prompting information for incorrect wearing.

60. The terminal device according to claim 59, wherein the processor is further configured to control the display screen to display a wearing guidance for correct wearing.

61. The terminal device according to any one of claims 57 to 60, wherein
the signal prompting element comprises a vibrating element; and
the controlling the signal prompting element to output prompting information based on the wearing information comprises:
in response to correct wearing, controlling the breast pump and/or a current device to vibrate for a first preset number of times; and
in response to incorrect wearing, controlling the breast pump and/or the current device to vibrate for a second preset number of times.

62. A terminal device, comprising:
a receiver configured to receive wearing information transmitted from a breast pump;
a signal prompting element; and
a processor configured to control the signal prompting element to output wearing prompting information according to the wearing information.

63. A wearing prompting system for the breast pump according to claim 49, wherein the prompting information comprises a prompt tone, a prompting page, and a wearing guidance.

64. A wearing prompting system for the breast pump according to claim 50, wherein the wearing detection mechanism comprises a sensor configured to detect whether the breast pump body closely fits a breast and/or detect whether a mother's nipple is placed along a nipple channel and/or detect whether the breast pump body is worn obliquely.

65. A wearing guidance method for a breast pump, comprising:
showing wearing guidance information for a target breast pump in response to a wearing operation triggered for the target breast pump, wherein the wearing guidance information is configured for guiding a user to wear the target breast pump in an operating mode.

66. The wearing guidance method for a breast pump according to claim 65, further comprising: determining whether a current wearing state of the target breast pump satisfies a preset operating wearing state; and
when the current wearing state of the target breast pump satisfies the operating wearing state, configuring the target breast pump with configuration parameters corresponding to a target operating mode and/or showing prompting information for successful wearing.

67. The wearing guidance method according to claim 66, wherein the configuring the target breast pump with configuration parameters corresponding to a target operating mode comprises:
displaying a parameter configuration page; and
configuring the target breast pump with the configuration parameters corresponding to the target operating mode in response to a parameter configuration operation for the parameter configuration page.

68. The wearing guidance method according to claim 66 or 67, further comprising:
displaying an experience control; and
controlling the target breast pump to operate according to a preset suction force strategy in the target operating mode in response to a tapping operation for the experience control.

69. The wearing guidance method according to any one of claims 65 to 68, further comprising:
showing an option control for wearing the target breast pump on a left breast or a right breast in a wearing guidance page.

70. The wearing guidance method according to claim 69, further comprising:
displaying a wearing position of the target breast pump in the wearing guidance page in response to a selection operation for the option control.

71. The wearing guidance method according to any one of claims 65 to 70, further comprising:
displaying a wearing prompt for a user to select to wear the target breast pump on the left breast or the right breast in the wearing guidance page.

72. The wearing guidance method according to any one of claims 65 to 71, further comprising:
when the current wearing state of the target breast pump does not satisfy the operating wearing state, showing prompting information for incorrect wearing in the wearing guidance page.

73. A breast pump system, comprising:
a breast pump body configured to be worn on a mother's breast and express milk from the breast, wherein the breast pump body comprises:
a breast milk container comprising a milk storage container and a connection portion, wherein the milk storage container is connected to the connection portion;
a milk pumping member comprising an attachment portion and a nipple channel, wherein a shape of the attachment portion is adapted to the breast, and the nipple channel is connected to the milk storage container via the connection portion; and
a wearing detection mechanism configured to detect whether the milk pumping member is worn correctly.

74. The breast pump system according to claim 73, wherein the wearing detection mechanism comprises a first sensor disposed on the attachment portion and a second sensor disposed on the nipple channel; and the breast pump system further comprises a controller disposed on the breast pump body and configured to determine whether the milk pumping member is worn correctly according to induction information of the first sensor and induction information of the second sensor.

75. The breast pump system according to claim 74, wherein the first sensor is configured to detect whether the attachment portion closely fits the breast.

76. The breast pump system according to any one of claim 74 or 75, wherein the second sensor is configured to detect whether the mother's nipple is placed along the nipple channel.

77. The breast pump system according to any one of claims 73 to 76, wherein the wearing detection mechanism further comprises a third sensor configured to detect whether the breast pump body is worn obliquely.

78. The breast pump system according to any one of claims 73 to 77, further comprising a communication connection module configured to connect the breast pump body and a terminal device or a server and transmit milk expression-related information of the breast pump body.

79. The breast pump system according to any one of claims 73 to 78, further comprising an identification element configured at least to identify one target breast pump body such that the terminal device or the server identifies and matches the target breast pump body.

80. The breast pump system according to any one of claims 73 to 79, further comprising identification elements, namely a first identification element and a second identification element that are configured at least to identify two target breast pump bodies, respectively, such that the terminal device or the server identifies and matches each of the target breast pump bodies.

81. A wearing position recording method for a breast pump, comprising:
displaying a wearing marking interface upon detecting that a target breast pump is in a wearing state; and
marking a wearing position of the target breast pump in the wearing marking interface and/or enabling a marking prompt for the target breast pump in response to a first marking operation.

82. The wearing position recording method according to claim 81, wherein the wearing marking interface comprises a first option and a second option; and the marking a wearing position of the target breast pump in the wearing marking interface in response to a first marking operation comprises:
marking the wearing position of the target breast pump as a first position in response to a tapping operation for the first option; and
marking the wearing position of the target breast pump as a second position in response to a tapping operation for the second option.

83. The wearing position recording method according to claim 81, wherein the marking a wearing position of the target breast pump in the wearing marking interface in response to a first marking operation comprises:
identifying an operation direction of the first marking operation in response to the first marking operation;
when the operation direction is identified as a first preset direction, marking the wearing position of the target breast pump as a first position; and
when the operation direction is identified as a second preset direction, marking the wearing position of the target breast pump as a second position.

84. The wearing position recording method according to claim 81, wherein an auxiliary marking object is shown in the wearing marking interface; and the marking a wearing position of the target breast pump in the wearing marking interface in response to a first marking operation comprises:
in response to a triggering operation for the auxiliary marking object, controlling the auxiliary marking object to vary according to operation information of the triggering operation; and
marking the wearing position of the target breast pump in the wearing marking interface based on the varied auxiliary marking object.

85. The wearing position recording method according to claim 81, wherein the marking a wearing position of the target breast pump in the wearing marking interface in response to a first marking operation comprises:
in response to the first marking operation, obtaining a number of taps corresponding to the first marking operation; and
marking the wearing position of the target breast pump in the wearing marking interface based on the number of taps.

86. The wearing position recording method according to claim 81, wherein the enabling a marking prompt for the target breast pump in response to a first marking operation comprises:
in response to the first marking operation, determining a marking position corresponding to the first marking operation; and
enabling a marking prompt corresponding to the marking position for the target breast pump.

87. The wearing position recording method according to claim 86, wherein a display region is set on the target breast pump to display the marking prompt.

88. The wearing position recording method according to claim 86, wherein the marking prompt is prompting information for wearing the target breast pump on a left breast or a right breast.

89. A wearing position recording method for a breast pump, comprising:
when a target breast pump is in a wearing state, activating a marking indicator lamp of the target breast pump and/or showing a wearing mark in a wearing marking interface in response to a second marking operation for the target breast pump.

90. The wearing position recording method according to claim 89, further comprising:
when a position altering operation for the target breast pump is detected, updating the wearing mark in the wearing marking interface based on an altered indicator lamp.

91. A wearing position recording method for a breast pump, comprising:
configuring a detection element disposed on a bra and/or a breast pump to detect whether a target breast pump is worn on a left breast or a right breast; and
marking a wearing position of the target breast pump in a wearing marking interface and/or enabling a marking prompt for the target breast pump according to wearing information of the target breast pump.

92. A terminal device, comprising: a memory and a processor;
wherein the memory is configured to store a computer program;
the processor is configured to execute the computer program; and
the computer program is configured to cause the processor to perform the method according to claims 79 to 89.

93. A computer-readable storage medium, having a computer program stored thereon, wherein the computer program, when executed by a processor, causes the processor to perform the wearing position recording method according to any one of claims 79 to 89.
